# EUROPEAN PATENT SPECIFICATION

(11) **EP 2 625 180 B1**
(45) Date of publication and mention of the grant of the patent: **02.03.2016**
(21) Application number: 11831640.5
(22) Date of filing: 06.10.2011
(51) Int. Cl.: C07D 487/22, A61K 31/409, A61P 25/16, A61P 25/00

(54) **PORPHYRIN TREATMENT OF NEURODEGENERATIVE DISEASES**
PORPHYRINBEHANDLUNG NEURODEGENERATIVER ERKRANKUNGEN
TRAITEMENT À LA PORPHYRINE DE MALADIES NEURODÉGÉNÉRATIVES

(30) Priority: 06.10.2010 US 390270 P
(43) Date of publication of application: 14.08.2013
(73) Proprietor: Aeolus Sciences, Inc., Mission Viejo, CA 92691 (US); National Jewish Health, Denver, CO 80206 (US); The Regents of the University of Colorado, A Body Corporate, Denver, CO 80203 (US)
(72) Inventor: PATEL, Manisha, Englewood Colorado 80111 (US); DAY, Brian, Englewood Colorado 80111 (US); MCMANUS, John, Mission Viejo California 92691 (US)
(74) Representative: Couchman, Jonathan Hugh
(86) International application number: PCT/US2011/055172
(87) International publication number: WO 2012/048164

(56) References cited:
- EP-A2- 0 665 230
- WO-A1-00/43395
- WO-A1-99/23097
- WO-A1-99/55388
- WO-A2-2011/028935
- US-A- 5 571 908
- US-A1- 2004 023 941
- US-A1- 2008 039 436
- US-B1- 6 479 477
- TROVA, MICHAEL P. ET AL: "Superoxide dismutase mimetics. Part 2: synthesis and structure-activity relationship of glyoxylate- and glyoxamide-derived metalloporphyrins", BIOORGANIC & MEDICINAL CHEMISTRY, CODEN: BMECEP; ISSN: 0968-0896, vol. 11, no. 13, 2003, pages 2695-2707, XP002721477, DOI: 10.1016/S0968-0896(03)00272-4
- NORIKAZU NISHINO ET AL: "Synthesis of Linear Amphipathic Porphyrin Dimers and Trimers: An Approach to Bilayer Lipid Membrane Spanning Porphyrin Arrays", THE JOURNAL OF ORGANIC CHEMISTRY, vol. 61, no. 21, 1996, pages 7534-7544, XP055106086, ISSN: 0022-3263, DOI: 10.1021/jo9611576
- RYU SAKAMOTO ET AL: "meso-Trifluoromethyl-substituted Subporphyrin from Ring-splitting Reaction of meso-Trifluoromethyl-substituted [32]Heptaphyrin(1.1.1.1.1.1.1)", CHEMISTRY LETTERS, vol. 39, no. 5, 31 March 2010 (2010-03-31) , pages 439-441, XP055106083, ISSN: 0366-7022, DOI: 10.1246/cl.2010.439
- NORA Y. NELSON ET AL: "Synthesis and unusual properties of the first 2,3,7,8,12,13,17,18-octabromo-5,10,15,20-t etraalkylporphyrin", CHEMICAL COMMUNICATIONS, no. 20, 1999, pages 2071-2072, XP055106084, ISSN: 1359-7345, DOI: 10.1039/a904532e
- GOLL, JAMES G. ET AL: "Synthesis, Structure, Electronic Spectroscopy, Photophysics, Electrochemistry, and X-ray Photoelectron Spectroscopy of Highly-Electron-Deficient [5,10,15,20- Tetrakis(perfluoroalkyl)porphinato]zinc(II ) Complexes and Their Free Base Derivatives", JOURNAL OF THE AMERICAN CHEMICAL SOCIETY , 118(35), 8344-8354 CODEN: JACSAT; ISSN: 0002-7863, vol. 118, no. 35, 1996, pages 8344-8354, XP002721478, DOI: 10.1021/JA9610904

## Description

### BACKGROUND OF THE INVENTION

Neurodegenerative orders affect millions of people worldwide. For example, Parkinson's disease (PD) is an age-related neurodegenerative disease in which the role of reactive oxygen species (ROS) is strongly implicated. There remains a need in the art for effective treatments of neurodegenerative disorders. The present invention provides solutions to these and other needs in the art.

### BRIEF SUMMARY OF THE INVENTION

In a first aspect, provided herein is a compound having the formula: 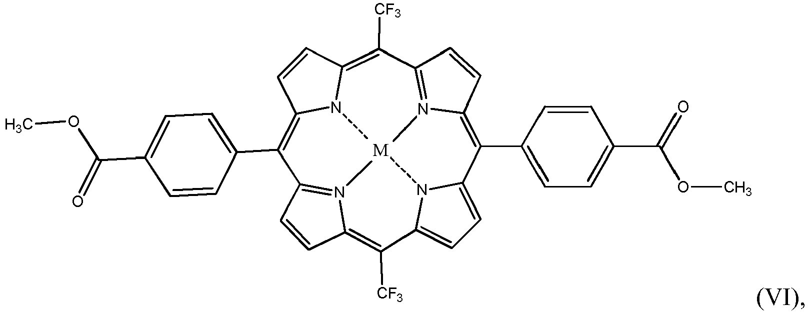 wherein M is a metal. In some embodiments M is a metal ion. In some embodiments M is selected from the group consisting of manganese, iron, cobalt, copper, nickel, zinc, chromium, aluminium and magnesium. In som embodiments M is manganese.

A second aspect provides a pharmaceutical composition comprising a compound of the first aspect or a pharmaceutically acceptable salt thereof and a pharmaceutically acceptable excipient.

A third aspect provides a compound of the first aspect or a composition of the second aspect for use as a pharmaceutical.

A fourth aspect provides a compound of the first aspect or a composition of the second aspect for use in treating a neurodegenerative disorder. In some embodiments, said neurodegenerative disorder is an acute or chronic neurodegenerative disease. In some embodiments, said neurodegenerative disorder in Parkinson's disease, Alzheimer's disease, Pick's disease, Huntington's disease, amyotrophic lateral sclerosis, prion diseases, dystonia, dementia with Lewy bodies, multiple system atrophy, progressive supranuclear palsy, Friedreich's Ataxia, temporal lope epilepsy, stroke, traumatic brain injury, or a mitochondrial encephalopathy. In some embodiments, said neuorodegenerative disorder is Parkinson's disease.

### BRIEF DESCRIPTION OF THE DRAWINGS

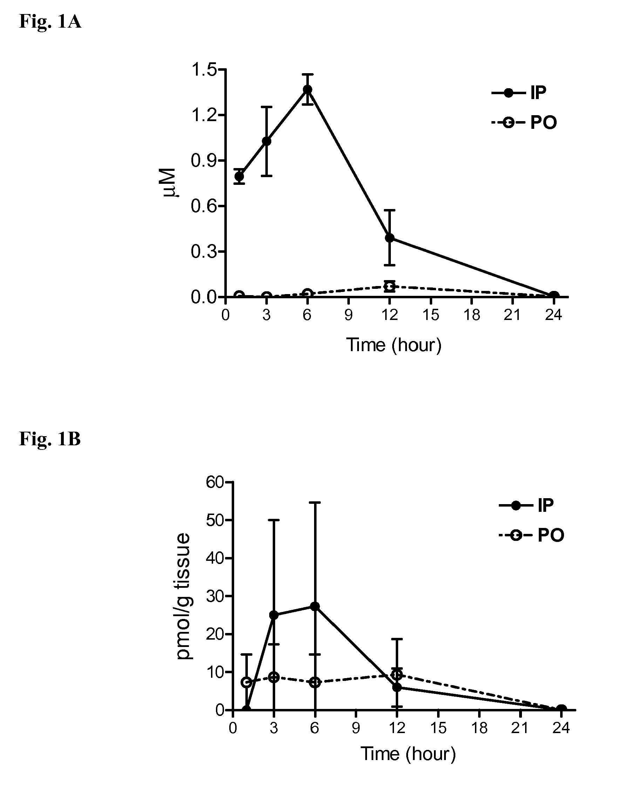

**Figure 1****.** The concentration of 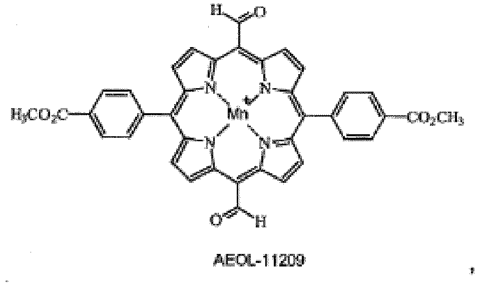 in the plasma (Fig.1A) and brains (Fig.1B) of C57BL/6 mice at different times points after a single dose of AEOL11209 (15mg/kg) administered by the i.p or p.o. route. Points represent mean ± S.E.M. Each point is the average of 3-4 animals.

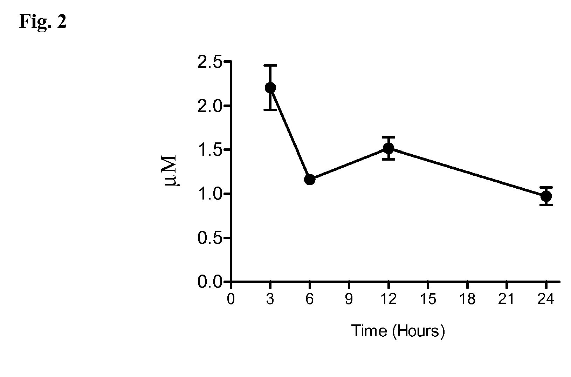

**Figure 2****.** The concentration of 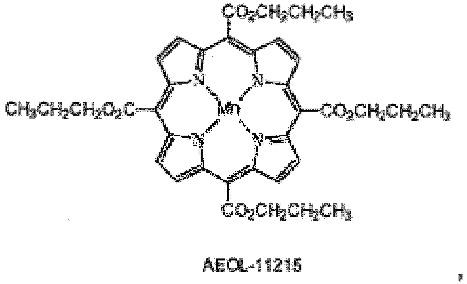 in the plasma of the C57BL/6 mice at different times points after a single dose of AEOL11215 (15mg/kg) administered by the i.p route. Points represent mean ± S.E.M. Each point is the average of 3-4 animals.

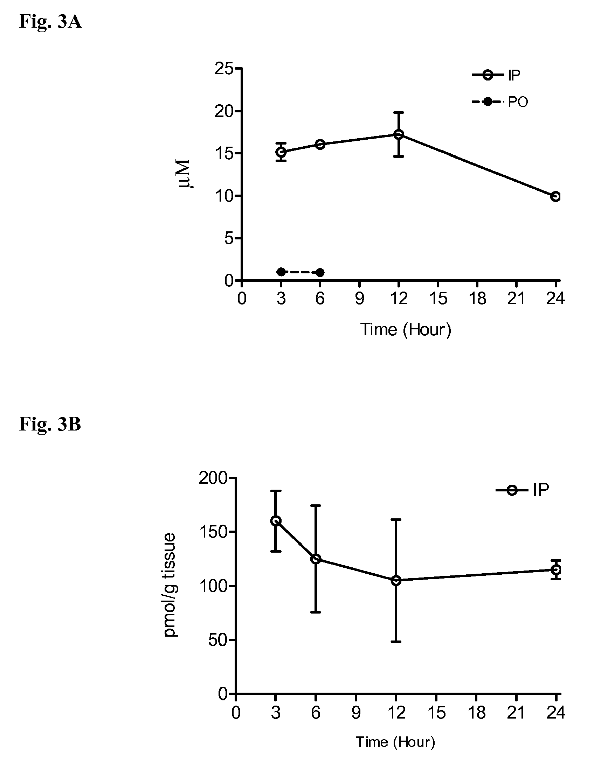

**Figure 3****.** The concentration of 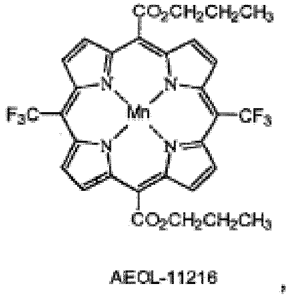 in the plasma (Fig.3A) and brains (Fig.3B) of C57BL/6 mice at different times points after a single dose of AEOL11216 (15mg/kg) administered by the i.p. and p.o route. Points represent mean + S.E.M. Bars represent mean + S.E.M. n=3-6 mice per group.

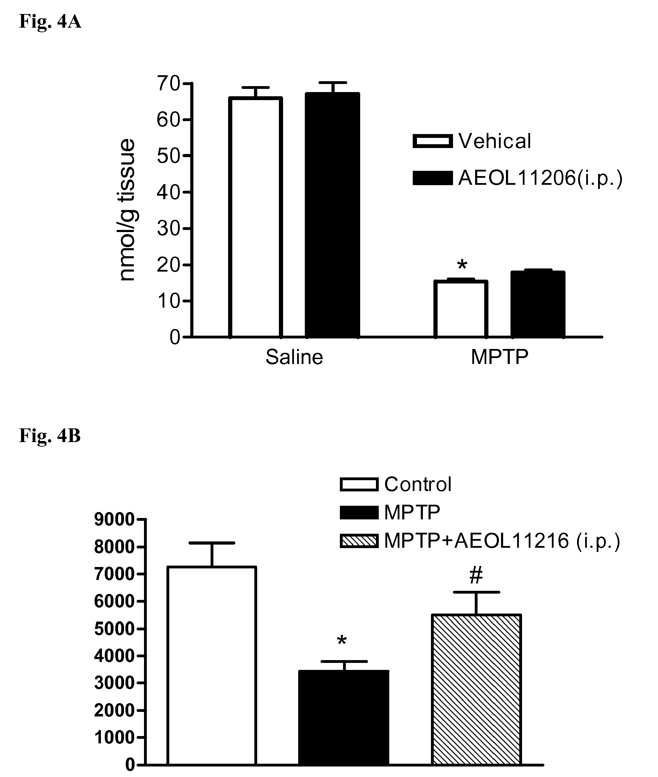

**Figure 4****.** The histogram of Figure 4A appears in the order (left to right): vehicle; AEOL 11206 (i.p.). The histogram of Figure 4B appears in the order (left to right): control; MPTP; MPTP+AEOL 11206 (i.p.). Dopamine levels in the striata (Fig.4A) number of TH+ neurons in the substantia nigra pars compacta (Fig.4B) of C57BL/6 mice 7 days after injection of MPTP (15mg/kgx3, s.c., 24h intervals) alone or in the presence of AEOL11216 15 mg/kg i.p. (1 hr prior to MPTP and daily thereafter). Bars represent mean + S.E.M. n=3-6 mice per group.

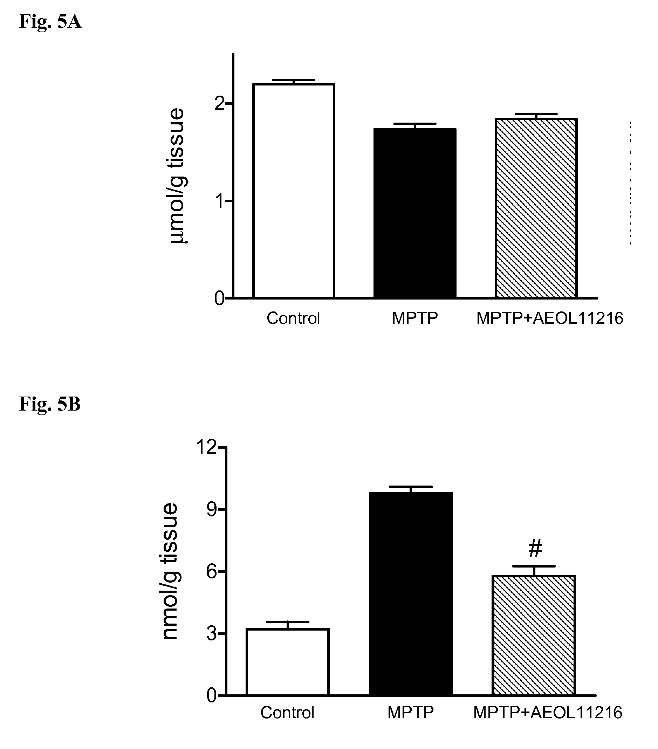

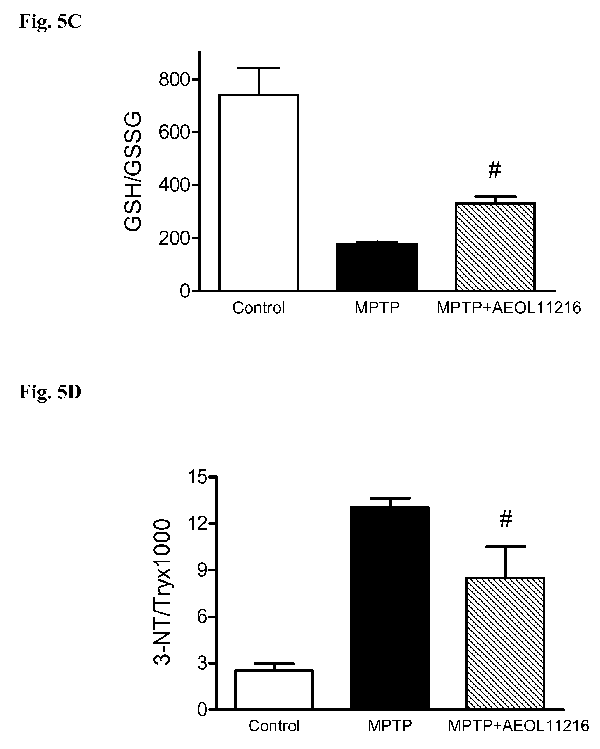

**Figure 5****.** The histograms of Figures 5A-5D appear in the order (left to right): control; MPTP; MPTP+AEOL 11216. GSH, GSSG, GSH/GSSG and 3NT/Tyr ratios in the ventral midbrains of C57/B6 mice 24 hours after injection of MPTP (15mg/kgx3, s.c., 24h intervals) alone or in the presence of AEOL11216 i.p. (15mg/kgx3, daily). Bars represent mean + S.E.M. # p<0.01 vs MPTP. One-way, ANOVA, n=6 mice per group.

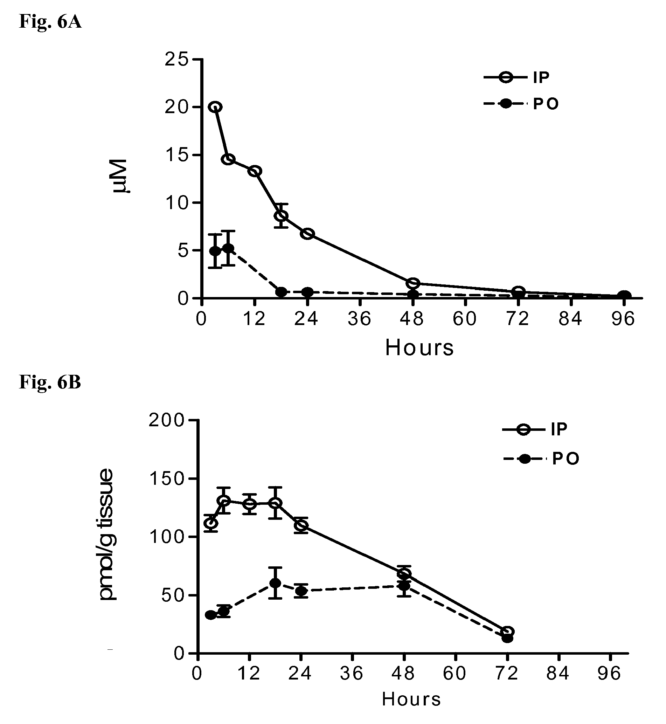

**Figure 6****.** The concentration of 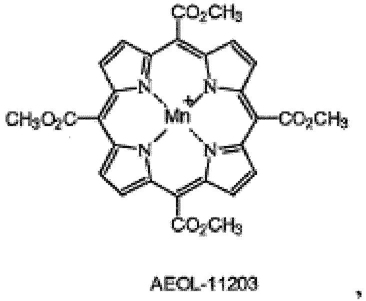 in the plasma (Fig.6A) and brains (Fig.6B) of C57BL/6 mice at different times points after a single dose of AEOL11203 (15mg/kg) administered by the i.p. and p.o. route. Points represent mean ± S.E.M. Each point is the average of 3-4 animals.

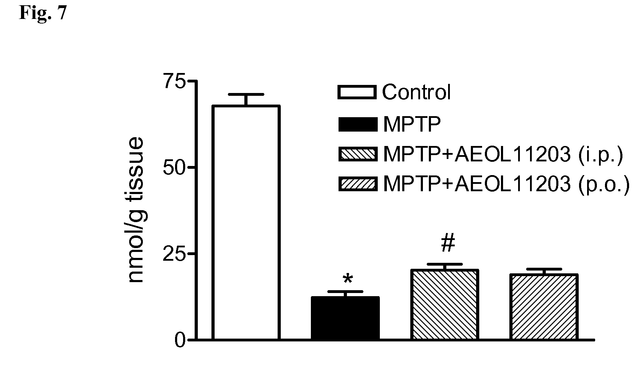

**Figure 7****.** The histogram of Figure 7 appears in the order (left to right): control; MPTP; MPTP+AEOL 11203 (i.p.); MPTP+AEOL 11203 (i.o.). Dopamine levels in the striata of C57BL/6 mice 7 days after injection of MPTP (15mg/kgx3, s.c., 24h intervals) alone or in the presence of AEOL11203 15 mg/kg i.p or p.o. (1 hr prior to MPTP and daily thereafter). Bars represent mean + S.E.M. n=6-8 mice per group.

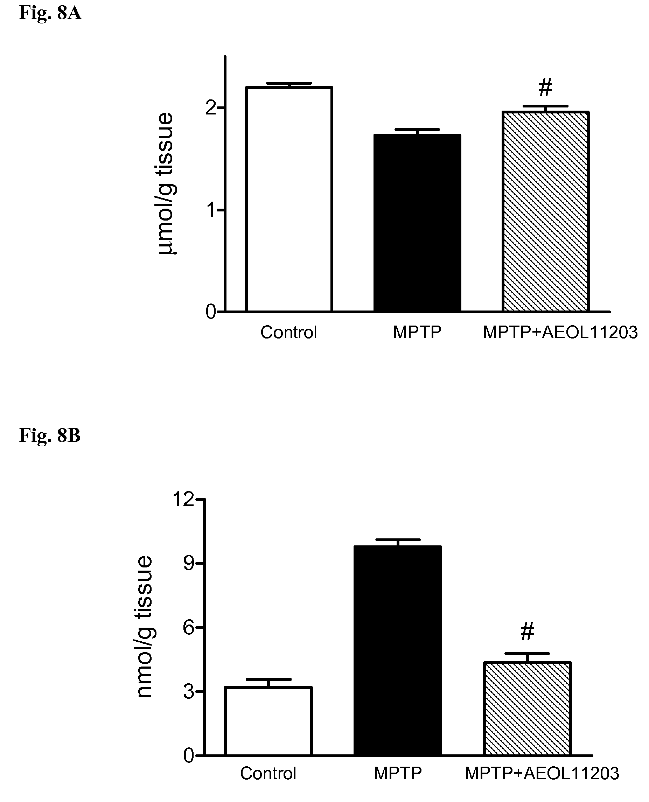

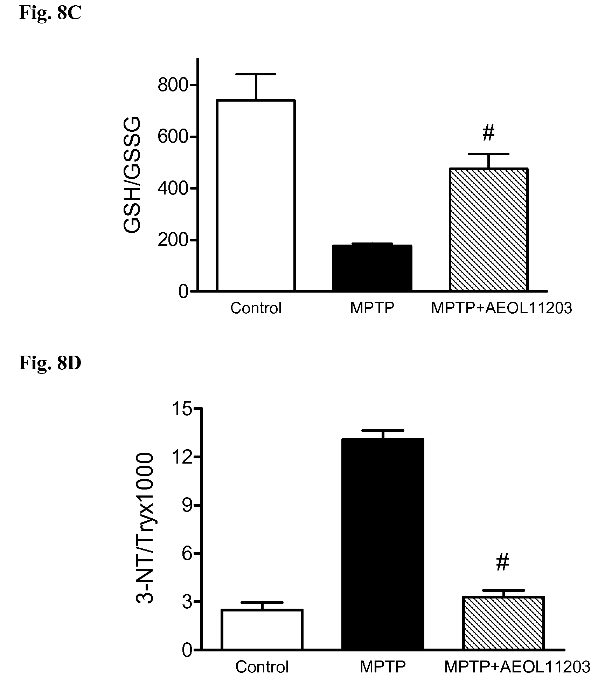

**Figure 8****.** The histograms of Figures 8A-8D appear in the order (left to right): control; MPTP; MPTP+AEOL 11203. GSH, GSSG, GSH/GSSG and 3NT/Tyr ratios in the SN of C57/B6 mice 24 hours after injection of MPTP (15mg/kgx3, s.c., 24h intervals) alone or in the presence of AEOL11203 i.p. (15mg/kgx3, daily). Bars represent mean + S.E.M. # p<0.01 vs MPTP. One-way, ANOVA, n=6 mice per group.

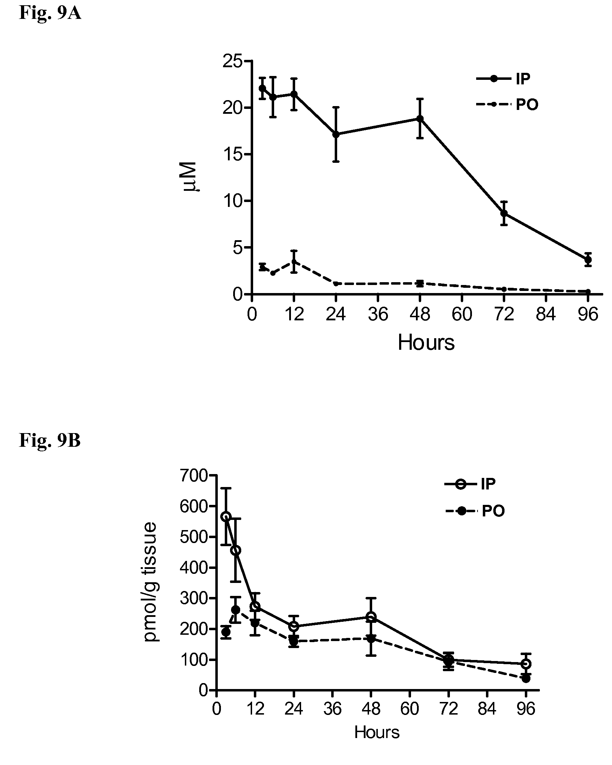

**Figure 9****.** The concentration of 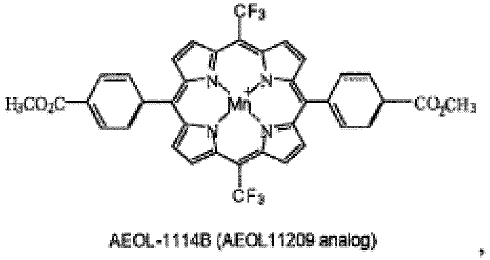 in the plasma (Fig.9A) and brains (Fig.9B) of C57BL/6 mice at different times points after a single dose of AEOL1114 (15mg/kg) administered by the i.p. or p.o. route. Points represent mean + S.E.M. Each point is the average of 4 animals.

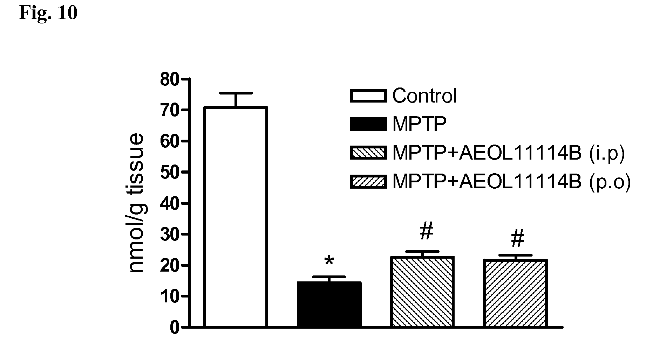

**Figure 10****.** The histogram of Figure 10 appears in the order (left to right): control; MPTP; MPTP+AEOL 11114B (i.p.); MPTP+AEOL 11114B (i.o.). Dopamine levels in the striata of C57BL/6 mice 7 days after injection of MPTP (15mg/kgx3, s.c., 24h intervals) alone or in the presence of AEOL 1114B 15 mg/kg i.p or p.o. (1 hr prior to MPTP and daily thereafterx3). Bars represent mean + S.E.M. n=6 mice per group.

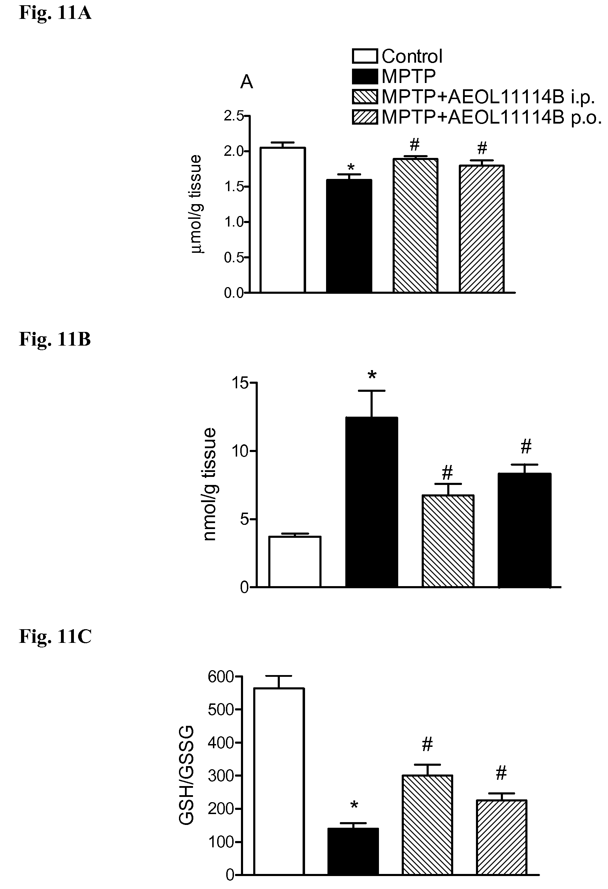

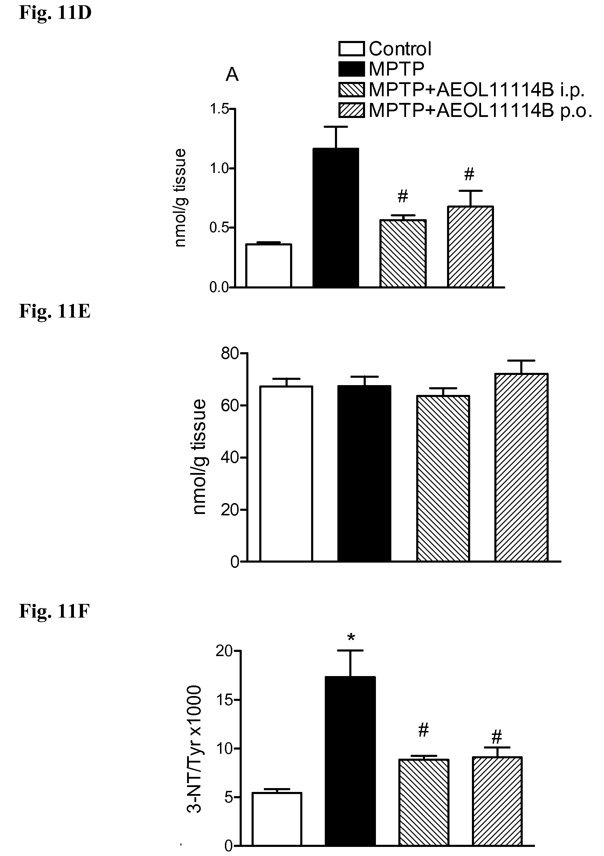

**Figure 11****.** The histograms of Figures 11A-11F appear in the order (left to right): control; MPTP; MPTP+AEOL 11114B (i.p.); MPTP+AEOL 11114B (i.o.). GSH, GSSG, GSH/GSSG and 3NT/Tyr ratios in the ventral midbrains of C57/B6 mice 24 hours after last injection of MPTP (15mg/kgx3, s.c., 24h intervals) alone or in the presence of AEOL1114 i.p. or p.o. (15mg/kgx3, daily). Bars represent mean + S.E.M. # p<0.01 vs MPTP. One-way, ANOVA, n=6 mice per group.

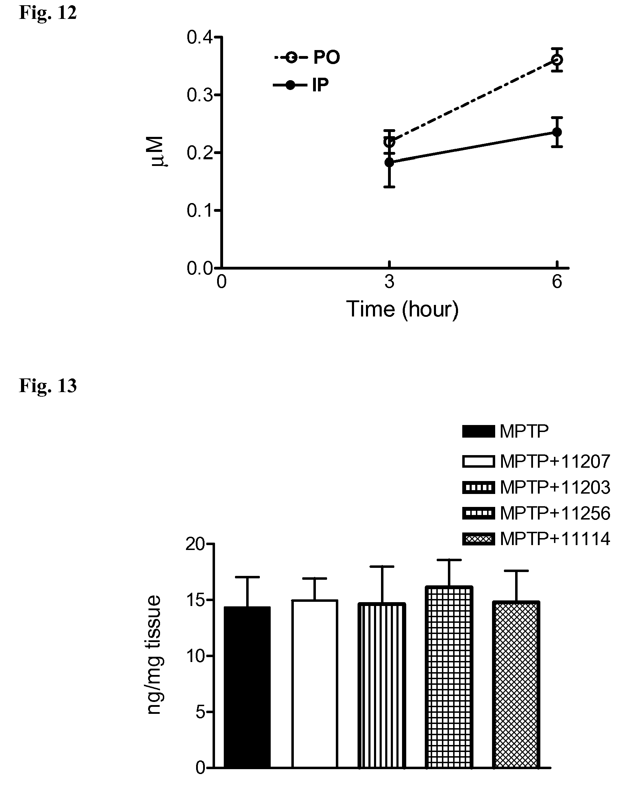

**Figure 12****.** The concentration of 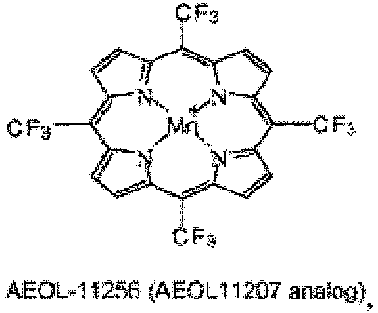 in the plasma of C57BL/6 mice at different times points after a single dose of AEOL11256 (15mg/kg) administered by the i.p. or p.o. route. Points represent mean + S.E.M. Each point is the average of 4 animals.
**Figure 13****.** The histogram of Figure 13 appears in the order (left to right): MPTP; MPTP+AEOL 11207; MPTP+AEOL 11203; MPTP+AEOL 11256; MPTP+AEOL 11114. Striatal MPP+ levels in mice at 3 h after MPTP (15mg/kg, s.c.) alone or in the presence of AEOL11207, 11203, 11256 and 1114B (15mg/kg, i.p.) administered 1h before MPTP treatment. Bars represent mean + S.E.M. n=4-6 mice per group.

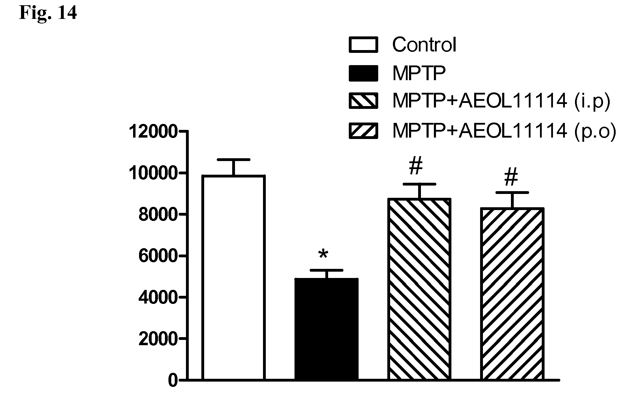

**Figure 14****.** The histogram of Figure 14 appears in the order (left to right): control; MPTP; MPTP+AEOL 11114 (i.p.); MPTP+AEOL 11114 (i.o.). Tyrosine hydroxylase positive (TH+) cell counts indicative of dopaminergic neuron viability in the substantia nigra pars compacta of C57BL/6 mice 7 days after injection of MPTP (15mg/kgx3, s.c., 24h intervals) alone or in the presence of AEOL1114B 15 mg/kg i.p or p.o. (1 hr prior to MPTP and daily thereafterx3). Bars represent mean + S.E.M. n=6.

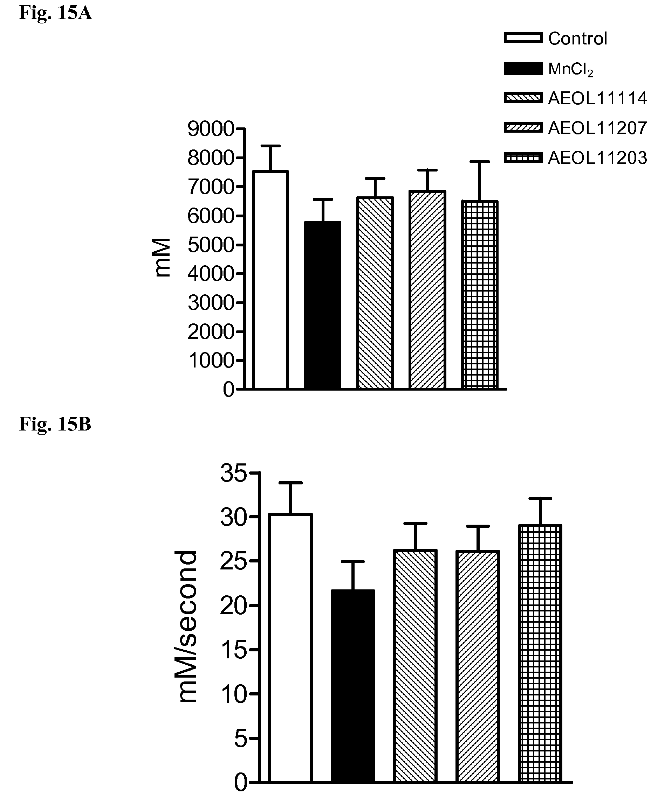

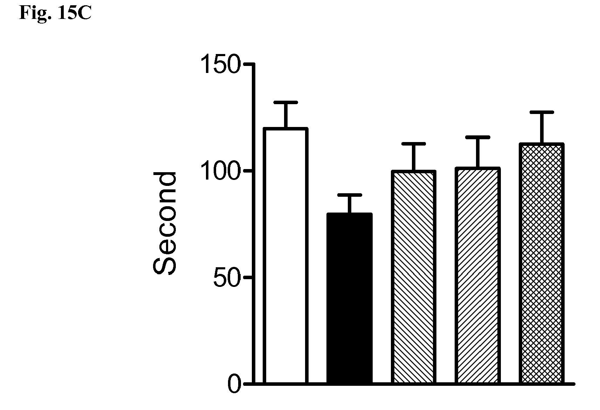

**Figure 15****.** The histograms of Figures 15A-15C appear in the order (left to right): control; MnCl₂; AEOL 11114; AEOL 11207; AEOL 11203. Behavioral testing after dosing mice with AEOL11207, AEOL11114B or AEOL11203 (MnCl2 positive control). Mice (6 per group) were divided 5 groups treated with AEOL11207, AEOL11203, AEOL11114B, MnCl₂ (all compound dissolved in 5% DMSO) or 5%DMSO alone by 15mg/kg i.p. daily for a period of 7 days. At the end of treatment, mice were performed the behavioral tests by open field and accelerating rotorod test in 3-4h after last injection. The total moving distance (Fig.15A), moving velocity (Fig.15B) in 5 minutes measured by open field test and the latency (seconds) from acceleration to fall from the rod (Fig.15C) in the mice 7 days after injection of vehicle, MnCl₂, AEOL11114, AEOL11207 and AEOL11203 i.p. (15mg/kg daily). Bars represent mean + S.E.M. One-way, ANOVA, n=6-8 mice per group.

### DETAILED DESCRIPTION OF THE INVENTION

### I. Definitions

The abbreviations used herein have their conventional meaning within the chemical and biological arts. The chemical structures and formulae set forth herein are constructed according to the standard rules of chemical valency known in the chemical arts.

Where substituent groups are specified by their conventional chemical formulae, written from left to right, they equally encompass the chemically identical substituents that would result from writing the structure from right to left, e.g., -CH₂O- is equivalent to -OCH₂-.

The term "alkyl," by itself or as part of another substituent, means, unless otherwise stated, a straight (i.e., unbranched) or branched chain, or combination thereof, which may be fully saturated, mono- or polyunsaturated and can include di- and multivalent radicals, having the number of carbon atoms designated (i.e., C₁-C₁₀ means one to ten carbons). Examples of saturated hydrocarbon radicals include, but are not limited to, groups such as methyl, ethyl, n-propyl, isopropyl, n-butyl, t-butyl, isobutyl, sec-butyl, (cyclohexyl)methyl, homologs and isomers of, for example, n-pentyl, n-hexyl, n-heptyl, n-octyl, and the like. An unsaturated alkyl group is one having one or more double bonds or triple bonds. Examples of unsaturated alkyl groups include, but are not limited to, vinyl, 2-propenyl, crotyl, 2-isopentenyl, 2-(butadienyl), 2,4-pentadienyl, 3-(1,4-pentadienyl), ethynyl, 1- and 3-propynyl, 3-butynyl, and the higher homologs and isomers. An alkoxy is an alkyl attached to the remainder of the molecule via an oxygen linker (-O-).

The term "alkylene," by itself or as part of another substituent, means, unless otherwise stated, a divalent radical derived from an alkyl, as exemplified, but not limited by, -CH₂CH₂CH₂CH₂-. Typically, an alkyl (or alkylene) group will have from 1 to 24 carbon atoms, with those groups having 10 or fewer carbon atoms being preferred in the present invention. A "lower alkyl" or "lower alkylene" is a shorter chain alkyl or alkylene group, generally having eight or fewer carbon atoms.

The term "heteroalkyl," by itself or in combination with another term, means, unless otherwise stated, a stable straight or branched chain, or combinations thereof, consisting of at least one carbon atom and at least one heteroatom selected from the group consisting of O, N, P, Si, and S, and wherein the nitrogen and sulfur atoms may optionally be oxidized, and the nitrogen heteroatom may optionally be quaternized. The heteroatom(s) O, N, P, S, and Si may be placed at any interior position of the heteroalkyl group or at the position at which the alkyl group is attached to the remainder of the molecule. Examples include, but are not limited to: -CH₂-CH₂-O-CH₃, -CH₂-CH₂-NH-CH₃, -CH₂-CH₂-N(CH₃)-CH₃, -CH₂-S-CH₂-CH₃, -CH₂-CH₂, -S(O)-CH₃, -CH₂-CH₂-S(O)₂-CH₃, -CH=CH-O-CH₃, -Si(CH₃)₃, -CH₂-CH=N-OCH₃, -CH=CH-N(CH₃)-CH₃, -O-CH₃, -O-CH₂-CH₃, and -CN. Up to two heteroatoms may be consecutive, such as, for example, -CH₂-NH-OCH₃.

Similarly, the term "heteroalkylene," by itself or as part of another substituent, means, unless otherwise stated, a divalent radical derived from heteroalkyl, as exemplified, but not limited by, -CH₂-CH₂-S-CH₂-CH₂- and -CH₂-S-CH₂-CH₂-NH-CH₂-. For heteroalkylene groups, heteroatoms can also occupy either or both of the chain termini (e.g., alkyleneoxy, alkylenedioxy, alkyleneamino, alkylenediamino, and the like). Still further, for alkylene and heteroalkylene linking groups, no orientation of the linking group is implied by the direction in which the formula of the linking group is written. For example, the formula -C(O)₂R'- represents both -C(O)₂R'- and -R'C(O)₂-. As described above, heteroalkyl groups, as used herein, include those groups that are attached to the remainder of the molecule through a heteroatom, such as -C(O)R', -C(O)NR', -NR'R", -OR', -SR', and/or -SO₂R'. Where "heteroalkyl" is recited, followed by recitations of specific heteroalkyl groups, such as -NR'R" or the like, it will be understood that the terms heteroalkyl and -NR'R" are not redundant or mutually exclusive. Rather, the specific heteroalkyl groups are recited to add clarity. Thus, the term "heteroalkyl" should not be interpreted herein as excluding specific heteroalkyl groups, such as -NR'R" or the like.

The terms "cycloalkyl" and "heterocycloalkyl," by themselves or in combination with other terms, mean, unless otherwise stated, cyclic versions of "alkyl" and "heteroalkyl," respectively. Additionally, for heterocycloalkyl, a heteroatom can occupy the position at which the heterocycle is attached to the remainder of the molecule. Examples of cycloalkyl include, but are not limited to, cyclopropyl, cyclobutyl, cyclopentyl, cyclohexyl, 1-cyclohexenyl, 3-cyclohexenyl, cycloheptyl, and the like. Examples of heterocycloalkyl include, but are not limited to, 1-(1,2,5,6-tetrahydropyridyl), 1-piperidinyl, 2-piperidinyl, 3-piperidinyl, 4-morpholinyl, 3-morpholinyl, tetrahydrofuran-2-yl, tetrahydrofuran-3-yl, tetrahydrothien-2-yl, tetrahydrothien-3-yl, 1-piperazinyl, 2-piperazinyl, and the like. A "cycloalkylene" and a "heterocycloalkylene," alone or as part of another substituent, means a divalent radical derived from a cycloalkyl and heterocycloalkyl, respectively.

The terms "halo" or "halogen," by themselves or as part of another substituent, mean, unless otherwise stated, a fluorine, chlorine, bromine, or iodine atom. Additionally, terms such as "haloalkyl" are meant to include monohaloalkyl and polyhaloalkyl. For example, the term "halo(C₁-C₄)alkyl" includes, but is not limited to, fluoromethyl, difluoromethyl, trifluoromethyl, 2,2,2-trifluoroethyl, 4-chlorobutyl, 3-bromopropyl, and the like.

The term "acyl" means, unless otherwise stated, -C(O)R where R is a substituted or unsubstituted alkyl, substituted or unsubstituted cycloalkyl, substituted or unsubstituted heteroalkyl, substituted or unsubstituted heterocycloalkyl, substituted or unsubstituted aryl, or substituted or unsubstituted heteroaryl.

The term "aryl" means, unless otherwise stated, a polyunsaturated, aromatic, hydrocarbon substituent, which can be a single ring or multiple rings (e.g. from 1 to 3 rings) that are fused together (i.e., a fused ring aryl) or linked covalently. A fused ring aryl refers to multiple rings fused together wherein at least one of the fused rings is an aryl ring. The term "heteroaryl" refers to aryl groups (or rings) that contain at least one heteroatom selected from N, O, and S, wherein the nitrogen and sulfur atoms are optionally oxidized, and the nitrogen atom(s) are optionally quaternized. Thus, the term "heteroaryl" includes fused ring heteroaryl groups (i.e., multiple rings fused together wherein at least one of the fused rings is a heteroaromatic ring). A 5,6-fused ring heteroaryl refers to two rings fused together, wherein one ring has 5 members and the other ring has 6 members, and wherein at least one ring is a heteroaryl ring. Likewise, a 6,6-fused ring heteroaryl refers to two rings fused together, wherein one ring has 6 members and the other ring has 6 members, and wherein at least one ring is a heteroaryl ring. And a 6,5-fused ring heteroaryl refers to two rings fused together, wherein one ring has 6 members and the other ring has 5 members, and wherein at least one ring is a heteroaryl ring. A heteroaryl group can be attached to the remainder of the molecule through a carbon or heteroatom. Non-limiting examples of aryl and heteroaryl groups include phenyl, 1-naphthyl, 2-naphthyl, 4-biphenyl, 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, 4-pyridyl, 2-pyrimidyl, 4-pyrimidyl, 5-benzothiazolyl, purinyl, 2-benzimidazolyl, 5-indolyl, 1-isoquinolyl, 5-isoquinolyl, 2-quinoxalinyl, 5-quinoxalinyl, 3-quinolyl, and 6-quinolyl. Substituents for each of the above noted aryl and heteroaryl ring systems are selected from the group of acceptable substituents described below. An "arylene" and a "heteroarylene," alone or as part of another substituent, mean a divalent radical derived from an aryl and heteroaryl, respectively.

The term "oxo," as used herein, means an oxygen that is double bonded to a carbon atom.

The term "alkylsulfonyl," as used herein, means a moiety having the formula -S(O₂)-R', where R' is an alkyl group as defined above. R' may have a specified number of carbons (e.g., "C₁-C₄ alkylsulfonyl").

Each of the above terms (e.g., "alkyl," "heteroalkyl," "aryl," and "heteroaryl") includes both substituted and unsubstituted forms of the indicated radical. Preferred substituents for each type of radical are provided below.

Substituents for the alkyl and heteroalkyl radicals (including those groups often referred to as alkylene, alkenyl, heteroalkylene, heteroalkenyl, alkynyl, cycloalkyl, heterocycloalkyl, cycloalkenyl, and heterocycloalkenyl) are as disclosed herein or can be one or more of a variety of groups selected from, but not limited to, -OR', =O, =NR', =N-OR', -NR'R", -SR', -halogen, -SiR'R"R"', -OC(O)R', -C(O)R', -CO₂R', -CONR'R", -OC(O)NR'R", -NR"C(O)R', -NR'-C(O)NR"R"', -NR"C(O)₂R', -NR-C(NR'R"R"')=NR"", -NR-C(NR'R")=NR"', -S(O)R', -S(O)₂R', -S(O)₂NR'R", -NRSO₂R', -CN, and -NO₂ in a number ranging from zero to (2m'+1), where m' is the total number of carbon atoms in such radical. R', R", R"', and R"" each preferably independently refer to hydrogen, substituted or unsubstituted heteroalkyl, substituted or unsubstituted cycloalkyl, substituted or unsubstituted heterocycloalkyl, substituted or unsubstituted aryl (e.g., aryl substituted with 1-3 halogens), substituted or unsubstituted alkyl, alkoxy, or thioalkoxy groups, or arylalkyl groups. When a compound disclosed herein includes more than one R group, for example, each of the R groups is independently selected as are each R', R", R"', and R"" group when more than one of these groups is present. When R' and R" are attached to the same nitrogen atom, they can be combined with the nitrogen atom to form a 4-, 5-, 6-, or 7-membered ring. For example, -NR'R" includes, but is not limited to, 1-pyrrolidinyl and 4-morpholinyl. From the above discussion of substituents, one of skill in the art will understand that the term "alkyl" is meant to include groups including carbon atoms bound to groups other than hydrogen groups, such as haloalkyl (e.g., -CF₃ and -CH₂CF₃) and acyl (e.g., -C(O)CH₃, -C(O)CF₃, -C(O)CH₂OCH₃, and the like).

Similar to the substituents described for the alkyl radical, substituents for the aryl and heteroaryl groups are varied and are disclosed herein or may be selected from, for example: -OR', -NR'R", -SR', -halogen, -SiR'R"R"', -OC(O)R', -C(O)R', -CO₂R', -CONR'R", -OC(O)NR'R", -NR"C(O)R', -NR'-C(O)NR"R"', -NR"C(O)₂R', -NR-C(NR'R"R"')=NR"", -NR-C(NR'R")=NR"', -S(O)R', -S(O)₂R, -S(O)₂NR'R", -NRSO₂R', -CN, -NO₂, -R', -N₃, -CH(Ph)₂, fluoro(C₁-C₄)alkoxy, and fluoro(C₁-C₄)alkyl, in a number ranging from zero to the total number of open valences on the aromatic ring system; and where R', R", R"', and R"" are preferably independently selected from hydrogen, substituted or unsubstituted alkyl, substituted or unsubstituted heteroalkyl, substituted or unsubstituted cycloalkyl, substituted or unsubstituted heterocycloalkyl, substituted or unsubstituted aryl, and substituted or unsubstituted heteroaryl. When a compound includes more than one R group, for example, each of the R groups is independently selected as are each R', R", R"', and R"" groups when more than one of these groups is present.

Two or more substituents may optionally be joined to form aryl, heteroaryl, cycloalkyl, or heterocycloalkyl groups. Such so-called ring-forming substituents are typically, though not necessarily, found attached to a cyclic base structure. The ring-forming substituents may be attached to adjacent members of the base structure. For example, two ring-forming substituents attached to adjacent members of a cyclic base structure create a fused ring structure. The ring-forming substituents may be attached to a single member of the base structure. For example, two ring-forming substituents attached to a single member of a cyclic base structure create a spirocyclic structure. The ring-forming substituents may be attached to non-adjacent members of the base structure.

Two of the substituents on adjacent atoms of the aryl or heteroaryl ring may optionally form a ring of the formula -T-C(O)-(CRR')_{q}-U-, wherein T and U are independently -NR-, -O-, -CRR'-, or a single bond, and q is an integer of from 0 to 3. Alternatively, two of the substituents on adjacent atoms of the aryl or heteroaryl ring may optionally be replaced with a substituent of the formula -A-(CH₂)ᵣ-B-, wherein A and B are independently -CRR'-, -O-, -NR-, -S-, -S(O) -, -S(O)₂-, -S(O)₂NR'-, or a single bond, and r is an integer of from 1 to 4. One of the single bonds of the new ring so formed may optionally be replaced with a double bond. Alternatively, two of the substituents on adjacent atoms of the aryl or heteroaryl ring may optionally be replaced with a substituent of the formula -(CRR')ₛ-X'- (C"R"')_{d}-, where s and d are independently integers of from 0 to 3, and X' is -O-, -NR'-, -S-, -S(O)-, -S(O)₂-, or -S(O)₂NR'-. The substituents R, R', R", and R"' are preferably independently selected from hydrogen, substituted or unsubstituted alkyl, substituted or unsubstituted cycloalkyl, substituted or unsubstituted heterocycloalkyl, substituted or unsubstituted aryl, and substituted or unsubstituted heteroaryl.

As used herein, the terms "heteroatom" or "ring heteroatom" are meant to include oxygen (O), nitrogen (N), sulfur (S), phosphorus (P), and silicon (Si).

A "substituent group," as used herein, means a group selected from the following moieties:
(A) -OH, -NH₂, -SH, -CN, -CF₃, -NO₂, oxo, halogen, unsubstituted alkyl, unsubstituted heteroalkyl, unsubstituted cycloalkyl, unsubstituted heterocycloalkyl, unsubstituted aryl, unsubstituted heteroaryl, and
(B) alkyl, heteroalkyl, cycloalkyl, heterocycloalkyl, aryl, and heteroaryl, substituted with at least one substituent selected from:
   (i) oxo, -OH, -NH₂, -SH, -CN, -CF₃, -NO₂, halogen, unsubstituted alkyl, unsubstituted heteroalkyl, unsubstituted cycloalkyl, unsubstituted heterocycloalkyl, unsubstituted aryl, unsubstituted heteroaryl, and
   (ii) alkyl, heteroalkyl, cycloalkyl, heterocycloalkyl, aryl, and heteroaryl, substituted with at least one substituent selected from:
      (a) oxo, -OH, -NH₂, -SH, -CN, -CF₃, -NO₂, halogen, unsubstituted alkyl, unsubstituted heteroalkyl, unsubstituted cycloalkyl, unsubstituted heterocycloalkyl, unsubstituted aryl, unsubstituted heteroaryl, and
      (b) alkyl, heteroalkyl, cycloalkyl, heterocycloalkyl, aryl, or heteroaryl, substituted with at least one substituent selected from: oxo, -OH, -NH₂, -SH, -CN, -CF₃, -NO₂, halogen, unsubstituted alkyl, unsubstituted heteroalkyl, unsubstituted cycloalkyl, unsubstituted heterocycloalkyl, unsubstituted aryl, and unsubstituted heteroaryl.

A "size-limited substituent" or " size-limited substituent group," as used herein, means a group selected from all of the substituents described above for a "substituent group," wherein each substituted or unsubstituted alkyl is a substituted or unsubstituted C₁-C₂₀ alkyl, each substituted or unsubstituted heteroalkyl is a substituted or unsubstituted 2 to 20 membered heteroalkyl, each substituted or unsubstituted cycloalkyl is a substituted or unsubstituted C₄-C₈ cycloalkyl, and each substituted or unsubstituted heterocycloalkyl is a substituted or unsubstituted 4 to 8 membered heterocycloalkyl.

A "lower substituent" or " lower substituent group," as used herein, means a group selected from all of the substituents described above for a "substituent group," wherein each substituted or unsubstituted alkyl is a substituted or unsubstituted C₁-C₈ alkyl, each substituted or unsubstituted heteroalkyl is a substituted or unsubstituted 2 to 8 membered heteroalkyl, each substituted or unsubstituted cycloalkyl is a substituted or unsubstituted C₅-C₇ cycloalkyl, and each substituted or unsubstituted heterocycloalkyl is a substituted or unsubstituted 5 to 7 membered heterocycloalkyl.

Unless otherwise stated, structures depicted herein are also meant to include all stereochemical forms of the structure; i.e., the R and S configurations for each asymmetric center. Therefore, single stereochemical isomers as well as enantiomeric and diastereomeric mixtures of the present compounds are within the scope of the invention.

Unless otherwise stated, structures depicted herein are also meant to include compounds which differ only in the presence of one or more isotopically enriched atoms. For example, compounds having the present structures except for the replacement of a hydrogen by a deuterium or tritium, or the replacement of a carbon by ¹³C- or ¹⁴C-enriched carbon are within the scope of this invention.

The compounds of the present invention may also contain unnatural proportions of atomic isotopes at one or more of atoms that constitute such compounds. For example, the compounds may be radiolabeled with radioactive isotopes, such as for example tritium (³H), iodine-125 (¹²⁵I) or carbon-14 (¹⁴C). All isotopic variations of the compounds of the present invention, whether radioactive or not, are encompassed within the scope of the present invention.

The terms "a," "an," or "a(n)", when used in reference to a group of substituents herein, mean at least one. For example, where a compound is substituted with "an" alkyl or aryl, the compound is optionally substituted with at least one alkyl and/or at least one aryl. Moreover, where a moiety is substituted with an R substituent, the group may be referred to as "R-substituted." Where a moiety is R-substituted, the moiety is substituted with at least one R substituent and each R substituent is optionally different.

Description of compounds of the disclosure are limited by principles of chemical bonding known to those skilled in the art. Accordingly, where a group may be substituted by one or more of a number of substituents, such substitutions are selected so as to comply with principles of chemical bonding and to give compounds which are not inherently unstable and/or would be known to one of ordinary skill in the art as likely to be unstable under ambient conditions, such as aqueous, neutral, and several known physiological conditions. For example, a heterocycloalkyl or heteroaryl is attached to the remainder of the molecule via a ring heteroatom in compliance with principles of chemical bonding known to those skilled in the art thereby avoiding inherently unstable compounds.

The term "effective amount" or "therapeutically effective amount" refers to the amount of an active agent sufficient to induce a desired biological result. That result may be alleviation of the signs, symptoms, or causes of a disease, or any other desired alteration of a biological system. The term "therapeutically effective amount" is used herein to denote any amount of the formulation which causes a substantial improvement in a disease condition when applied to the affected areas repeatedly over a period of time. The amount will vary with the condition being treated, the stage of advancement of the condition, and the type and concentration of formulation applied. Appropriate amounts in any given instance will be readily apparent to those skilled in the art or capable of determination by routine experimentation.

As used herein, "treatment" or "treating," or "palliating" or "ameliorating" are used interchangeably herein. These terms refer to an approach for obtaining beneficial or desired results including but not limited to therapeutic benefit and/or a prophylactic benefit. By therapeutic benefit is meant eradication or amelioration of the underlying disorder being treated. Also, a therapeutic benefit is achieved with the eradication or amelioration of one or more of the physiological symptoms associated with the underlying disorder such that an improvement is observed in the patient, notwithstanding that the patient may still be afflicted with the underlying disorder. For prophylactic benefit, the compositions may be administered to a patient at risk of developing a particular disease, or to a patient reporting one or more of the physiological symptoms of a disease, even though a diagnosis of this disease may not have been made. Treatment includes preventing the disease, that is, causing the clinical symptoms of the disease not to develop by administration of a protective composition prior to the induction of the disease; suppressing the disease, that is, causing the clinical symptoms of the disease not to develop by administration of a protective composition after the inductive event but prior to the clinical appearance or reappearance of the disease; inhibiting the disease, that is, arresting the development of clinical symptoms by administration of a protective composition after their initial appearance; preventing re-occurring of the disease and/or relieving the disease, that is, causing the regression of clinical symptoms by administration of a protective composition after their initial appearance.

The term "pharmaceutically acceptable salt" refers to salts derived from a variety of organic and inorganic counter ions well known in the art and include, by way of example only, sodium, potassium, calcium, magnesium, ammonium, tetraalkylammonium, and the like; and when the molecule contains a basic functionality, salts of organic or inorganic acids, such as hydrochloride, hydrobromide, tartrate, mesylate, acetate, maleate, oxalate and the like.

A "subject," "individual," or "patient," is used interchangeably herein, which refers to a vertebrate, preferably a mammal, more preferably a human. Mammals include, but are not limited to, murines, simians, humans, farm animals, sport animals, and pets. Tissues, cells and their progeny of a biological entity obtained *in vitro* or cultured *in vitro* are also encompassed.

Where a moiety herein is R-substituted (e.g. "R^{12A}-substituted or unsubstituted alkyl"), that moity may be substituted with a plurality of R groups that are each optionally different (e.g. a plurality of R^{12A} substituents may be attached to the alkyl moiety wherein each R^{12A} substituent is optionally different). Moreover, where more than one R group is present (e.g. more than one R^{17D}), each R group is optionally different (e.g. each R^{17D} group is independently one fo the subistutents provided in its definition or Markush group).

### II. Compositions

In accordance with the disclosure, the compound useful in the methods provided herein is a porphyrin compound, such as a metalloporphyrin. The compound may have the formula:

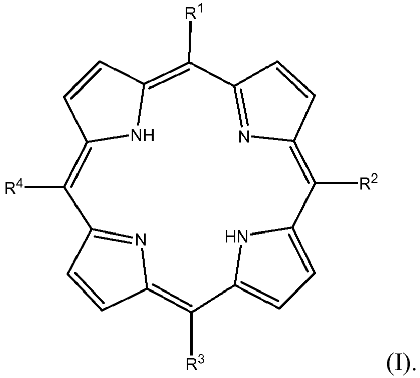

In Formula I, the substituted porphyrin may be bound to a metal. The metal may be manganese, iron, cobalt, copper, nickel, zinc, chromium, aluminum, or magnesium, including ions thereof (e.g., Mn²⁺, Mn³⁺, Fe²⁺, Fe³⁺, Co²⁺, Cu²⁺, Ni²⁺, Zn²⁺, Cr²⁺, Cr³⁺, Al³⁺, Mg²⁺). The compound of the disclosure may have the formula of Formula (II), wherein M is a metal (e.g. metal ion): 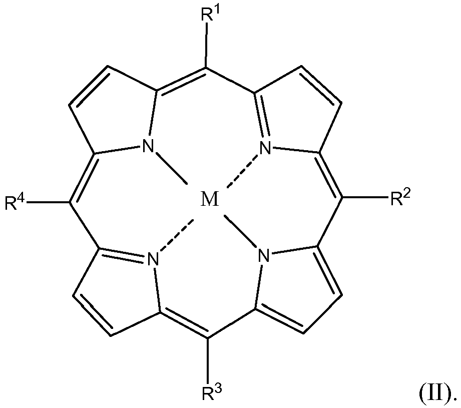 M may be manganese, iron, cobalt, copper, nickel, zinc, chromium, aluminum, or magnesium, including ions thereof (e.g., Mn²⁺, Mn³⁺, Fe²⁺, Fe³⁺, Co²⁺, Cu²⁺, Ni²⁺, Zn²⁺, Cr²⁺, Cr³⁺, Al³⁺, Mg²⁺). In a specific varient, the compound is the compound of formula II, wherein M is manganese. For example, the compound of the disclosure may have the formula:

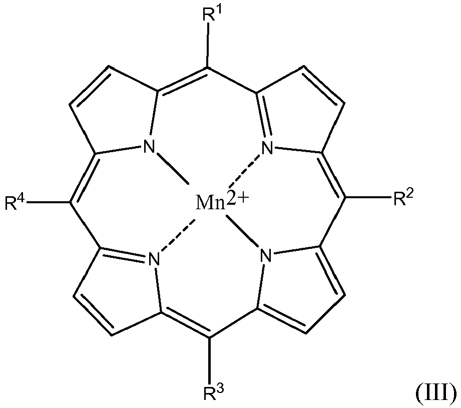

R¹ may be independently -C(X¹)₃, -COOR^{5A}, 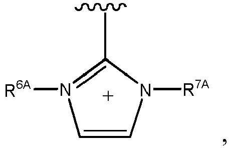 or 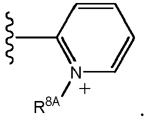 R³ may be independently -C(X³)₃, -COOR^{5B}, 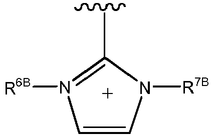 or 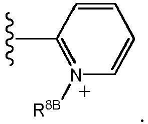 R² may be independently -C(X²)₃, -COOR^{5C}, or 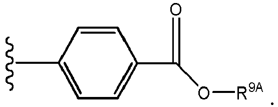 R⁴ may be independently -C(X⁴)₃, -COOR^{5D} or 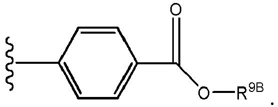 In some compounds of the disclosure, R¹ is -C(X¹)₃ and R³ is -C(X³)₃. In other compounds of the disclosure, R¹ is -COOR^{5A} and R³ is -COOR^{5B}. In some compounds of the disclosure, R¹ is 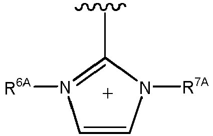 and R³ 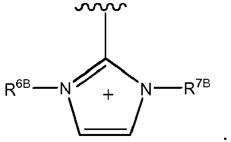 In other compounds of the disclosure, R¹ is 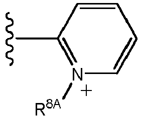 and R³ is 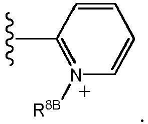 In other compounds of the disclosure, R² is -C(X²)₃ and R⁴ is -C(X⁴)₃. In other compounds of the disclosure, R² is -COOR^{5C} and R⁴ is -COOR^{5D}. In some compounds of the disclosure, R² is 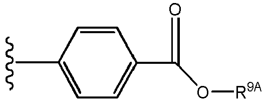 and R⁴ 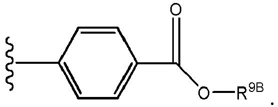 X¹, X², X³ and X⁴ are halogens, such as F, Cl or Br (e.g. F).

In some compounds of the disclosure, R¹, R², R³ and R⁴ are not simultaneously-COOR^{5A}, -COOR^{5B}, -COOR^{5C}, and -COOR^{5D}, respectively. In some compounds of the disclosure, if R² is -COOR^{5C} and R⁴ is -COOR^{5D}, R¹ is not 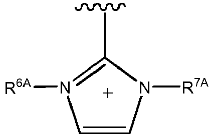 and R³ is not 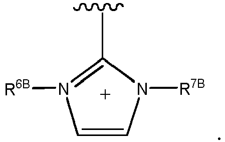 In some compounds of the disclosure, if R² is -C(X²)₃ and R⁴ is -C(X⁴)₃, R¹ is not 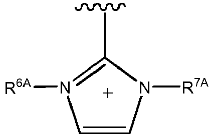 and R³ is not

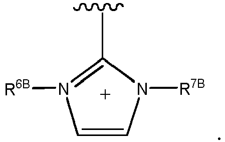

In some compounds of the disclosure, if R¹ and R³ are both -CF₃, then R² and R⁴ are both not -CO₂CH₃. In other words, if R¹ and R³ are both -CF₃, then R² and R⁴ are not simultaneously -CO₂CH₃. In some compounds of the disclosure, if R¹ and R³ are both-CF₃, then R² and R⁴ are both not -CO₂CH₂CH₂CH₃. In other words, if R¹ and R³ are both-CF₃, then R² and R⁴ are not simultaneously -CO₂ CH₂CH₂CH₃.

In some compounds of the disclosure, if R¹ and R³ are both -CF₃, then R² and R⁴ are not COOR^{5C} and COOR^{5D}, respectively, when R^{5C} and R^{5D} are methyl or propyl. In some compounds of the disclosure, if R¹ and R³ are both -CF₃, then R² and R⁴ are not COOR^{5C} and COOR^{5D}, respectively, when R^{5C} and R^{5D} are unsubstituted C₁-C₃ alkyl. In some compounds of the disclosure, if R¹ and R³ are both -CF₃, then R² and R⁴ are not COOR^{5C} and COOR^{5D}, respectively, when R^{5C} and R^{5D} are unsubstituted C₁-C₄ alkyl. In some compounds of the disclosure, if R¹ and R³ are both -CF₃, then R² and R⁴ are not COOR^{5C} and COOR^{5D}, respectively, when R^{5C} and R^{5D} are unsubstituted C₁-C₅ alkyl. In some compounds of the disclosure, if R¹ and R³ are both -CF₃, then R² and R⁴ are not COOR^{5C} and COOR^{5D}, respectively, when R^{5C} and R^{5D} are unsubstituted C₁-C₆ alkyl. In some compounds of the disclosure, if R¹ and R³ are both -CF₃, then R² and R⁴ are not COOR^{5C} and COOR^{5D}, respectively, when R^{5C} and R^{5D} are unsubstituted C₁-C₇ alkyl. In some compounds of the disclosure, if R¹ and R³ are both -CF₃, then R² and R⁴ are not COOR^{5C} and COOR^{5D}, respectively, when R^{5C} and R^{5D} are unsubstituted C₁-C₈ alkyl.

In some compounds of the disclosure, if R¹ and R³ are both -CF₃, then R² and R⁴ are not COOR^{5C} and COOR^{5D}, respectively, when R^{5C} and R^{5D} are substituted or unsubstituted C₁-C₃ alkyl. In some compounds of the disclosure, if R¹ and R³ are both-CF₃, then R² and R⁴ are not COOR^{5C} and COOR^{5D}, respectively, when R^{5C} and R^{5D} are substituted or unsubstituted C₁-C₄ alkyl. In some compounds of the disclosure, if R¹ and R³ are both -CF₃, then R² and R⁴ are not COOR^{5C} and COOR^{5D}, respectively, when R^{5C} and R^{5D} are substituted or unsubstituted C₁-C₅ alkyl. In some compounds of the disclosure, if R¹ and R³ are both -CF₃, then R² and R⁴ are not COOR^{5C} and COOR^{5D}, respectively, when R^{5C} and R^{5D} are substituted or unsubstituted C₁-C₆ alkyl. In some compounds of the disclosure, if R¹ and R³ are both -CF₃, then R² and R⁴ are not COOR^{5C} and COOR^{5D}, respectively, when R^{5C} and R^{5D} are substituted or unsubstituted C₁-C₇ alkyl. In some compounds of the disclosure, if R¹ and R³ are both -CF₃, then R² and R⁴ are not COOR^{5C} and COOR^{5D}, respectively, when R^{5C} and R^{5D} are substituted or unsubstituted C₁-C₈ alkyl.

In some compounds of the disclosure, if R¹ and R³ are both -CF₃, then R² and R⁴ are not COOR^{5C} and COOR^{5D}, respectively, when R^{5C} and R^{5D} are unsubstituted alkyl. In some compounds of the disclosure, if R¹ and R³ are both -CF₃, then R² and R⁴ are not COOR^{5C} and COOR^{5D}, respectively, when R^{5C} and R^{5D} are substituted or unsubstituted alkyl. In some compounds of the disclosure, if R¹ and R³ are both -CF₃, then R² and R⁴ are not COOR^{5C} and COOR^{5D}.

In other compounds of the disclosure, if R¹ and R³ are both -CO₂CH₃, R² and R⁴ are both not -CF₃. In other words, if R¹ and R³ are both -CO₂CH₃, R² and R⁴ are not simultaneously -CF₃. In other compounds of the disclosure, if R¹ and R³ are both-CO₂CH₂CH₂CH₃, R² and R⁴ are both not -CF₃. In other words, if R¹ and R³ are both-CO₂CH₂CH₂CH₃, R² and R⁴ are not simultaneously -CF₃. In some compounds of the disclosure, if R¹ and R³ are both -CO₂CH₃, R² and R⁴ are not C(X²)₃ and C(X⁴)₃, respectively, when X² and X⁴ are both fluorine. In some compounds of the disclosure, if R¹ and R³ are both -CO₂CH₃, R² and R⁴ are not C(X²)₃ and C(X⁴)₃, respectively, when X² and X⁴ are both halogen. In some compounds of the disclosure, if R¹ and R³ are both-CO₂CH₃, R² and R⁴ are not C(X²)₃ and C(X⁴)₃, respectively. In some compounds of the disclosure, if R¹ and R³ are both -CO₂CH₃, R² and R⁴ are both not alkyl. In some compounds of the disclosure, if R¹ and R³ are both -CO₂CH₂CH₂CH₃, R² and R⁴ are not C(X²)₃ and C(X⁴)₃, respectively, when X² and X⁴ are both fluorine. In some compounds of the disclosure, if R¹ and R³ are both -CO₂CH₂CH₂CH₃, R² and R⁴ are not C(X²)₃ and C(X⁴)₃, respectively, when X² and X⁴ are both halogen. In some compounds of the disclosure, if R¹ and R³ are both -CO₂CH₂CH₂CH₃, R² and R⁴ are not C(X²)₃ and C(X⁴)₃, respectively. In some compounds of the disclosure, if R¹ and R³ are both-CO₂CH₂CH₂CH₃, R² and R⁴ are both not alkyl.

In some compounds of the disclosure, if R¹ and R³ are COOR^{5A} and COOR^{5B}, respectively, then R² and R⁴ are not -CF₃, when R^{5A} and R^{5B} are methyl or propyl. In some compounds of the disclosure, if R¹ and R³ are COOR^{5A} and COOR^{5B}, respectively, then R² and R⁴ are not -CF₃, when R^{5A} and R^{5B} are unsubstituted C₁-C₃ alkyl. In some compounds of the disclosure, if R¹ and R³ are COOR^{5A} and COOR^{5B}, respectively, then R² and R⁴ are not -CF₃, when R^{5A} and R^{5B} are unsubstituted C₁-C₄ alkyl. In some compounds of the disclosure, if R¹ and R³ are COOR^{5A} and COOR^{5B}, respectively, then R² and R⁴ are not -CF₃, when R^{5A} and R^{5B} are unsubstituted C₁-C₅ alkyl. In some compounds of the disclosure, if R¹ and R³ are COOR^{5A} and COOR^{5B}, respectively, then R² and R⁴ are not-CF₃, when R^{5A} and R^{5B} are unsubstituted C₁-C₆ alkyl. In some compounds of the disclosure, if R¹ and R³ are COOR^{5A} and COOR^{5B}, respectively, then R² and R⁴ are not-CF₃, when R^{5A} and R^{5B} are unsubstituted C₁-C₇ alkyl. In some compounds of the disclosure, if R¹ and R³ are COOR^{5A} and COOR^{5B}, respectively, then R² and R⁴ are not-CF₃, when R^{5A} and R^{5B} are unsubstituted C₁-C₈ alkyl.

In some compounds of the disclosure, if R¹ and R³ are COOR^{5A} and COOR^{5B}, respectively, then R² and R⁴ are not -CF₃, when R^{5A} and R^{5B} are substituted or unsubstituted C₁-C₃ alkyl. In some compounds of the disclosure, if R¹ and R³ are COOR^{5A} and COOR^{5B}, respectively, then R² and R⁴ are not -CF₃, when R^{5A} and R^{5B} are substituted or unsubstituted C₁-C₄ alkyl. In some compounds of the disclosure, if R¹ and R³ are COOR^{5A} and COOR^{5B}, respectively, then R² and R⁴ are not -CF₃, when R^{5A} and R^{5B} are substituted or unsubstituted C₁-C₅ alkyl. In some compounds of the disclosure, if R¹ and R³ are COOR^{5A} and COOR^{5B}, respectively, then R² and R⁴ are not -CF₃, when R^{5A} and R^{5B} are substituted or unsubstituted C₁-C₆ alkyl. In some compounds of the disclosure, if R¹ and R³ are COOR^{5A} and COOR^{5B}, respectively, then R² and R⁴ are not -CF₃, when R^{5A} and R^{5B} are substituted or unsubstituted C₁-C₇ alkyl. In some compounds of the disclosure, if R¹ and R³ are COOR^{5A} and COOR^{5B}, respectively, then R² and R⁴ are not -CF₃, when R^{5A} and R^{5B} are substituted or unsubstituted C₁-C₈ alkyl.

In some compounds of the disclosure, if R¹ and R³ are COOR^{5A} and COOR^{5B}, respectively, then R² and R⁴ are not -CF₃, when R^{5A} and R^{5B} are unsubstituted alkyl. In some compounds of the disclosure, if R¹ and R³ are COOR^{5A} and COOR^{5B}, respectively, then R² and R⁴ are not -CF₃, when R^{5A} and R^{5B} are substituted or unsubstituted alkyl. In some compounds of the disclosure, if R¹ and R³ are COOR^{5A} and COOR^{5B}, then R² and R⁴ are not -CF₃.

In other compounds of the disclosure, if R¹ and R³ are both -CF₃, R² and R⁴ are both not -CO₂CH₃. In other words, if R¹ and R³ are both -CF₃, R² and R⁴ are not simultaneously -CO₂CH₃. In other compounds of the disclosure, if R¹ and R³ are both-CF₃, R² and R⁴ are both not -CO₂CH₂CH₂CH₃. In other words, if R¹ and R³ are both -CF₃, R² and R⁴ are not simultaneously -CO₂CH₂CH₂CH₃. In some compounds of the disclosure, if R¹ and R³ are C(X¹)₃ and C(X³)₃, respectively, R² and R⁴ are not -CO₂CH₃, when X¹ and X³ are both fluorine. In some compounds of the disclosure, if R¹ and R³ are C(X¹)₃ and C(X³)₃, respectively, R² and R⁴ are not -CO₂CH₃, when X² and X⁴ are both halogen. In some compounds of the disclosure, if R¹ and R³ are C(X¹)₃ and C(X³)₃, respectively, R² and R⁴ are not -CO₂CH₃. In some compounds of the disclosure, if R¹ and R³ are both alkyl, R² and R⁴ are not -CO₂CH₃. In some compounds of the disclosure, if R¹ and R³ are C(X¹)₃ and C(X³)₃, respectively, R² and R⁴ are not -CO₂CH₂CH₂CH₃, when X¹ and X³ are both fluorine. In some compounds of the disclosure, if R¹ and R³ are C(X¹)₃ and C(X³)₃, respectively, R² and R⁴ are not -CO₂CH₂CH₂CH₃, when X¹ and X³ are both halogen. In some compounds of the disclosure, if R¹ and R³ are C(X¹)₃ and C(X³)₃, R² and R⁴ are not -CO₂CH₂CH₂CH₃. In some compounds of the disclosure, if R¹ and R³ are both alkyl, R² and R⁴ are not -CO₂CH₂CH₂CH₃.

A person of ordinary skill will immediately understand that the compounds provided herein may have a net charge (e.g. a net positive charge). In such cases, it is understood that any appropriate counterion may be present (e.g. chlorine). For example, the compounds provided herein may be present as a pharmaceutically acceptable salt. Thus,for example, where the compound overall or R¹, R², R³, and R⁴ contain a positive charge, one of skill will immediately recognize that an anionic compound or molecule will be present where the compound is in solution. Any applicable anionic compound or molecule may be used as a counterion to the positively charged substituents, including for example chloride, fluoride, sulfide, a sulfate, a carbonate, or a phosphate.

Each R^{6A} and R^{8A} may be the same or different and may each independently be hydrogen, halogen, -CN, -CF₃, -OH, -NH₂, -COOH, -CH₂COOH, -COOR^{10A}, -CH₂COOR^{10A}, unsubstituted or substituted alkyl, unsubstituted or substituted heteroalkyl, unsubstituted or substituted cycloalkyl, unsubstituted or substituted heterocycloalkyl, unsubstituted or substituted aryl, or an unsubstituted or substituted heteroaryl.

R^{10A} may be unsubstituted or substituted alkyl, unsubstituted or substituted heteroalkyl, unsubstituted or substituted cycloalkyl, unsubstituted or substituted heterocycloalkyl, unsubstituted or substituted aryl, or unsubstituted or substituted heteroaryl. R^{10A} may also be substituted or unsubstituted C₁-C₁₀ (e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In certain compounds of the disclosure, R^{10A} is R^{11A}-substituted or unsubstituted alkyl, R^{11A}-substituted or unsubstituted heteroalkyl, R^{11A}-substituted or unsubstituted cycloalkyl, R^{11A}-substituted or unsubstituted heterocycloalkyl, R^{11A}-substituted or unsubstituted aryl, or R^{11A}-substituted or unsubstituted heteroaryl. R^{10A} may also be R^{11A}-substituted or unsubstituted alkyl, R^{11A}-substituted or unsubstituted heteroalkyl, R^{11A}-substituted or unsubstituted cycloalkyl, R^{11A}-substituted or unsubstituted heterocycloalkyl, R^{11A}-substituted or unsubstituted aryl, or R^{11A}-substituted or unsubstituted heteroaryl. In some compounds of the disclosure, R^{10A} is an unsubstituted alkyl such as unsubstituted C₁-C₁₀ alkyl (e.g., -CH₃ or unsubstituted C₁-C₅ alkyl).

In some compounds of the disclosure, R^{6A} and R^{8A} are each independently hydrogen, halogen, -CN, -CF₃, -OH, -NH₂, -COOH, -CH₂COOH, -COOR^{10A}, -CH₂COOR^{10A}, substituted or unsubstituted C₁-C₁₀ (e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In some compounds of the disclosure, one or both of R^{6A} and R^{8A} is unsubstituted. In some coumpounds of the disclosure, R^{6A} and R^{8A} are independently hydrogen or substituted or unsubstituted C₁-C₁₀ (e.g., C₁-C₆ or C₁-C₃) alkyl. In some compounds of the disclosure, R^{6A} and R^{8A} are independently hydrogen or unsubstituted C₁-C₁₀ (e.g., unsubstituted C₁-C₆ or unsubstituted C₁-C₃) alkyl.

In some compounds of the disclosure, R^{6A} and R^{8A} are hydrogen, halogen, -CN, -CF₃, -OH, -NH₂, -COOH, -CH₂COOH, -COOR^{10A}, -CH₂COOR^{10A}, R^{11A}-substituted or unsubstituted alkyl, R^{11A}-substituted or unsubstituted heteroalkyl, R^{11A}-substituted or unsubstituted cycloalkyl, R^{11A}-substituted or unsubstituted heterocycloalkyl, R^{11A}-substituted or unsubstituted aryl, or R^{11A}-substituted or unsubstituted heteroaryl.

In some compounds of the disclosure, R^{6A} and R^{8A} are independently hydrogen, halogen, -CN, -CF₃, -OH, -NH₂, -COOH, -COOR^{10A}, -CH₂COOH, -CH₂COOR^{10A}, R^{11A}-substituted or unsubstituted C₁-C₁₀ (e.g., C₁-C₆) alkyl, R^{11A}-substituted or unsubstituted 2 to 10 membered (e.g., 2 to 6 membered) heteroalkyl, R^{11A}-substituted or unsubstituted C₃-C₈ (e.g., C₅-C₇) cycloalkyl, R^{11A}-substituted or unsubstituted 3 to 8 membered (e.g., 3 to 6 membered) heterocycloalkyl, R^{11A}-substituted or unsubstituted C₅-C₈ (e.g., C₅-C₆) aryl, or R^{11A}-substituted or unsubstituted 5 to 8 membered (e.g., 5 to 6 membered) heteroaryl.

R^{11A} is halogen, -CN, -CF₃, -OH, -NH₂, -COOH, -COOR^{15A}, -CH₂COOR^{15A}, -CH₂COOH, R^{12A}-substituted or unsubstituted alkyl, R^{12A}-substituted or unsubstituted heteroalkyl, R^{12A}-substituted or unsubstituted cycloalkyl, R^{12A}-substituted or unsubstituted heterocycloalkyl, R^{12A}-substituted or unsubstituted aryl, or R^{12A}-substituted or unsubstituted heteroaryl. In certain compounds of the disclosure, R^{11A} is halogen, -CN, -CF₃, -OH, -NH₂, -COOH, -COOR^{15A}, -CH₂COOR^{15A}, R^{12A}-substituted or unsubstituted C₁-C₁₀ (e.g., C₁-C₆) alkyl, R^{12A}-substituted or unsubstituted 2 to 10 membered (e.g., 2 to 6 membered) heteroalkyl, R^{12A}-substituted or unsubstituted C₃-C₈ (e.g., C₅-C₇) cycloalkyl, R^{12A}-substituted or unsubstituted 3 to 8 membered (e.g., 3 to 6 membered) heterocycloalkyl, R^{12A}-substituted or unsubstituted C₅-C₈ (e.g., C₅-C₆) aryl, or R^{12A}-substituted or unsubstituted 5 to 8 membered (e.g., 5 to 6 membered) heteroaryl.

R^{15A} may be a unsubstituted or substituted alkyl, unsubstituted or substituted heteroalkyl, unsubstituted or substituted cycloalkyl, unsubstituted or substituted heterocycloalkyl, unsubstituted or substituted aryl, or unsubstituted or substituted heteroaryl. R^{15A} may also be substituted or unsubstituted C₁-C₁₀ (e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In some compounds of the disclosure, R^{15A} is R^{12A}-substituted or unsubstituted alkyl, R^{12A}-substituted or unsubstituted heteroalkyl, R^{12A}-substituted or unsubstituted cycloalkyl, R^{12A}-substituted or unsubstituted heterocycloalkyl, R^{12A}-substituted or unsubstituted aryl, or R^{12A}-substituted or unsubstituted heteroaryl. R^{15A} may also be R^{12A}-substituted or unsubstituted C₁-C₁₀ (e.g., C₁-C₆) alkyl, R^{12A}-substituted or unsubstituted 2 to 10 membered (e.g., 2 to 6 membered) heteroalkyl, R^{12A}-substituted or unsubstituted C₃-C₈ (e.g., C₅-C₇) cycloalkyl, R^{12A}-substituted or unsubstituted 3 to 8 membered (e.g., 3 to 6 membered) heterocycloalkyl, R^{12A}-substituted or unsubstituted C₅-C₈ (e.g., C₅-C₆) aryl, or R^{12A}-substituted or unsubstituted 5 to 8 membered (e.g., 5 to 6 membered) heteroaryl.

R^{12A} is halogen, -CN, -CF₃, -OH, -NH₂, -COOH, -COOR^{16A}, -CH₂COOR^{16A}, -CH₂COOH, R^{13A}-substituted or unsubstituted alkyl, R^{13A}-substituted or unsubstituted heteroalkyl, R^{13A}-substituted or unsubstituted cycloalkyl, R^{13A}-substituted or unsubstituted heterocycloalkyl, R^{13A}-substituted or unsubstituted aryl, or R^{13A}-substituted or unsubstituted heteroaryl. In some compounds of the disclosure, R^{12A} is halogen, -CN, -CF₃, -OH, -NH₂, -COOH, -COOR^{16A}, -CH₂COOR^{16A}, R^{13A}-substituted or unsubstituted C₁-C₁₀ (e.g., C₁-C₆) alkyl, R^{13A}-substituted or unsubstituted 2 to 10 membered (e.g., 2 to 6 membered) heteroalkyl, R^{13A}-substituted or unsubstituted C₃-C₈ (e.g., C₅-C₇) cycloalkyl, R^{13A}-substituted or unsubstituted 3 to 8 membered (e.g., 3 to 6 membered) heterocycloalkyl, R^{13A}-substituted or unsubstituted C₅-C₈ (e.g., C₅-C₆) aryl, or R^{13A}-substituted or unsubstituted 5 to 8 membered (e.g., 5 to 6 membered) heteroaryl.

R^{16A} may be a unsubstituted or substituted alkyl, unsubstituted or substituted heteroalkyl, unsubstituted or substituted cycloalkyl, unsubstituted or substituted heterocycloalkyl, unsubstituted or substituted aryl, or unsubstituted or substituted heteroaryl. R^{16A} may also be substituted or unsubstituted C₁-C₁₀ (e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In some compounds of the disclosure, R^{16A} is R^{13A}-substituted or unsubstituted alkyl, R^{13A}-substituted or unsubstituted heteroalkyl, R^{13A}-substituted or unsubstituted cycloalkyl, R^{13A}-substituted or unsubstituted heterocycloalkyl, R^{13A}-substituted or unsubstituted aryl, or R^{13A}-substituted or unsubstituted heteroaryl. R^{16A} may also be R^{13A}-substituted or unsubstituted C₁-C₁₀ (e.g., C₁-C₆) alkyl, R^{13A}-substituted or unsubstituted 2 to 10 membered (e.g., 2 to 6 membered) heteroalkyl, R^{13A}-substituted or unsubstituted C₃-C₈ (e.g., C₅-C₇) cycloalkyl, R^{13A}-substituted or unsubstituted 3 to 8 membered (e.g., 3 to 6 membered) heterocycloalkyl, R^{13A}-substituted or unsubstituted C₅-C₈ (e.g., C₅-C₆) aryl, or R^{13A}-substituted or unsubstituted 5 to 8 membered (e.g., 5 to 6 membered) heteroaryl.

R^{13A} is halogen, -CN, -CF₃, -OH, -NH₂, -COOH, -COOR^{17A}, -CH₂COOR^{17A}, -CH₂COOH, R^{14A}-substituted or unsubstituted alkyl, R^{14A}-substituted or unsubstituted heteroalkyl, R^{14A}-substituted or unsubstituted cycloalkyl, R^{14A}-substituted or unsubstituted heterocycloalkyl, R^{14A}-substituted or unsubstituted aryl, or R^{14A}-substituted or unsubstituted heteroaryl. In one variant, R^{13A} is halogen, -CN, -CF₃, -OH, -NH₂, -COOH, -COOR^{17A}, -CH₂COOR^{17A}, R^{14A}-substituted or unsubstituted C₁-C₁₀ (e.g., C₁-C₆) alkyl, R^{14A}-substituted or unsubstituted 2 to 10 membered (e.g., 2 to 6 membered) heteroalkyl, R^{14A}-substituted or unsubstituted C₃-C₈ (e.g., C₅-C₇) cycloalkyl, R^{14A}-substituted or unsubstituted 3 to 8 membered (e.g., 3 to 6 membered) heterocycloalkyl, R^{14A}-substituted or unsubstituted C₅-C₈ (e.g., C₅-C₆) aryl, or R^{14A}-substituted or unsubstituted 5 to 8 membered (e.g., 5 to 6 membered) heteroaryl. R^{14A} is halogen, -CN, -CF₃, -OH, -NH₂, -COOH, -COOR^{10A}, -CH₂COOR^{10A}, -CH₂COOH, unsubstituted alkyl, unsubstituted heteroalkyl, unsubstituted cycloalkyl, unsubstituted heterocycloalkyl, unsubstituted aryl, or unsubstituted heteroaryl.

R^{17A} may be a unsubstituted or substituted alkyl, unsubstituted or substituted heteroalkyl, unsubstituted or substituted cycloalkyl, unsubstituted or substituted heterocycloalkyl, unsubstituted or substituted aryl, or unsubstituted or substituted heteroaryl. R^{17A} may also be substituted or unsubstituted C₁-C₁₀ (e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In some compounds of the disclosure, R^{17A} is R^{14A}-substituted or unsubstituted alkyl, R^{14A}-substituted or unsubstituted heteroalkyl, R^{14A}-substituted or unsubstituted cycloalkyl, R^{14A}-substituted or unsubstituted heterocycloalkyl, R^{14A}-substituted or unsubstituted aryl, or R^{14A}-substituted or unsubstituted heteroaryl. R^{17A} may also be R^{14A}-substituted or unsubstituted C₁-C₁₀ (e.g., C₁-C₆) alkyl, R^{14A}-substituted or unsubstituted 2 to 10 membered (e.g., 2 to 6 membered) heteroalkyl, R^{14A}-substituted or unsubstituted C₃-C₈ (e.g., C₅-C₇) cycloalkyl, R^{14A}-substituted or unsubstituted 3 to 8 membered (e.g., 3 to 6 membered) heterocycloalkyl, R^{14A}-substituted or unsubstituted C₅-C₈ (e.g., C₅-C₆) aryl, or R^{14A}-substituted or unsubstituted 5 to 8 membered (e.g., 5 to 6 membered) heteroaryl.

R^{7A} may be hydrogen, halogen, -CN, -CF₃, -OH, -NH₂, -COOH, -CH₂COOH, -COOR^{10B}, -CH₂COOR^{10B}, unsubstituted or substituted alkyl, unsubstituted or substituted heteroalkyl, unsubstituted or substituted cycloalkyl, unsubstituted or substituted heterocycloalkyl, unsubstituted or substituted aryl, or an unsubstituted or substituted heteroaryl.

R^{10B} may be unsubstituted or substituted alkyl, unsubstituted or substituted heteroalkyl, unsubstituted or substituted cycloalkyl, unsubstituted or substituted heterocycloalkyl, unsubstituted or substituted aryl, or unsubstituted or substituted heteroaryl. R^{10B} may also be substituted or unsubstituted C₁-C₁₀ (e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In certain compounds of the disclosure, R^{10B} is R^{11B}-substituted or unsubstituted alkyl, R^{11B}-substituted or unsubstituted heteroalkyl, R^{11B}substituted or unsubstituted cycloalkyl, R^{11B}-substituted or unsubstituted heterocycloalkyl, R^{11B}-substituted or unsubstituted aryl, or R^{11B}-substituted or unsubstituted heteroaryl. R^{10B} may also be R^{11B}-substituted or unsubstituted alkyl, R^{11B}-substituted or unsubstituted heteroalkyl, R^{11B}-substituted or unsubstituted cycloalkyl, R^{11B}-substituted or unsubstituted heterocycloalkyl, R^{11B}-substituted or unsubstituted aryl, or R^{11B}-substituted or unsubstituted heteroaryl. In some compounds of the disclosure, R^{10B} is an unsubstituted alkyl such as C₁-C₁₀ alkyl (e.g., -CH₃ or a C₁-C₅ alkyl).

In some compounds of the disclosure, R^{7A} is hydrogen, halogen, -CN, -CF₃, -OH, -NH₂, -COOH, -CH₂COOH , -COOR^{10B}, -CH₂COOR^{10B}, substituted or unsubstituted C₁₋C₁₀(e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In some compounds of the disclosure, R^{7A} is unsubstituted. In some compounds of the disclosure, R^{7A} is hydrogen or substituted or unsubstituted C₁-C₁₀ (e.g., C₁-C₆ or C₁-C₃) alkyl. In some compounds of the disclosure, R^{7A} is hydrogen or unsubstituted C₁-C₁₀ (e.g., unsubstituted C₁-C₆ or unsubstituted C₁-C₃) alkyl.

In some compounds of the disclosure, R^{7A} is hydrogen, halogen, -CN, -CF₃, -OH, -NH₂, -COOH, -CH₂COOH, -COOR^{10B}, -CH₂COOR^{10B}, R^{11B}-substituted or unsubstituted alkyl, R^{11B}-substituted or unsubstituted heteroalkyl, R^{11B}-substituted or unsubstituted cycloalkyl, R^{11B}-substituted or unsubstituted heterocycloalkyl, R^{11B}-substituted or unsubstituted aryl, or R^{11B}-substituted or unsubstituted heteroaryl.

In some compounds of the disclosure, R^{7A} is hydrogen, halogen, -CN, -CF₃, -OH, -NH₂, -COOH, -COOR^{10B}, -CH₂COOH, -CH₂COOR^{10B}, R^{11B}-substituted or unsubstituted C₁-C₁₀ (e.g., C₁-C₆) alkyl, R^{11B}-substituted or unsubstituted 2 to 10 membered (e.g., 2 to 6 membered) heteroalkyl, R^{11B}-substituted or unsubstituted C₃-C₈ (e.g., C₅-C₇) cycloalkyl, R^{11B}-substituted or unsubstituted 3 to 8 membered (e.g., 3 to 6 membered) heterocycloalkyl, R^{11B}-substituted or unsubstituted C₅-C₈ (e.g., C₅-C₆) aryl, or R^{11B}-substituted or unsubstituted 5 to 8 membered (e.g., 5 to 6 membered) heteroaryl.

R^{11B} is halogen, -CN, -CF₃, -OH, -NH₂, -COOH, -COOR^{15B}, -CH₂COOR^{15B}, -CH₂COOH, R^{12B}-substituted or unsubstituted alkyl, R^{12B}-substituted or unsubstituted heteroalkyl, R^{12B}-substituted or unsubstituted cycloalkyl, R^{12B}-substituted or unsubstituted heterocycloalkyl, R^{12B}-substituted or unsubstituted aryl, or R^{12B}-substituted or unsubstituted heteroaryl. In certain compounds of the disclosure, R^{11B} is halogen, -CN, -CF₃, -OH, -NH₂, -COOH, -COOR^{15B}, -CH₂COOR^{15B}, R^{12B}-substituted or unsubstituted C₁-C₁₀ (e.g., C₁-C₆) alkyl, R^{12B}-substituted or unsubstituted 2 to 10 membered (e.g., 2 to 6 membered) heteroalkyl, R^{12B}-substituted or unsubstituted C₃-C₈ (e.g., C₅-C₇) cycloalkyl, R^{12B}-substituted or unsubstituted 3 to 8 membered (e.g., 3 to 6 membered) heterocycloalkyl, R^{12B}-substituted or unsubstituted C₅-C₈ (e.g., C₅-C₆) aryl, or R^{12B}-substituted or unsubstituted 5 to 8 membered (e.g., 5 to 6 membered) heteroaryl.

R^{15B} may be a unsubstituted or substituted alkyl, unsubstituted or substituted heteroalkyl, unsubstituted or substituted cycloalkyl, unsubstituted or substituted heterocycloalkyl, unsubstituted or substituted aryl, or unsubstituted or substituted heteroaryl. R^{15B} may also be substituted or unsubstituted C₁-C₁₀ (e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In some compounds of the disclosure, R^{15B} is R^{12B}-substituted or unsubstituted alkyl, R^{12B}-substituted or unsubstituted heteroalkyl, R^{12B}-substituted or unsubstituted cycloalkyl, R^{12B}-substituted or unsubstituted heterocycloalkyl, R^{12B}-substituted or unsubstituted aryl, or R^{12B}-substituted or unsubstituted heteroaryl. R^{15B} may also be R^{12B}-substituted or unsubstituted C₁-C₁₀ (e.g., C₁-C₆) alkyl, R^{12B}-substituted or unsubstituted 2 to 10 membered (e.g., 2 to 6 membered) heteroalkyl, R^{12B}-substituted or unsubstituted C₃-C₈ (e.g., C₅-C₇) cycloalkyl, R^{12B}-substituted or unsubstituted 3 to 8 membered (e.g., 3 to 6 membered) heterocycloalkyl, R^{12B}-substituted or unsubstituted C₅-C₈ (e.g., C₅-C₆) aryl, or R^{12B}-substituted or unsubstituted 5 to 8 membered (e.g., 5 to 6 membered) heteroaryl.

R^{12B} is halogen, -CN, -CF₃, -OH, -NH₂, -COOH, -COOR^{16B}, -CH₂COOR^{16B}, -CH₂COOH, R^{13B}-substituted or unsubstituted alkyl, R^{13B}-substituted or unsubstituted heteroalkyl, R^{13B}-substituted or unsubstituted cycloalkyl, R^{13B}-substituted or unsubstituted heterocycloalkyl, R^{13B}-substituted or unsubstituted aryl, or R^{13B}-substituted or unsubstituted heteroaryl. In one variant, R^{12B} is halogen, -CN, -CF₃, -OH, -NH₂, -COOH, -COOR^{16B}, -CH₂COOR^{16B}, R^{13B}-substituted or unsubstituted C₁-C₁₀ (e.g., C₁-C₆) alkyl, R^{13B}-substituted or unsubstituted 2 to 10 membered (e.g., 2 to 6 membered) heteroalkyl, R^{13B}-substituted or unsubstituted C₃-C₈ (e.g., C₅-C₇) cycloalkyl, R^{13B}-substituted or unsubstituted 3 to 8 membered (e.g., 3 to 6 membered) heterocycloalkyl, R^{13B}-substituted or unsubstituted C₅-C₈ (e.g., C₅-C₆) aryl, or R^{13B}-substituted or unsubstituted 5 to 8 membered (e.g., 5 to 6 membered) heteroaryl.

R^{16B} may be a unsubstituted or substituted alkyl, unsubstituted or substituted heteroalkyl, unsubstituted or substituted cycloalkyl, unsubstituted or substituted heterocycloalkyl, unsubstituted or substituted aryl, or unsubstituted or substituted heteroaryl. R^{16B} may also be substituted or unsubstituted C₁-C₁₀ (e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In some compounds of the disclosure, R^{16B} is R^{13B}-substituted or unsubstituted alkyl, R^{13B}-substituted or unsubstituted heteroalkyl, R^{13B}-substituted or unsubstituted cycloalkyl, R^{13B}-substituted or unsubstituted heterocycloalkyl, R^{13B}-substituted or unsubstituted aryl, or R^{13B}-substituted or unsubstituted heteroaryl. R^{16B} may also be R^{13B}-substituted or unsubstituted C₁-C₁₀ (e.g., C₁-C₆) alkyl, R^{13B}-substituted or unsubstituted 2 to 10 membered (e.g., 2 to 6 membered) heteroalkyl, R^{13B}-substituted or unsubstituted C₃-C₈ (e.g., C₅-C₇) cycloalkyl, R^{13B}-substituted or unsubstituted 3 to 8 membered (e.g., 3 to 6 membered) heterocycloalkyl, R^{13B}-substituted or unsubstituted C₅-C₈ (e.g., C₅-C₆) aryl, or R^{13B}-substituted or unsubstituted 5 to 8 membered (e.g., 5 to 6 membered) heteroaryl.

R^{13B} is halogen, -CN, -CF₃, -OH, -NH₂, -COOH, -COOR^{17B}, -CH₂COOR^{17B}, -CH₂COOH, R^{14B}-substituted or unsubstituted alkyl, R^{14B}-substituted or unsubstituted heteroalkyl, R^{14B}-substituted or unsubstituted cycloalkyl, R^{14B}-substituted or unsubstituted heterocycloalkyl, R^{14B}-substituted or unsubstituted aryl, or R^{14B}-substituted or unsubstituted heteroaryl. In one variant, R^{13B} is halogen, -CN, -CF₃, -OH, -NH₂, -COOH, -COOR^{17B}, -CH₂COOR^{17B}, R^{14B}-substituted or unsubstituted C₁-C₁₀ (e.g., C₁-C₆) alkyl, R^{14B}-substituted or unsubstituted 2 to 10 membered (e.g., 2 to 6 membered) heteroalkyl, R^{14B}-substituted or unsubstituted C₃-C₈ (e.g., C₅-C₇) cycloalkyl, R^{14B}-substituted or unsubstituted 3 to 8 membered (e.g., 3 to 6 membered) heterocycloalkyl, R^{14B}-substituted or unsubstituted C₅-C₈ (e.g., C₅-C₆) aryl, or R^{14B}-substituted or unsubstituted 5 to 8 membered (e.g., 5 to 6 membered) heteroaryl. R^{14B} is halogen, -CN, -CF₃, -OH, -NH₂, -COOH, -COOR^{10B}, -CH₂COOR^{10B}, -CH₂COOH, unsubstituted alkyl, unsubstituted heteroalkyl, unsubstituted cycloalkyl, unsubstituted heterocycloalkyl, unsubstituted aryl, or unsubstituted heteroaryl.

R^{17B} may be a unsubstituted or substituted alkyl, unsubstituted or substituted heteroalkyl, unsubstituted or substituted cycloalkyl, unsubstituted or substituted heterocycloalkyl, unsubstituted or substituted aryl, or unsubstituted or substituted heteroaryl. R^{17B} may also be substituted or unsubstituted C₁-C₁₀ (e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In some compounds of the disclosure, R^{17B} is R^{14B}-substituted or unsubstituted alkyl, R^{14B}-substituted or unsubstituted heteroalkyl, R^{14B}-substituted or unsubstituted cycloalkyl, R^{14B}-substituted or unsubstituted heterocycloalkyl, R^{14B}-substituted or unsubstituted aryl, or R^{14B}-substituted or unsubstituted heteroaryl. R^{17B} may also be R^{14B}-substituted or unsubstituted C₁-C₁₀ (e.g., C₁-C₆) alkyl, R^{14B}-substituted or unsubstituted 2 to 10 membered (e.g., 2 to 6 membered) heteroalkyl, R^{14B}-substituted or unsubstituted C₃-C₈ (e.g., C₅-C₇) cycloalkyl, R^{14B}-substituted or unsubstituted 3 to 8 membered (e.g., 3 to 6 membered) heterocycloalkyl, R^{14B}-substituted or unsubstituted C₅-C₈ (e.g., C₅-C₆) aryl, or R^{14B}-substituted or unsubstituted 5 to 8 membered (e.g., 5 to 6 membered) heteroaryl.

Each R^{6B} and R^{8B} may be the same or different and may each independently be hydrogen, halogen, -CN, -CF₃, -OH, -NH₂, -COOH, -CH₂COOH, -COOR^{10C}, -CH₂COOR^{10C}, unsubstituted or substituted alkyl, unsubstituted or substituted heteroalkyl, unsubstituted or substituted cycloalkyl, unsubstituted or substituted heterocycloalkyl, unsubstituted or substituted aryl, or an unsubstituted or substituted heteroaryl.

R^{10C} may be unsubstituted or substituted alkyl, unsubstituted or substituted heteroalkyl, unsubstituted or substituted cycloalkyl, unsubstituted or substituted heterocycloalkyl, unsubstituted or substituted aryl, or unsubstituted or substituted heteroaryl. R^{10C} may also be substituted or unsubstituted C₁-C₁₀ (e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In certain compounds of the disclosure, R^{10C} is R^{11C}-substituted or unsubstituted alkyl, R^{11C}-substituted or unsubstituted heteroalkyl, R^{11C}-substituted or unsubstituted cycloalkyl, R^{11C}-substituted or unsubstituted heterocycloalkyl, R^{11C}-substituted or unsubstituted aryl, or R^{11C}-substituted or unsubstituted heteroaryl. R^{10C} may also be R^{11C}-substituted or unsubstituted alkyl, R^{11C}-substituted or unsubstituted heteroalkyl, R^{11C}-substituted or unsubstituted cycloalkyl, R^{11C}-substituted or unsubstituted heterocycloalkyl, R^{11C}-substituted or unsubstituted aryl, or R^{11C}-substituted or unsubstituted heteroaryl. In some compounds of the disclosure, R^{10C} is an unsubstituted alkyl such as C₁-C₁₀ alkyl (e.g., -CH₃ or a C₁-C₅ alkyl).

In some compounds of the disclosure, R^{6B} and R^{8B} are each independently hydrogen, halogen, -CN, -CF₃, -OH, -NH₂, -COOH, -CH₂COOH , -COOR^{10C}, -CH₂COOR^{10C}, substituted or unsubstituted C₁-C₁₀ (e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In some compounds of the disclosure, one or both of R^{6B} and R^{8B} is unsubstituted. In certain compounds of the disclosure, R^{6B} and R^{8B} are independently hydrogen or substituted or unsubstituted C₁-C₁₀ (e.g., C₁-C₆ or C₁-C₃) alkyl.

In certain compounds of the disclosure, R^{6B} and R^{8B} are hydrogen, halogen, -CN, -CF₃, -OH, -NH₂, -COOH, -CH₂COOH, -COOR^{10C}, -CH₂COOR^{10C}, R^{11C}-substituted or unsubstituted alkyl, R^{11C}-substituted or unsubstituted heteroalkyl, R^{11C}-substituted or unsubstituted cycloalkyl, R^{11C}-substituted or unsubstituted heterocycloalkyl, R^{11C}-substituted or unsubstituted aryl, or R^{11C}-substituted or unsubstituted heteroaryl.

In certain compounds of the disclosure, R^{6B} and R^{8B} are independently hydrogen, halogen, -CN, -CF₃, -OH, -NH₂, -COOH, -COOR^{10C}, -CH₂COOH, -CH₂COOR^{10C}, R^{11C}-substituted or unsubstituted C₁-C₁₀ (e.g., C₁-C₆) alkyl, R^{11C}-substituted or unsubstituted 2 to 10 membered (e.g., 2 to 6 membered) heteroalkyl, R^{11C}-substituted or unsubstituted C₃-C₈ (e.g., C₅-C₇) cycloalkyl, R^{11C}-substituted or unsubstituted 3 to 8 membered (e.g., 3 to 6 membered) heterocycloalkyl, R^{11C}-substituted or unsubstituted C₅-C₈ (e.g., C₅-C₆) aryl, or R^{11C}-substituted or unsubstituted 5 to 8 membered (e.g., 5 to 6 membered) heteroaryl.

R^{11C} is halogen, -CN, -CF₃, -OH, -NH₂, -COOH, -COOR^{15C}, -CH₂COOR^{15C}, -CH₂COOH, R^{12C}-substituted or unsubstituted alkyl, R^{12C}-substituted or unsubstituted heteroalkyl, R^{12C}-substituted or unsubstituted cycloalkyl, R^{12C}-substituted or unsubstituted heterocycloalkyl, R^{12C}-substituted or unsubstituted aryl, or R^{12C}-substituted or unsubstituted heteroaryl. In certain compounds of the disclosure, R^{11C} is halogen, -CN, -CF₃, -OH, -NH₂, -COOH, -COOR^{15C}, -CH₂COOR^{15C}, R^{12C}-substituted or unsubstituted C₁-C₁₀ (e.g., C₁-C₆) alkyl, R^{12C}-substituted or unsubstituted 2 to 10 membered (e.g., 2 to 6 membered) heteroalkyl, R^{12C}-substituted or unsubstituted C₃-C₈ (e.g., C₅-C₇) cycloalkyl, R^{12C}-substituted or unsubstituted 3 to 8 membered (e.g., 3 to 6 membered) heterocycloalkyl, R^{12C}-substituted or unsubstituted C₅-C₈ (e.g., C₅-C₆) aryl, or R^{12C}-substituted or unsubstituted 5 to 8 membered (e.g., 5 to 6 membered) heteroaryl.

R^{15C} may be a unsubstituted or substituted alkyl, unsubstituted or substituted heteroalkyl, unsubstituted or substituted cycloalkyl, unsubstituted or substituted heterocycloalkyl, unsubstituted or substituted aryl, or unsubstituted or substituted heteroaryl. R^{15C} may also be substituted or unsubstituted C₁-C₁₀ (e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In some compounds of the disclosure, R^{15C} is R^{12C}-substituted or unsubstituted alkyl, R^{12C}-substituted or unsubstituted heteroalkyl, R^{12C}-substituted or unsubstituted cycloalkyl, R^{12C}-substituted or unsubstituted heterocycloalkyl, R^{12C}-substituted or unsubstituted aryl, or R^{12C}-substituted or unsubstituted heteroaryl. R^{15C} may also be R^{12C}-substituted or unsubstituted C₁-C₁₀ (e.g., C₁-C₆) alkyl, R^{12C}-substituted or unsubstituted 2 to 10 membered (e.g., 2 to 6 membered) heteroalkyl, R^{12C}-substituted or unsubstituted C₃-C₈ (e.g., C₅-C₇) cycloalkyl, R^{12C}-substituted or unsubstituted 3 to 8 membered (e.g., 3 to 6 membered) heterocycloalkyl, R^{12C}-substituted or unsubstituted C₅-C₈ (e.g., C₅-C₆) aryl, or R^{12C}-substituted or unsubstituted 5 to 8 membered (e.g., 5 to 6 membered) heteroaryl.

R^{12C} is halogen, -CN, -CF₃, -OH, -NH₂, -COOH, -COOR^{16C}, -CH₂COOR^{16C}, -CH₂COOH, R^{13C}-substituted or unsubstituted alkyl, R^{13C}-substituted or unsubstituted heteroalkyl, R^{13C}-substituted or unsubstituted cycloalkyl, R^{13C}-substituted or unsubstituted heterocycloalkyl, R^{13C}-substituted or unsubstituted aryl, or R^{13C}-substituted or unsubstituted heteroaryl. In certain compounds of the disclosure, R^{12C} is halogen, -CN, -CF₃, -OH, -NH₂, -COOH, -COOR^{16C}, -CH₂COOR^{16C}, R^{13C}-substituted or unsubstituted C₁-C₁₀ (e.g., C₁-C₆) alkyl, R^{13C}-substituted or unsubstituted 2 to 10 membered (e.g., 2 to 6 membered) heteroalkyl, R^{13C}-substituted or unsubstituted C₃-C₈ (e.g., C₅-C₇) cycloalkyl, R^{13C}-substituted or unsubstituted 3 to 8 membered (e.g., 3 to 6 membered) heterocycloalkyl, R^{13C}-substituted or unsubstituted C₅-C₈ (e.g., C₅-C₆) aryl, or R^{13C}-substituted or unsubstituted 5 to 8 membered (e.g., 5 to 6 membered) heteroaryl.

R^{16C} may be a unsubstituted or substituted alkyl, unsubstituted or substituted heteroalkyl, unsubstituted or substituted cycloalkyl, unsubstituted or substituted heterocycloalkyl, unsubstituted or substituted aryl, or unsubstituted or substituted heteroaryl. R^{16C} may also be substituted or unsubstituted C₁-C₁₀ (e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In some compounds of the disclosure, R^{16C} is R^{13C}-substituted or unsubstituted alkyl, R^{13C}-substituted or unsubstituted heteroalkyl, R^{13C}-substituted or unsubstituted cycloalkyl, R^{13C}-substituted or unsubstituted heterocycloalkyl, R^{13C}-substituted or unsubstituted aryl, or R^{13C}-substituted or unsubstituted heteroaryl. R^{16C} may also be R^{13C}-substituted or unsubstituted C₁-C₁₀ (e.g., C₁-C₆) alkyl, R^{13C}-substituted or unsubstituted 2 to 10 membered (e.g., 2 to 6 membered) heteroalkyl, R^{13C}-substituted or unsubstituted C₃-C₈ (e.g., C₅-C₇) cycloalkyl, R^{13C}-substituted or unsubstituted 3 to 8 membered (e.g., 3 to 6 membered) heterocycloalkyl, R^{13C}-substituted or unsubstituted C₅-C₈ (e.g., C₅-C₆) aryl, or R^{13C}-substituted or unsubstituted 5 to 8 membered (e.g., 5 to 6 membered) heteroaryl.

R^{13C} is halogen, -CN, -CF₃, -OH, -NH₂, -COOH, -COOR^{17C}, -CH₂COOR^{17C}, -CH₂COOH, R^{14C}-substituted or unsubstituted alkyl, R^{14C}-substituted or unsubstituted heteroalkyl, R^{14C}-substituted or unsubstituted cycloalkyl, R^{14C}-substituted or unsubstituted heterocycloalkyl, R^{14C}-substituted or unsubstituted aryl, or R^{14C}-substituted or unsubstituted heteroaryl. In certain compounds of the disclosure, R^{13C} is halogen, -CN, -CF₃, -OH, -NH₂, -COOH, -COOR^{17C}, -CH₂COOR^{17C}, R^{14C}-substituted or unsubstituted C₁-C₁₀ (e.g., C₁-C₆) alkyl, R^{14C}-substituted or unsubstituted 2 to 10 membered (e.g., 2 to 6 membered) heteroalkyl, R^{14C}-substituted or unsubstituted C₃-C₈ (e.g., C₅-C₇) cycloalkyl, R^{14C}-substituted or unsubstituted 3 to 8 membered (e.g., 3 to 6 membered) heterocycloalkyl, R^{14C}-substituted or unsubstituted C₅-C₈ (e.g., C₅-C₆) aryl, or R^{14C}-substituted or unsubstituted 5 to 8 membered (e.g., 5 to 6 membered) heteroaryl. R^{14C} is halogen, -CN, -CF₃, -OH, -NH₂, -COOH, -COOR^{10C}, -CH₂COOR^{10C}, -CH₂COOH, unsubstituted alkyl, unsubstituted heteroalkyl, unsubstituted cycloalkyl, unsubstituted heterocycloalkyl, unsubstituted aryl, or unsubstituted heteroaryl.

R^{17C} may be a unsubstituted or substituted alkyl, unsubstituted or substituted heteroalkyl, unsubstituted or substituted cycloalkyl, unsubstituted or substituted heterocycloalkyl, unsubstituted or substituted aryl, or unsubstituted or substituted heteroaryl. R^{17C} may also be substituted or unsubstituted C₁-C₁₀ (e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In some compounds of the disclosure, R^{17C} is R^{14C}-substituted or unsubstituted alkyl, R^{14C}-substituted or unsubstituted heteroalkyl, R^{14C}-substituted or unsubstituted cycloalkyl, R^{14C}-substituted or unsubstituted heterocycloalkyl, R^{14C}-substituted or unsubstituted aryl, or R^{14C}-substituted or unsubstituted heteroaryl. R^{17C} may also be R^{14C}-substituted or unsubstituted C₁-C₁₀ (e.g., C₁-C₆) alkyl, R^{14C}-substituted or unsubstituted 2 to 10 membered (e.g., 2 to 6 membered) heteroalkyl, R^{14C}-substituted or unsubstituted C₃-C₈ (e.g., C₅-C₇) cycloalkyl, R^{14C}-substituted or unsubstituted 3 to 8 membered (e.g., 3 to 6 membered) heterocycloalkyl, R^{14C}-substituted or unsubstituted C₅-C₈ (e.g., C₅-C₆) aryl, or R^{14C}-substituted or unsubstituted 5 to 8 membered (e.g., 5 to 6 membered) heteroaryl.

R^{7B} may be hydrogen, halogen, -CN, -CF₃, -OH, -NH₂, -COOH, -CH₂COOH, -COOR^{10D}, -CH₂COOR^{10D}, unsubstituted or substituted alkyl, unsubstituted or substituted heteroalkyl, unsubstituted or substituted cycloalkyl, unsubstituted or substituted heterocycloalkyl, unsubstituted or substituted aryl, or an unsubstituted or substituted heteroaryl.

R^{10D} may be unsubstituted or substituted alkyl, unsubstituted or substituted heteroalkyl, unsubstituted or substituted cycloalkyl, unsubstituted or substituted heterocycloalkyl, unsubstituted or substituted aryl, or unsubstituted or substituted heteroaryl. R^{10D} may also be substituted or unsubstituted C₁-C₁₀ (e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In certain compounds of the disclosure, R^{10D} is R^{11D}-substituted or unsubstituted alkyl, R^{11D}-substituted or unsubstituted heteroalkyl, R^{11D}-substituted or unsubstituted cycloalkyl, R^{11D}-substituted or unsubstituted heterocycloalkyl, R^{11D}-substituted or unsubstituted aryl, or R^{11D}-substituted or unsubstituted heteroaryl. R^{10D} may also be R^{11D}-substituted or unsubstituted alkyl, R^{11D}-substituted or unsubstituted heteroalkyl, R^{11D}-substituted or unsubstituted cycloalkyl, R^{11D}-substituted or unsubstituted heterocycloalkyl, R^{11D}-substituted or unsubstituted aryl, or R^{11D}-substituted or unsubstituted heteroaryl. In some compounds of the disclosure, R^{10D} is an unsubstituted alkyl such as C₁-C₁₀ alkyl (e.g., -CH₃ or a C₁-C₅ alkyl).

In some compounds of the disclosure, R^{7B} is hydrogen, halogen, -CN, -CF₃, -OH, -NH₂, -COOH, -CH₂COOH , -COOR^{10D}, -CH₂COOR^{10D}, substituted or unsubstituted C₁-C₁₀ (e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In some compounds of the disclosure, R^{7B} is unsubstituted. In certain compounds of the disclosure, R^{7B} is hydrogen or substituted or unsubstituted C₁-C₁₀ (e.g., C₁-C₆ or C₁-C₃) alkyl.

In certain compounds of the disclosure, R^{7B} is hydrogen, halogen, -CN, -CF₃, -OH, -NH₂, -COOH, -CH₂COOH, -COOR^{10D}, -CH₂COOR^{10D}, R^{11D}-substituted or unsubstituted alkyl, R^{11D}-substituted or unsubstituted heteroalkyl, R^{11D}-substituted or unsubstituted cycloalkyl, R^{11D}-substituted or unsubstituted heterocycloalkyl, R^{11D}-substituted or unsubstituted aryl, or R^{11D}-substituted or unsubstituted heteroaryl.

In certain compounds of the disclosure, R^{7B} is hydrogen, halogen, -CN, -CF₃, -OH, -NH₂, -COOH, -COOR^{10D}, -CH₂COOH, -CH₂COOR^{10D}, R^{11D}-substituted or unsubstituted C₁-C₁₀ (e.g., C₁-C₆) alkyl, R^{11D}-substituted or unsubstituted 2 to 10 membered (e.g., 2 to 6 membered) heteroalkyl, R^{11D}-substituted or unsubstituted C₃-C₈ (e.g., C₅-C₇) cycloalkyl, R^{11D}-substituted or unsubstituted 3 to 8 membered (e.g., 3 to 6 membered) heterocycloalkyl, R^{11D}-substituted or unsubstituted C₅-C₈ (e.g., C₅-C₆) aryl, or R^{11D}-substituted or unsubstituted 5 to 8 membered (e.g., 5 to 6 membered) heteroaryl.

R^{11D} is halogen, -CN, -CF₃, -OH, -NH₂, -COOH, -COOR^{15D}, -CH₂COOR^{15D}, -CH₂COOH, R^{12D}-substituted or unsubstituted alkyl, R^{12D}-substituted or unsubstituted heteroalkyl, R^{12D}-substituted or unsubstituted cycloalkyl, R^{12D}-substituted or unsubstituted heterocycloalkyl, R^{12D}-substituted or unsubstituted aryl, or R^{12D}-substituted or unsubstituted heteroaryl. In certain compounds of the disclosure, R^{11D} is halogen, -CN, -CF₃, -OH, -NH₂, -COOH, -COOR^{15D}, -CH₂COOR^{15D}, R^{12D}-substituted or unsubstituted C₁-C₁₀ (e.g., C₁-C₆) alkyl, R^{12D}-substituted or unsubstituted 2 to 10 membered (e.g., 2 to 6 membered) heteroalkyl, R^{12D}-substituted or unsubstituted C₃-C₈ (e.g., C₅-C₇) cycloalkyl, R^{12D}-substituted or unsubstituted 3 to 8 membered (e.g., 3 to 6 membered) heterocycloalkyl, R^{12D}-substituted or unsubstituted C₅-C₈ (e.g., C₅-C₆) aryl, or R^{12D}-substituted or unsubstituted 5 to 8 membered (e.g., 5 to 6 membered) heteroaryl.

R^{15D} may be a unsubstituted or substituted alkyl, unsubstituted or substituted heteroalkyl, unsubstituted or substituted cycloalkyl, unsubstituted or substituted heterocycloalkyl, unsubstituted or substituted aryl, or unsubstituted or substituted heteroaryl. R^{15D} may also be substituted or unsubstituted C₁-C₁₀ (e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In some compounds of the disclosure, R^{15D} is R^{12D}-substituted or unsubstituted alkyl, R^{12D}-substituted or unsubstituted heteroalkyl, R^{12D}-substituted or unsubstituted cycloalkyl, R^{12D}-substituted or unsubstituted heterocycloalkyl, R^{12D}-substituted or unsubstituted aryl, or R^{12D}-substituted or unsubstituted heteroaryl. R^{15D} may also be R^{12D}-substituted or unsubstituted C₁-C₁₀ (e.g., C₁-C₆) alkyl, R^{12D}-substituted or unsubstituted 2 to 10 membered (e.g., 2 to 6 membered) heteroalkyl, R^{12D}-substituted or unsubstituted C₃-C₈ (e.g., C₅-C₇) cycloalkyl, R^{12D}-substituted or unsubstituted 3 to 8 membered (e.g., 3 to 6 membered) heterocycloalkyl, R^{12D}-substituted or unsubstituted C₅-C₈ (e.g., C₅-C₆) aryl, or R^{12D}-substituted or unsubstituted 5 to 8 membered (e.g., 5 to 6 membered) heteroaryl.

R^{12D} is halogen, -CN, -CF₃, -OH, -NH₂, -COOH, -COOR^{16D}, -CH₂COOR^{16D}, -CH₂COOH, R^{13D}-substituted or unsubstituted alkyl, R^{13D}-substituted or unsubstituted heteroalkyl, R^{13D}-substituted or unsubstituted cycloalkyl, R^{13D}-substituted or unsubstituted heterocycloalkyl, R^{13D}-substituted or unsubstituted aryl, or R^{13D}-substituted or unsubstituted heteroaryl. In certain compounds of the disclosure t, R^{12D} is halogen, -CN, -CF₃, -OH, -NH₂, -COOH, -COOR^{16D}, -CH₂COOR^{16D}, R^{13D}-substituted or unsubstituted C₁-C₁₀ (e.g., C₁-C₆) alkyl, R^{13D}-substituted or unsubstituted 2 to 10 membered (e.g., 2 to 6 membered) heteroalkyl, R^{13D}-substituted or unsubstituted C₃-C₈ (e.g., C₅-C₇) cycloalkyl, R^{13D}-substituted or unsubstituted 3 to 8 membered (e.g., 3 to 6 membered) heterocycloalkyl, R^{13D}-substituted or unsubstituted C₅-C₈ (e.g., C₅-C₆) aryl, or R^{13D}-substituted or unsubstituted 5 to 8 membered (e.g., 5 to 6 membered) heteroaryl.

R^{16D} may be a unsubstituted or substituted alkyl, unsubstituted or substituted heteroalkyl, unsubstituted or substituted cycloalkyl, unsubstituted or substituted heterocycloalkyl, unsubstituted or substituted aryl, or unsubstituted or substituted heteroaryl. R^{16D} may also be substituted or unsubstituted C₁-C₁₀ (e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In some compounds of the disclosure, R^{16D} is R^{13D}-substituted or unsubstituted alkyl, R^{13D}-substituted or unsubstituted heteroalkyl, R^{13D}-substituted or unsubstituted cycloalkyl, R^{13D}-substituted or unsubstituted heterocycloalkyl, R^{13D}-substituted or unsubstituted aryl, or R^{13D}-substituted or unsubstituted heteroaryl. R^{16D} may also be R^{13D}-substituted or unsubstituted C₁-C₁₀ (e.g., C₁-C₆) alkyl, R^{13D}-substituted or unsubstituted 2 to 10 membered (e.g., 2 to 6 membered) heteroalkyl, R^{13D}-substituted or unsubstituted C₃-C₈ (e.g., C₅-C₇) cycloalkyl, R^{13D}-substituted or unsubstituted 3 to 8 membered (e.g., 3 to 6 membered) heterocycloalkyl, R^{13D}-substituted or unsubstituted C₅-C₈ (e.g., C₅-C₆) aryl, or R^{13D}-substituted or unsubstituted 5 to 8 membered (e.g., 5 to 6 membered) heteroaryl.

R^{13D} is halogen, -CN, -CF₃, -OH, -NH₂, -COOH, -COOR^{17D}, -CH₂COOR^{17D}, -CH₂COOH, R^{14D}-substituted or unsubstituted alkyl, R^{14D}-substituted or unsubstituted heteroalkyl, R^{14D}-substituted or unsubstituted cycloalkyl, R^{14D}-substituted or unsubstituted heterocycloalkyl, R^{14D}-substituted or unsubstituted aryl, or R^{14D}-substituted or unsubstituted heteroaryl. In certain compounds of the disclosure, R^{13D} is halogen, -CN, -CF₃, -OH, -NH₂, -COOH, -COOR^{17D}, -CH₂COOR^{17D}, R^{14D}-substituted or unsubstituted C₁-C₁₀ (e.g., C₁-C₆) alkyl, R^{14D}-substituted or unsubstituted 2 to 10 membered (e.g., 2 to 6 membered) heteroalkyl, R^{14D}-substituted or unsubstituted C₃-C₈ (e.g., C₅-C₇) cycloalkyl, R^{14D}-substituted or unsubstituted 3 to 8 membered (e.g., 3 to 6 membered) heterocycloalkyl, R^{14D}-substituted or unsubstituted C₅-C₈ (e.g., C₅-C₆) aryl, or R^{14D}-substituted or unsubstituted 5 to 8 membered (e.g., 5 to 6 membered) heteroaryl. R^{14D} is halogen, -CN, -CF₃, -OH, -NH₂, -COOH, -COOR^{10D}, -CH₂COOR^{10D}, -CH₂COOH, unsubstituted alkyl, unsubstituted heteroalkyl, unsubstituted cycloalkyl, unsubstituted heterocycloalkyl, unsubstituted aryl, or unsubstituted heteroaryl.

R^{17D} may be an unsubstituted or substituted alkyl, unsubstituted or substituted heteroalkyl, unsubstituted or substituted cycloalkyl, unsubstituted or substituted heterocycloalkyl, unsubstituted or substituted aryl, or unsubstituted or substituted heteroaryl. R^{17D} may also be substituted or unsubstituted C₁-C₁₀ (e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In some compounds of the disclosure, R^{17D} is R^{14D}-substituted or unsubstituted alkyl, R^{14D}-substituted or unsubstituted heteroalkyl, R^{14D}-substituted or unsubstituted cycloalkyl, R^{14D}-substituted or unsubstituted heterocycloalkyl, R^{14D}-substituted or unsubstituted aryl, or R^{14D}-substituted or unsubstituted heteroaryl. R^{17D} may also be R^{14D}-substituted or unsubstituted C₁-C₁₀ (e.g., C₁-C₆) alkyl, R^{14D}-substituted or unsubstituted 2 to 10 membered (e.g., 2 to 6 membered) heteroalkyl, R^{14D}-substituted or unsubstituted C₃-C₈ (e.g., C₅-C₇) cycloalkyl, R^{14D}-substituted or unsubstituted 3 to 8 membered (e.g., 3 to 6 membered) heterocycloalkyl, R^{14D}-substituted or unsubstituted C₅-C₈ (e.g., C₅-C₆) aryl, or R^{14D}-substituted or unsubstituted 5 to 8 membered (e.g., 5 to 6 membered) heteroaryl.

R^{5A} may be hydrogen, unsubstituted or substituted alkyl, unsubstituted or substituted heteroalkyl, unsubstituted or substituted cycloalkyl, unsubstituted or substituted heterocycloalkyl, unsubstituted or substituted aryl, or an unsubstituted or substituted heteroaryl. In some compounds of the disclosure, R^{5A} is hydrogen, unsubstituted or substituted alkyl, unsubstituted or substituted heteroalkyl, unsubstituted or substituted cycloalkyl, unsubstituted or substituted heterocycloalkyl, unsubstituted or substituted aryl, or an unsubstituted or substituted heteroaryl. R^{5A} may also be substituted or unsubstituted C₁-C₁₀ (e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In some compounds of the disclosure, R^{5A} is R¹⁸-substituted or unsubstituted alkyl, R¹⁸-substituted or unsubstituted heteroalkyl, R¹⁸-substituted or unsubstituted cycloalkyl, R¹⁸-substituted or unsubstituted heterocycloalkyl, R¹⁸-substituted or unsubstituted aryl, or R¹⁸-substituted or unsubstituted heteroaryl. R^{5A} may also be R¹⁸-substituted or unsubstituted C₁-C₁₀ (e.g., C₁-C₆) alkyl, R¹⁸-substituted or unsubstituted 2 to 10 membered (e.g., 2 to 6 membered) heteroalkyl, R¹⁸-substituted or unsubstituted C₃-C₈ (e.g., C₅-C₇) cycloalkyl, R¹⁸-substituted or unsubstituted 3 to 8 membered (e.g., 3 to 6 membered) heterocycloalkyl, R¹⁸-substituted or unsubstituted C₅-C₈ (e.g., C₅-C₆) aryl, or R¹⁸-substituted or unsubstituted 5 to 8 membered (e.g., 5 to 6 membered) heteroaryl.

R¹⁸ is hydrogen, halogen, -CN, -CF₃, -OH, -NH₂, -COOH, unsubstituted or substituted alkyl, unsubstituted or substituted heteroalkyl, unsubstituted or substituted cycloalkyl, unsubstituted or substituted heterocycloalkyl, unsubstituted or substituted aryl, or an unsubstituted or substituted heteroaryl. R¹⁸ may also be hydrogen, halogen, -CN, -CF₃, -OH, -NH₂, -COOH, substituted or unsubstituted C₁-C₁₀ (e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In some compounds of the disclosure, R¹⁸ is halogen, -CN, -CF₃, -OH, -NH₂, -COOH, R¹⁹-substituted or unsubstituted alkyl, R¹⁹-substituted or unsubstituted heteroalkyl, R¹⁹-substituted or unsubstituted cycloalkyl, R¹⁹-substituted or unsubstituted heterocycloalkyl, R¹⁹-substituted or unsubstituted aryl, or R¹⁹-substituted or unsubstituted heteroaryl. R¹⁸ may also be halogen, -CN, -CF₃, -OH, -NH₂, -COOH, R¹⁹-substituted or unsubstituted C₁-C₁₀ (e.g., C₁-C₆) alkyl, R¹⁹-substituted or unsubstituted 2 to 10 membered (e.g., 2 to 6 membered) heteroalkyl, R¹⁹-substituted or unsubstituted C₃-C₈ (e.g., C₅-C₇) cycloalkyl, R¹⁹-substituted or unsubstituted 3 to 8 membered (e.g., 3 to 6 membered) heterocycloalkyl, R¹⁹-substituted or unsubstituted C₅-C₈ (e.g., C₅-C₆) aryl, or R¹⁹-substituted or unsubstituted 5 to 8 membered (e.g., 5 to 6 membered) heteroaryl.

R¹⁹ is halogen, -CN, -CF₃, -OH, -NH₂, -COOH, unsubstituted or substituted alkyl, unsubstituted or substituted heteroalkyl, unsubstituted or substituted cycloalkyl, unsubstituted or substituted heterocycloalkyl, unsubstituted or substituted aryl, or an unsubstituted or substituted heteroaryl. R¹⁹ may also be halogen, -CN, -CF₃, -OH, -NH₂, -COOH, substituted or unsubstituted C₁-C₁₀ (e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In some compounds of the disclosure, R¹⁹ is halogen, -CN, -CF₃, -OH, -NH₂, -COOH, R²⁰-substituted or unsubstituted alkyl, R²⁰-substituted or unsubstituted heteroalkyl, R²⁰-substituted or unsubstituted cycloalkyl, R²⁰-substituted or unsubstituted heterocycloalkyl, R²⁰-substituted or unsubstituted aryl, or R²⁰-substituted or unsubstituted heteroaryl. R¹⁹ may also be halogen, -CN, -CF₃, -OH, -NH₂, -COOH, R²⁰-substituted or unsubstituted C₁-C₁₀ (e.g., C₁-C₆) alkyl, R²⁰-substituted or unsubstituted 2 to 10 membered (e.g., 2 to 6 membered) heteroalkyl, R²⁰-substituted or unsubstituted C₃-C₈ (e.g., C₅-C₇) cycloalkyl, R²⁰-substituted or unsubstituted 3 to 8 membered (e.g., 3 to 6 membered) heterocycloalkyl, R²⁰-substituted or unsubstituted C₅-C₈ (e.g., C₅-C₆) aryl, or R²⁰-substituted or unsubstituted 5 to 8 membered (e.g., 5 to 6 membered) heteroaryl.

R²⁰ is halogen, -CN, -CF₃, -OH, -NH₂, -COOH, unsubstituted or substituted alkyl, unsubstituted or substituted heteroalkyl, unsubstituted or substituted cycloalkyl, unsubstituted or substituted heterocycloalkyl, unsubstituted or substituted aryl, or an unsubstituted or substituted heteroaryl. R²⁰ may also be halogen, -CN, -CF₃, -OH, -NH₂, -COOH, substituted or unsubstituted C₁-C₁₀ (e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In some compounds of the disclosure, R²⁰ is halogen, -CN, -CF₃, -OH, -NH₂, -COOH, R²¹-substituted or unsubstituted alkyl, R²¹-substituted or unsubstituted heteroalkyl, R²¹-substituted or unsubstituted cycloalkyl, R²¹-substituted or unsubstituted heterocycloalkyl, R²¹-substituted or unsubstituted aryl, or R²¹-substituted or unsubstituted heteroaryl. R²⁰ may also be halogen, -CN, -CF₃, -OH, -NH₂, -COOH, R²¹-substituted or unsubstituted C₁-C₁₀ (e.g., C₁-C₆) alkyl, R²¹-substituted or unsubstituted 2 to 10 membered (e.g., 2 to 6 membered) heteroalkyl, R²¹-substituted or unsubstituted C₃-C₈ (e.g., C₅-C₇) cycloalkyl, R²¹-substituted or unsubstituted 3 to 8 membered (e.g., 3 to 6 membered) heterocycloalkyl, R²¹-substituted or unsubstituted C₅-C₈ (e.g., C₅-C₆) aryl, or R²¹-substituted or unsubstituted 5 to 8 membered (e.g., 5 to 6 membered) heteroaryl

R²¹ is halogen, -CN, -CF₃, -OH, -NH₂, -COOH, unsubstituted alkyl, unsubstituted heteroalkyl, unsubstituted cycloalkyl, unsubstituted heterocycloalkyl, unsubstituted aryl, or an unsubstituted heteroaryl.

R^{5B} may be hydrogen, unsubstituted or substituted alkyl, unsubstituted or substituted heteroalkyl, unsubstituted or substituted cycloalkyl, unsubstituted or substituted heterocycloalkyl, unsubstituted or substituted aryl, or an unsubstituted or substituted heteroaryl. In some compounds of the disclosure, R^{5B} is hydrogen, unsubstituted or substituted alkyl, unsubstituted or substituted heteroalkyl, unsubstituted or substituted cycloalkyl, unsubstituted or substituted heterocycloalkyl, unsubstituted or substituted aryl, or an unsubstituted or substituted heteroaryl. R^{5B} may also be substituted or unsubstituted C₁-C₁₀ (e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In some compounds of the disclosure, R^{5B} is R^{18A}-substituted or unsubstituted alkyl, R^{18A}-substituted or unsubstituted heteroalkyl, R^{18A}-substituted or unsubstituted cycloalkyl, R^{18A}-substituted or unsubstituted heterocycloalkyl, R^{18A}-substituted or unsubstituted aryl, or R^{18A}-substituted or unsubstituted heteroaryl. R^{5B} may also be R^{18A}-substituted or unsubstituted C₁-C₁₀ (e.g., C₁-C₆) alkyl, R^{18A}-substituted or unsubstituted 2 to 10 membered (e.g., 2 to 6 membered) heteroalkyl, R^{18A}-substituted or unsubstituted C₃-C₈ (e.g., C₅-C₇) cycloalkyl, R¹⁸-substituted or unsubstituted 3 to 8 membered (e.g., 3 to 6 membered) heterocycloalkyl, R^{18A}-substituted or unsubstituted C₅-C₈ (e.g., C₅-C₆) aryl, or R^{18A}-substituted or unsubstituted 5 to 8 membered (e.g., 5 to 6 membered) heteroaryl.

R^{18A} is hydrogen, halogen, -CN, -CF₃, -OH, -NH₂, -COOH, unsubstituted or substituted alkyl, unsubstituted or substituted heteroalkyl, unsubstituted or substituted cycloalkyl, unsubstituted or substituted heterocycloalkyl, unsubstituted or substituted aryl, or an unsubstituted or substituted heteroaryl. In some compounds of the disclosure, R^{18A} is unsubstituted or substituted alkyl, unsubstituted or substituted heteroalkyl, unsubstituted or substituted cycloalkyl, unsubstituted or substituted heterocycloalkyl, unsubstituted or substituted aryl, or an unsubstituted or substituted heteroaryl. R^{18A} may also be halogen, -CN, -CF₃, -OH, -NH₂, -COOH, substituted or unsubstituted C₁-C₁₀ (e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In some compounds of the disclosure, R^{18A} is halogen, -CN, -CF₃, -OH, -NH₂, -COOH,, R^{19A}-substituted or unsubstituted alkyl, R^{19A}-substituted or unsubstituted heteroalkyl, R^{19A}-substituted or unsubstituted cycloalkyl, R^{19A}-substituted or unsubstituted heterocycloalkyl, R^{19A}-substituted or unsubstituted aryl, or R^{19A}-substituted or unsubstituted heteroaryl. R^{18A} may also be halogen, -CN, -CF₃, -OH, -NH₂, -COOH, R^{19A}-substituted or unsubstituted C₁-C₁₀ (e.g., C₁-C₆) alkyl, R^{19A}-substituted or unsubstituted 2 to 10 membered (e.g., 2 to 6 membered) heteroalkyl, R^{19A}-substituted or unsubstituted C₃-C₈ (e.g., C₅-C₇) cycloalkyl, R^{19A}-substituted or unsubstituted 3 to 8 membered (e.g., 3 to 6 membered) heterocycloalkyl, R^{19A}-substituted or unsubstituted C₅-C₈ (e.g., C₅-C₆) aryl, or R^{19A}-substituted or unsubstituted 5 to 8 membered (e.g., 5 to 6 membered) heteroaryl.

R^{19A} is halogen, -CN, -CF₃, -OH, -NH₂, -COOH, unsubstituted or substituted alkyl, unsubstituted or substituted heteroalkyl, unsubstituted or substituted cycloalkyl, unsubstituted or substituted heterocycloalkyl, unsubstituted or substituted aryl, or an unsubstituted or substituted heteroaryl. In some compounds of the disclosure, R^{19A} is unsubstituted or substituted alkyl, unsubstituted or substituted heteroalkyl, unsubstituted or substituted cycloalkyl, unsubstituted or substituted heterocycloalkyl, unsubstituted or substituted aryl, or an unsubstituted or substituted heteroaryl. R^{19A} may also be halogen, -CN, -CF₃, -OH, -NH₂, -COOH, substituted or unsubstituted C₁-C₁₀ (e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In some compounds of the disclosure, R^{19A} is halogen, -CN, -CF₃, -OH, -NH₂, -COOH, R^{20A}-substituted or unsubstituted alkyl, R^{20A}-substituted or unsubstituted heteroalkyl, R^{20A}-substituted or unsubstituted cycloalkyl, R^{20A}-substituted or unsubstituted heterocycloalkyl, R^{20A}-substituted or unsubstituted aryl, or R^{20A}-substituted or unsubstituted heteroaryl. R^{19A} may also be halogen, -CN, -CF₃, -OH, -NH₂, -COOH, R^{20A}-substituted or unsubstituted C₁-C₁₀ (e.g., C₁-C₆) alkyl, R^{20A}-substituted or unsubstituted 2 to 10 membered (e.g., 2 to 6 membered) heteroalkyl, R^{20A}-substituted or unsubstituted C₃-C₈ (e.g., C₅-C₇) cycloalkyl, R^{20A}-substituted or unsubstituted 3 to 8 membered (e.g., 3 to 6 membered) heterocycloalkyl, R^{20A}-substituted or unsubstituted C₅-C₈ (e.g., C₅-C₆) aryl, or R^{20A}-substituted or unsubstituted 5 to 8 membered (e.g., 5 to 6 membered) heteroaryl.

R^{20A} is halogen, -CN, -CF₃, -OH, -NH₂, -COOH, unsubstituted or substituted alkyl, unsubstituted or substituted heteroalkyl, unsubstituted or substituted cycloalkyl, unsubstituted or substituted heterocycloalkyl, unsubstituted or substituted aryl, or an unsubstituted or substituted heteroaryl. In some compounds of the disclosure, R^{20A} is unsubstituted or substituted alkyl, unsubstituted or substituted heteroalkyl, unsubstituted or substituted cycloalkyl, unsubstituted or substituted heterocycloalkyl, unsubstituted or substituted aryl, or an unsubstituted or substituted heteroaryl. R^{20A} may also be halogen, -CN, -CF₃, -OH, -NH₂, -COOH, substituted or unsubstituted C₁-C₁₀ (e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In some compounds of the disclosure, R^{20A} is halogen, -CN, -CF₃, -OH, -NH₂, -COOH, R^{21A}-substituted or unsubstituted alkyl, R^{21A}-substituted or unsubstituted heteroalkyl, R^{21A}-substituted or unsubstituted cycloalkyl, R^{21A}-substituted or unsubstituted heterocycloalkyl, R^{21A}-substituted or unsubstituted aryl, or R^{21A}-substituted or unsubstituted heteroaryl. R^{20A} may also be halogen, -CN, -CF₃, -OH, -NH₂, -COOH, R^{21A}-substituted or unsubstituted C₁-C₁₀ (e.g., C₁-C₆) alkyl, R^{21A}-substituted or unsubstituted 2 to 10 membered (e.g., 2 to 6 membered) heteroalkyl, R^{21A}-substituted or unsubstituted C₃-C₈ (e.g., C₅-C₇) cycloalkyl, R^{21A}-substituted or unsubstituted 3 to 8 membered (e.g., 3 to 6 membered) heterocycloalkyl, R^{21A}-substituted or unsubstituted C₅-C₈ (e.g., C₅-C₆) aryl, or R^{21A}-substituted or unsubstituted 5 to 8 membered (e.g., 5 to 6 membered) heteroaryl

R^{21A} is halogen, -CN, -CF₃, -OH, -NH₂, -COOH, unsubstituted alkyl, unsubstituted heteroalkyl, unsubstituted cycloalkyl, unsubstituted heterocycloalkyl, unsubstituted aryl, or an unsubstituted heteroaryl.

Each R^{5C} and R^{9A} may be the same or different and may each independently be hydrogen, unsubstituted or substituted alkyl, unsubstituted or substituted heteroalkyl, unsubstituted or substituted cycloalkyl, unsubstituted or substituted heterocycloalkyl, unsubstituted or substituted aryl, or an unsubstituted or substituted heteroaryl. In some compounds of the disclosure, R^{5C} and R^{9A} are independently hydrogen, unsubstituted or substituted alkyl, unsubstituted or substituted heteroalkyl, unsubstituted or substituted cycloalkyl, unsubstituted or substituted heterocycloalkyl, unsubstituted or substituted aryl, or an unsubstituted or substituted heteroaryl. R^{5C} and R^{9A} may also be independently substituted or unsubstituted C₁-C₁₀ (e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In some compounds of the disclosure, R^{5C} and R^{9A} are R^{18B}-substituted or unsubstituted alkyl, R^{18B}-substituted or unsubstituted heteroalkyl, R^{18B}-substituted or unsubstituted cycloalkyl, R^{18B}-substituted or unsubstituted heterocycloalkyl, R^{18B}-substituted or unsubstituted aryl, or R^{18B}-substituted or unsubstituted heteroaryl. R^{5C} and R^{9A} may also be R^{18B}-substituted or unsubstituted C₁-C₁₀ (e.g., C₁-C₆) alkyl, R^{18B}-substituted or unsubstituted 2 to 10 membered (e.g., 2 to 6 membered) heteroalkyl, R^{18B}-substituted or unsubstituted C₃-C₈ (e.g., C₅-C₇) cycloalkyl, R^{18B}-substituted or unsubstituted 3 to 8 membered (e.g., 3 to 6 membered) heterocycloalkyl, R^{18B}-substituted or unsubstituted C₅-C₈ (e.g., C₅-C₆) aryl, or R^{18B}-substituted or unsubstituted 5 to 8 membered (e.g., 5 to 6 membered) heteroaryl.

R^{18B} is halogen, -CN, -CF₃, -OH, -NH₂, -COOH, unsubstituted or substituted alkyl, unsubstituted or substituted heteroalkyl, unsubstituted or substituted cycloalkyl, unsubstituted or substituted heterocycloalkyl, unsubstituted or substituted aryl, or an unsubstituted or substituted heteroaryl. In some compounds of the disclosure, R^{18B} is unsubstituted or substituted alkyl, unsubstituted or substituted heteroalkyl, unsubstituted or substituted cycloalkyl, unsubstituted or substituted heterocycloalkyl, unsubstituted or substituted aryl, or an unsubstituted or substituted heteroaryl. R^{18B} may also be halogen, -CN, -CF₃, -OH, -NH₂, -COOH, substituted or unsubstituted C₁-C₁₀ (e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In some compounds of the disclosure, R^{18B} is halogen, -CN, -CF₃, -OH, -NH₂, -COOH, R^{19B}-substituted or unsubstituted alkyl, R^{19B}-substituted or unsubstituted heteroalkyl, R^{19B}-substituted or unsubstituted cycloalkyl, R^{19B}-substituted or unsubstituted heterocycloalkyl, R^{19B}-substituted or unsubstituted aryl, or R^{19B}-substituted or unsubstituted heteroaryl. R^{18B} may also be halogen, -CN, -CF₃, -OH, -NH₂, -COOH, R^{19B}-substituted or unsubstituted C₁-C₁₀ (e.g., C₁-C₆) alkyl, R^{19B}-substituted or unsubstituted 2 to 10 membered (e.g., 2 to 6 membered) heteroalkyl, R^{19B}-substituted or unsubstituted C₃-C₈ (e.g., C₅-C₇) cycloalkyl, R^{19B}-substituted or unsubstituted 3 to 8 membered (e.g., 3 to 6 membered) heterocycloalkyl, R^{19B}-substituted or unsubstituted C₅-C₈ (e.g., C₅-C₆) aryl, or R^{19B}-substituted or unsubstituted 5 to 8 membered (e.g., 5 to 6 membered) heteroaryl.

R^{19B} is halogen, -CN, -CF₃, -OH, -NH₂, -COOH, unsubstituted or substituted alkyl, unsubstituted or substituted heteroalkyl, unsubstituted or substituted cycloalkyl, unsubstituted or substituted heterocycloalkyl, unsubstituted or substituted aryl, or an unsubstituted or substituted heteroaryl. In some compounds of the disclosure, R^{19B} is unsubstituted or substituted alkyl, unsubstituted or substituted heteroalkyl, unsubstituted or substituted cycloalkyl, unsubstituted or substituted heterocycloalkyl, unsubstituted or substituted aryl, or an unsubstituted or substituted heteroaryl. R^{19B} may also be halogen, -CN, -CF₃, -OH, -NH₂, -COOH, substituted or unsubstituted C₁-C₁₀ (e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In some compounds of the disclosure, R^{19B} is halogen, -CN, -CF₃, -OH, -NH₂, -COOH, R^{20B}-substituted or unsubstituted alkyl, R^{20B}-substituted or unsubstituted heteroalkyl, R^{20B}-substituted or unsubstituted cycloalkyl, R^{20B}-substituted or unsubstituted heterocycloalkyl, R^{20B}-substituted or unsubstituted aryl, or R^{20B}-substituted or unsubstituted heteroaryl. R^{19B} may also be halogen, -CN, -CF₃, -OH, -NH₂, -COOH, R^{20B}-substituted or unsubstituted C₁-C₁₀ (e.g., C₁-C₆) alkyl, R^{20B}-substituted or unsubstituted 2 to 10 membered (e.g., 2 to 6 membered) heteroalkyl, R^{20B}-substituted or unsubstituted C₃-C₈ (e.g., C₅-C₇) cycloalkyl, R^{20B}-substituted or unsubstituted 3 to 8 membered (e.g., 3 to 6 membered) heterocycloalkyl, R^{20B}-substituted or unsubstituted C₅-C₈ (e.g., C₅-C₆) aryl, or R^{20B}-substituted or unsubstituted 5 to 8 membered (e.g., 5 to 6 membered) heteroaryl.

R^{20B} is halogen, -CN, -CF₃, -OH, -NH₂, -COOH, unsubstituted or substituted alkyl, unsubstituted or substituted heteroalkyl, unsubstituted or substituted cycloalkyl, unsubstituted or substituted heterocycloalkyl, unsubstituted or substituted aryl, or an unsubstituted or substituted heteroaryl. In some compounds of the disclosure, R^{20B} is unsubstituted or substituted alkyl, unsubstituted or substituted heteroalkyl, unsubstituted or substituted cycloalkyl, unsubstituted or substituted heterocycloalkyl, unsubstituted or substituted aryl, or an unsubstituted or substituted heteroaryl. R^{20B} may also be halogen, -CN, -CF₃, -OH, -NH₂, -COOH, substituted or unsubstituted C₁-C₁₀ (e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In some compounds of the disclosure, R^{20B} is halogen, -CN, -CF₃, -OH, -NH₂, -COOH, R^{21B}-substituted or unsubstituted alkyl, R^{21B}-substituted or unsubstituted heteroalkyl, R^{21B}-substituted or unsubstituted cycloalkyl, R^{21B}-substituted or unsubstituted heterocycloalkyl, R^{21B}-substituted or unsubstituted aryl, or R^{21B}-substituted or unsubstituted heteroaryl. R^{20B} may also be halogen, -CN, -CF₃, -OH, -NH₂, -COOH, R^{21B}-substituted or unsubstituted C₁-C₁₀ (e.g., C₁-C₆) alkyl, R^{21B}-substituted or unsubstituted 2 to 10 membered (e.g., 2 to 6 membered) heteroalkyl, R^{21B}-substituted or unsubstituted C₃-C₈ (e.g., C₅-C₇) cycloalkyl, R^{21B}-substituted or unsubstituted 3 to 8 membered (e.g., 3 to 6 membered) heterocycloalkyl, R^{21B}-substituted or unsubstituted C₅-C₈ (e.g., C₅-C₆) aryl, or R^{21B}-substituted or unsubstituted 5 to 8 membered (e.g., 5 to 6 membered) heteroaryl.

R^{21B} is halogen, -CN, -CF₃, -OH, -NH₂, -COOH, unsubstituted alkyl, unsubstituted heteroalkyl, unsubstituted cycloalkyl, unsubstituted heterocycloalkyl, unsubstituted aryl, or an unsubstituted heteroaryl.

Each R^{5D} and R^{9B} may be the same or different and may each independently be hydrogen, unsubstituted or substituted alkyl, unsubstituted or substituted heteroalkyl, unsubstituted or substituted cycloalkyl, unsubstituted or substituted heterocycloalkyl, unsubstituted or substituted aryl, or an unsubstituted or substituted heteroaryl. In some compounds of the disclosure, R^{5D} and R^{9B} are independently hydrogen, unsubstituted or substituted alkyl, unsubstituted or substituted heteroalkyl, unsubstituted or substituted cycloalkyl, unsubstituted or substituted heterocycloalkyl, unsubstituted or substituted aryl, or an unsubstituted or substituted heteroaryl. R^{5D} and R^{9B} may also be independently substituted or unsubstituted C₁-C₁₀ (e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In some compounds of the disclosure, R^{5D} and R^{9B} are R^{18C}-substituted or unsubstituted alkyl, R^{18C}-substituted or unsubstituted heteroalkyl, R^{18C}-substituted or unsubstituted cycloalkyl, R^{18C}-substituted or unsubstituted heterocycloalkyl, R^{18C}-substituted or unsubstituted aryl, or R^{18C}-substituted or unsubstituted heteroaryl. R^{5D} and R^{9B} may also be R^{18C}-substituted or unsubstituted C₁-C₁₀ (e.g., C₁-C₆) alkyl, R^{18C}-substituted or unsubstituted 2 to 10 membered (e.g., 2 to 6 membered) heteroalkyl, R^{18C}-substituted or unsubstituted C₃-C₈ (e.g., C₅-C₇) cycloalkyl, R^{18C}-substituted or unsubstituted 3 to 8 membered (e.g., 3 to 6 membered) heterocycloalkyl, R^{18C}-substituted or unsubstituted C₅-C₈ (e.g., C₅-C₆) aryl, or R^{18C}-substituted or unsubstituted 5 to 8 membered (e.g., 5 to 6 membered) heteroaryl.

R^{18C} is halogen, -CN, -CF₃, -OH, -NH₂, -COOH, unsubstituted or substituted alkyl, unsubstituted or substituted heteroalkyl, unsubstituted or substituted cycloalkyl, unsubstituted or substituted heterocycloalkyl, unsubstituted or substituted aryl, or an unsubstituted or substituted heteroaryl. In some compounds of the disclosure, R^{18C} is unsubstituted or substituted alkyl, unsubstituted or substituted heteroalkyl, unsubstituted or substituted cycloalkyl, unsubstituted or substituted heterocycloalkyl, unsubstituted or substituted aryl, or an unsubstituted or substituted heteroaryl. R^{18C} may also be halogen, -CN, -CF₃, -OH, -NH₂, -COOH, substituted or unsubstituted C₁-C₁₀ (e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In some compounds of the disclosure, R^{18C} is halogen, -CN, -CF₃, -OH, -NH₂, -COOH, R^{19C}-substituted or unsubstituted alkyl, R^{19C}-substituted or unsubstituted heteroalkyl, R^{19C}-substituted or unsubstituted cycloalkyl, R^{19C}-substituted or unsubstituted heterocycloalkyl, R^{19C}-substituted or unsubstituted aryl, or R^{19C}-substituted or unsubstituted heteroaryl. R^{18C} may also be halogen, -CN, -CF₃, -OH, -NH₂, -COOH, R^{19C}-substituted or unsubstituted C₁-C₁₀ (e.g., C₁-C₆) alkyl, R^{19C}-substituted or unsubstituted 2 to 10 membered (e.g., 2 to 6 membered) heteroalkyl, R^{19C}-substituted or unsubstituted C₃-C₈ (e.g., C₅-C₇) cycloalkyl, R^{19C}-substituted or unsubstituted 3 to 8 membered (e.g., 3 to 6 membered) heterocycloalkyl, R^{19C}-substituted or unsubstituted C₅-C₈ (e.g., C₅-C₆) aryl, or R^{19C}-substituted or unsubstituted 5 to 8 membered (e.g., 5 to 6 membered) heteroaryl.

R^{19C} is halogen, -CN, -CF₃, -OH, -NH₂, -COOH, unsubstituted or substituted alkyl, unsubstituted or substituted heteroalkyl, unsubstituted or substituted cycloalkyl, unsubstituted or substituted heterocycloalkyl, unsubstituted or substituted aryl, or an unsubstituted or substituted heteroaryl. In some compounds of the disclosure, R^{19C} is unsubstituted or substituted alkyl, unsubstituted or substituted heteroalkyl, unsubstituted or substituted cycloalkyl, unsubstituted or substituted heterocycloalkyl, unsubstituted or substituted aryl, or an unsubstituted or substituted heteroaryl. R^{19C} may also be halogen, -CN, -CF₃, -OH, -NH₂, -COOH, substituted or unsubstituted C₁-C₁₀ (e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In some compounds of the disclosure, R^{19C} is halogen, -CN, -CF₃, -OH, -NH₂, -COOH, R^{20C}-substituted or unsubstituted alkyl, R^{20C}-substituted or unsubstituted heteroalkyl, R^{20C}-substituted or unsubstituted cycloalkyl, R^{20C}-substituted or unsubstituted heterocycloalkyl, R^{20C}-substituted or unsubstituted aryl, or R^{20C}-substituted or unsubstituted heteroaryl. R^{19C} may also be halogen, -CN, -CF₃, -OH, -NH₂, -COOH, R^{20C}-substituted or unsubstituted C₁-C₁₀ (e.g., C₁-C₆) alkyl, R^{20C}-substituted or unsubstituted 2 to 10 membered (e.g., 2 to 6 membered) heteroalkyl, R^{20C}-substituted or unsubstituted C₃-C₈ (e.g., C₅-C₇) cycloalkyl, R^{20C}-substituted or unsubstituted 3 to 8 membered (e.g., 3 to 6 membered) heterocycloalkyl, R^{20C}-substituted or unsubstituted C₅-C₈ (e.g., C₅-C₆) aryl, or R^{20C}-substituted or unsubstituted 5 to 8 membered (e.g., 5 to 6 membered) heteroaryl.

R^{20C} is halogen, -CN, -CF₃, -OH, -NH₂, -COOH, unsubstituted or substituted alkyl, unsubstituted or substituted heteroalkyl, unsubstituted or substituted cycloalkyl, unsubstituted or substituted heterocycloalkyl, unsubstituted or substituted aryl, or an unsubstituted or substituted heteroaryl. In some compounds of the disclosure, R^{20C} is unsubstituted or substituted alkyl, unsubstituted or substituted heteroalkyl, unsubstituted or substituted cycloalkyl, unsubstituted or substituted heterocycloalkyl, unsubstituted or substituted aryl, or an unsubstituted or substituted heteroaryl. R^{20C} may also be halogen, -CN, -CF₃, -OH, -NH₂, -COOH, substituted or unsubstituted C₁-C₁₀ (e.g., C₁-C₆) alkyl, substituted or unsubstituted 2 to 10 membered (e.g., 2 to 6 membered) heteroalkyl, substituted or unsubstituted C₃-C₈ (e.g., C₅-C₇) cycloalkyl, substituted or unsubstituted 3 to 8 membered (e.g., 3 to 6 membered) heterocycloalkyl, substituted or unsubstituted C₅-C₈ (e.g., C₅-C₆) aryl, or substituted or unsubstituted 5 to 8 membered (e.g., 5 to 6 membered) heteroaryl. In some compounds of the disclosure, R^{20C} is halogen, -CN, -CF₃, -OH, -NH₂, -COOH, R^{21C}-substituted or unsubstituted alkyl, R^{21C}-substituted or unsubstituted heteroalkyl, R^{21C}-substituted or unsubstituted cycloalkyl, R^{21C}-substituted or unsubstituted heterocycloalkyl, R^{21C}-substituted or unsubstituted aryl, or R^{21C}-substituted or unsubstituted heteroaryl. R^{20C} may also be halogen, -CN, -CF₃, -OH, -NH₂, -COOH, R^{21C}-substituted or unsubstituted C₁-C₁₀ (e.g., C₁-C₆) alkyl, R^{21C}-substituted or unsubstituted 2 to 10 membered (e.g., 2 to 6 membered) heteroalkyl, R^{21C}-substituted or unsubstituted C₃-C₈ (e.g., C₅-C₇) cycloalkyl, R^{21C}-substituted or unsubstituted 3 to 8 membered (e.g., 3 to 6 membered) heterocycloalkyl, R^{21C}-substituted or unsubstituted C₅-C₈ (e.g., C₅-C₆) aryl, or R^{21C}-substituted or unsubstituted 5 to 8 membered (e.g., 5 to 6 membered) heteroaryl.

R^{21C} is halogen, -CN, -CF₃, -OH, -NH₂, -COOH, unsubstituted alkyl, unsubstituted heteroalkyl, unsubstituted cycloalkyl, unsubstituted heterocycloalkyl, unsubstituted aryl, or an unsubstituted heteroaryl.

In some compounds of the disclosure, R¹ is -C(X¹)₃ and R³ is -C(X³)₃. In other compounds of the disclosure, R¹ is -COOR^{5A} and R³ is -COOR^{5B}. In some compounds of the disclosure, R¹ is 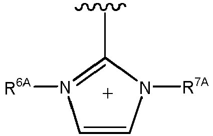 and R³ is 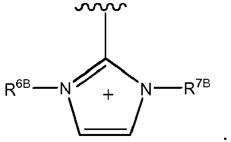 In other compounds of the disclosure, R¹ is 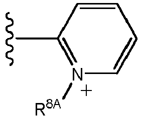 and R³ is 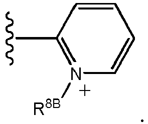 In other compounds of the disclosure, R² is -C(X²)₃ and R⁴ is -C(X⁴)₃. In other compounds of the disclosure, R² is -COOR^{5C} and R⁴ is -COOR^{5D}. In some compounds of the disclosure, R² is 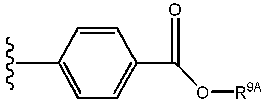 and R⁴ is 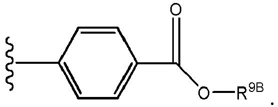 In some compounds of the disclosure, X¹, X², X³ and X⁴ are fluorine.

In more specific compounds of the disclosure, R¹ is -COOR^{5A}, R³ is -COOR^{5B}, R² is 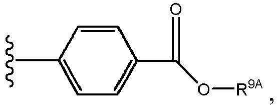 R⁴ is 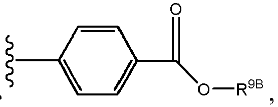 and R^{5A}, R^{5B}, R^{9A}, and R^{9B} are independently substituted or unsubstituted alkyl. In some compounds of the disclosure, each R^{5A}, R^{5B}, R^{9A}, and R^{9B} may be the same or different and may each independently be a substituted or unsubstituted alkyl (e.g. unsubstituted alkyl), and particularly a substituted or unsubstituted C₁-C₂₀ alkyl (e.g. unsubstituted C₁-C₂₀ alkyl), more particularly a substituted or unsubstituted C₁-C₁₀ alkyl (e.g. unsubstituted C₁-C₁₀ alkyl), more particularly a substituted or unsubstituted C₁-C₆ alkyl (e.g. unsubstituted C₁-C₆ alkyl), and even more particularly a substituted or unsubstituted C₁-C₄ alkyl (e.g. unsubstituted C₁-C₄ alkyl), and even more particularly, hydrogen, unsubstituted methyl, unsubstituted ethyl, or unsubstituted propyl.

In certain compounds of the disclosure, the metalloporphyrin compound has the formula:

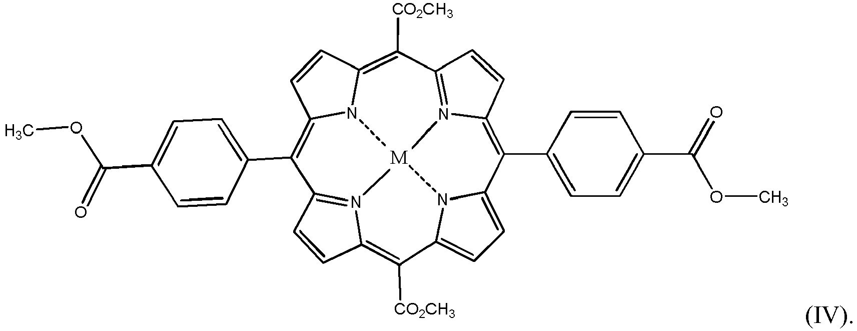

In other compounds of the disclosure, R¹ is 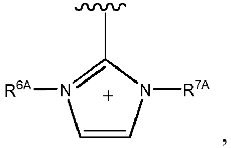 R³ is 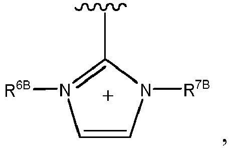 R² is 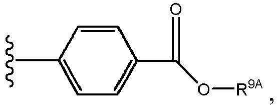 R⁴ is 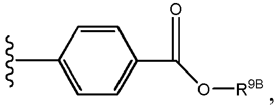 and
R^{6A}, R^{6B}, R^{7A}, R^{7B}, R^{9A} and R^{9B} are independently substituted or unsubstituted alkyl (e.g. unsubstituted alkyl). In some compounds of the disclosure, each R^{6A}, R^{6B}, R^{7A}, R^{7B}, R^{9A} and R^{9B} may be the same or different and may each independently be substituted or unsubstituted C₁-C₂₀ alkyl (e.g. unsubstituted C₁-C₂₀ alkyl), more particularly a substituted or unsubstituted C₁-C₁₀ (e.g. unsubstituted C₁-C₁₀ alkyl), more particularly a substituted or unsubstituted C₁-C₆ alkyl (e.g. unsubstituted C₁-C₆ alkyl), and even more particularly a substituted or unsubstituted C₁-C₄ alkyl (e.g. unsubstituted C₁-C₄ alkyl), and even more particularly, a hydrogen, unsubstituted methyl, unsubstituted ethyl, or unsubstituted propyl.

In specific compounds of the disclosure, the metalloporphyrin compound may have the formula:

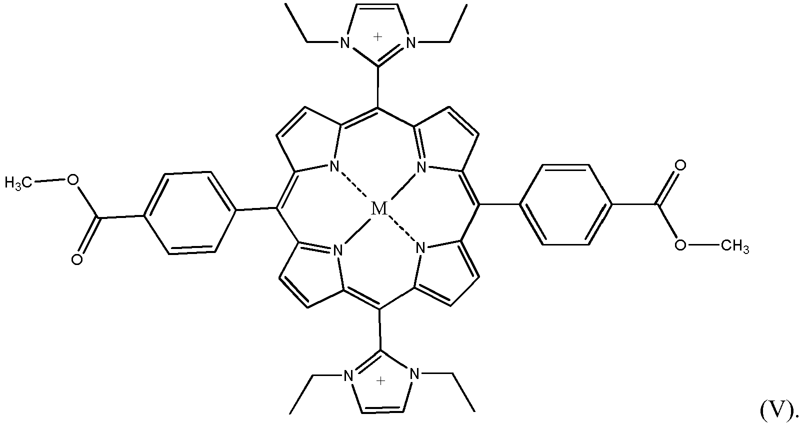

In other compounds of the disclosure, R¹ is -C(X¹)₃, R³ is -C(X³)₃, R² is 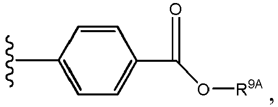 R⁴ is 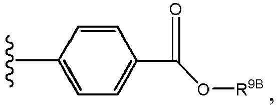 and R^{9A} and R^{9B} are independently substituted or unsubstituted alkyl. In some compounds of the disclosure, R^{9A} and R^{9B} may be independently particularly substituted or unsubstituted C₁-C₂₀ alkyl (e.g. unsubstituted C₁-C₂₀ alkyl), more particularly substituted or unsubstituted C₁-C₁₀ alkyl (e.g. unsubstituted C₁-C₁₀ alkyl), more particularly substituted or unsubstituted C₁-C₆ alkyl (e.g. unsubstituted C₁-C₆ alkyl), and even more particularly substituted or unsubstituted C₁-C₄ alkyl (e.g. unsubstituted C₁-C₄ alkyl), and even more particularly, hydrogen, unsubstituted methyl, unsubstituted ethyl, or unsubstituted propyl. In some compounds of the disclosure, X¹ and X³ are fluorine.

In an aspect, the metalloporphyrin compound of the invention has the formula:

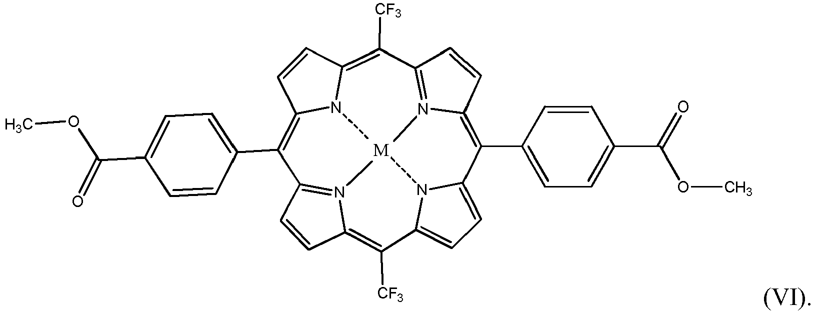

In other compounds of the disclosure, R¹ is -C(X¹)₃, R² is -C(X²)₃, R³ is -C(X³)₃ and R⁴ is -C(X⁴)₃. In some compounds of the disclosure, X¹, X², X³ and X⁴ are fluorine.

A specific metalloporphyrin compound of the disclosure may have the formula:

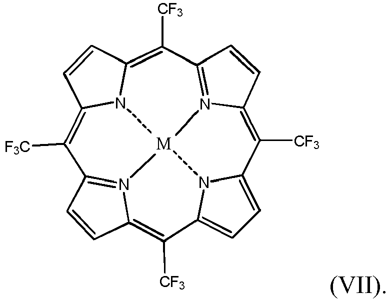

In other specific compounds of the disclosure, R¹ is 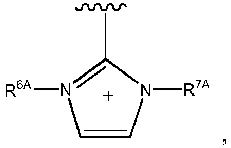 R³ is 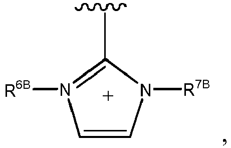 R² is -C(X²)₃, R⁴ᵢₛ -C(X⁴)₃, and R^{6A}, R^{6B}, R^{7A} and R^{7B} are independently substituted or unsubstituted alkyl (e.g. unsubstituted alkyl). In some compounds of the disclosure, each R^{6A}, R^{6B}, R^{7A} and R^{7B} may be the same or different and may each independently be an alkyl, and particularly a substituted or unsubstituted C₁-C₂₀ alkyl (e.g. unsubstituted C₁-C₂₀ alkyl), more particularly a substituted or unsubstituted C₁-C₁₀ alkyl (e.g. unsubstituted C₁-C₁₀ alkyl), more particularly a substituted or unsubstituted C₁-C₆ alkyl (e.g. unsubstituted C₁-C₆ alkyl), and even more particularly a substituted or unsubstituted C₁-C₄ alkyl (e.g. unsubstituted C₁-C₄ alkyl), and even more particularly, a hydrogen, unsubstituted methyl, unsubstituted ethyl, or unsubstituted propyl. In some compounds of the disclosure, X² and X⁴ are fluorine.

A specific metalloporphyrin compound of the disclosure may have the formula:

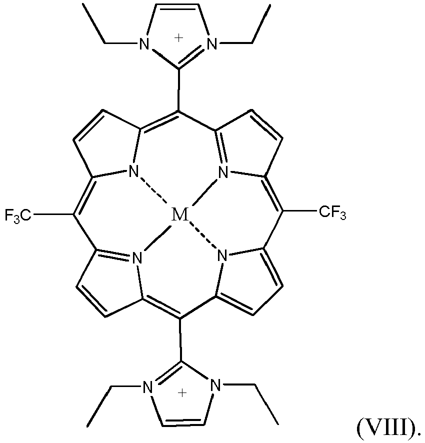

In other specific compounds of the disclosure, R¹ 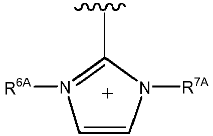 is, R³ is 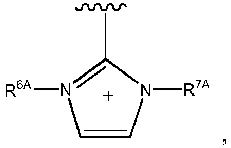 R² is -COOR^{5C}, R⁴ is -COOR^{5D}, and R^{6A}, R^{6B}, R^{7A} and R^{7B} are independently substituted or unsubstituted alkyl (e.g. unsubstituted alkyl). In some compounds of the disclosure, each R^{6A}, R^{6B}, R^{7A} and R^{7B} may be the same or different and may each independently be an alkyl, and particularly a substituted or unsubstituted C₁-C₂₀ alkyl (e.g. unsubstituted C₁-C₂₀ alkyl), more particularly a substituted or unsubstituted C₁-C₁₀ alkyl (e.g. unsubstituted C₁-C₁₀ alkyl), more particularly a substituted or unsubstituted C₁-C₆ alkyl (e.g. unsubstituted C₁-C₆ alkyl),and even more particularly a substituted or unsubstituted C₁-C₄ alkyl (e.g. unsubstituted C₁-C₄ alkyl), and even more particularly, a hydrogen, unsubstituted methyl, unsubstituted ethyl, or unsubstituted propyl.

A specific metalloporphyrin compound of the disclosure may have the formula:

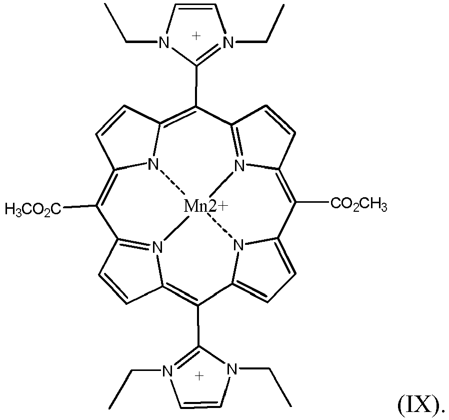

In other specific compounds of the disclosure, R¹ is 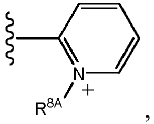 R³ is 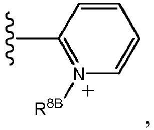 R² is -COOR^{5C}, R⁴ is -COOR^{5D}, and R^{5C}, R^{5D}, R^{8A} and R^{8B} are independently substituted or unsubstituted alkyl (e.g. unsubstituted alkyl). In some compounds of the disclosure, each R^{5C}, R^{5D}, R^{8A} and R^{8B} may be the same or different and may each independently be an alkyl, and particularly a substituted or unsubstituted C₁-C₂₀ alkyl (e.g. unsubstituted C₁-C₂₀ alkyl), more particularly a substituted or unsubstituted C₁-C₁₀ alkyl (e.g. unsubstituted C₁-C₁₀ alkyl), more particularly a substituted or unsubstituted C₁-C₆ alkyl (e.g. unsubstituted C₁-C₆ alkyl), and even more particularly a substituted or unsubstituted C₁-C₄ alkyl (e.g. unsubstituted C₁-C₄ alkyl), and even more particularly, a hydrogen, unsubstituted methyl, unsubstituted ethyl, or unsubstituted propyl.

A specific metalloporphyrin compound of the disclosure may have the formula:

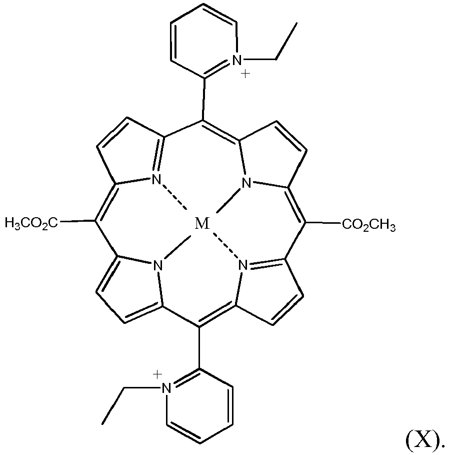

In other specific compounds of the disclosure, R¹ is 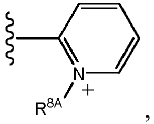 R³ is, 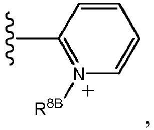 R² is -C(X²)₃, R⁴ is -C(X⁴)₃, and R^{8A} and R^{8B} are independently substituted or unsubstituted alkyl (e.g. unsubstituted alkyl). In some compounds of the disclosure, R^{8A} and R^{8B} may be an alkyl, and particularly a substituted or unsubstituted C₁-C₂₀ alkyl (e.g. unsubstituted C₁-C₂₀ alkyl), more particularly a substituted or unsubstituted C₁-C₁₀ alkyl (e.g. unsubstituted C₁-C₁₀ alkyl), more particularly a substituted or unsubstituted C₁-C₆ alkyl (e.g. unsubstituted C₁-C₆ alkyl), and even more particularly a substituted or unsubstituted C₁-C₄ alkyl (e.g. unsubstituted C₁-C₄ alkyl), and even more particularly, a hydrogen, unsubstituted methyl, unsubstituted ethyl, or unsubstituted propyl. In some compounds of the disclosure, X² and X⁴ are fluorine.

A specific metalloporphyrin compound of the disclosure may have the formula:

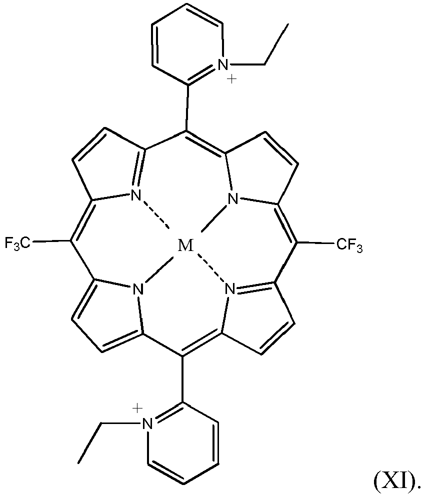

In some compounds of the disclosure, each substituted group described in the compounds above (e.g., Formulae (I)-(III)) is substituted with at least one substituent group. More specifically, in some compounds of the disclosure, each substituted alkyl, substituted heteroalkyl, substituted cycloalkyl, substituted heterocycloalkyl, substituted aryl, substituted heteroaryl, described in the compounds above (e.g., Formulae (I)-(III)) are substituted with at least one substituent group. In other compounds of the disclosure, at least one or all of these groups are substituted with at least one size-limited substituent group. Alternatively, at least one or all of these groups are substituted with at least one lower substituent group.

In other variants of the compounds described above (e.g., Formulae (I)-(III)) each substituted or unsubstituted alkyl is a substituted or unsubstituted C₁-C₂₀ alkyl, each substituted or unsubstituted heteroalkyl is a substituted or unsubstituted 2 to 20 membered heteroalkyl, each substituted or unsubstituted cycloalkyl is a substituted or unsubstituted C₃-C₈ cycloalkyl, and each substituted or unsubstituted heterocycloalkyl is a substituted or unsubstituted 3 to 8 membered heterocycloalkyl.

In some compounds of the disclosure, each substituted or unsubstituted alkyl is a substituted or unsubstituted C₁-C₈ alkyl, each substituted or unsubstituted heteroalkyl is a substituted or unsubstituted 2 to 8 membered heteroalkyl, each substituted or unsubstituted cycloalkyl is a substituted or unsubstituted C₅-C₇ cycloalkyl, and each substituted or unsubstituted heterocycloalkyl is a substituted or unsubstituted 5 to 7 membered heterocycloalkyl.

### III. Compounds and compositions for use

The invention provides compounds and pharmaceutical compositions of the invention for use in treating a neurodegenerative disorder. The use may comprise administering to a patient in need thereof an effective amount of a porphyrin compound described herein (e.g., porphyrin or metalloporphyrin compounds described in Section II) or a pharmaceutically acceptable salt thereof. In some embodiments, the neurodegenerative disease is an acute or chronic neurodegenerative disease. In some embodiments, the neurodegenerative disease is neurodegenerative disorder is Parkinson's disease, Alzheimer's disease, Pick's disease, Huntington's disease, amyotrophic lateral sclerosis, prion diseases, dystonia, dementia with Lewy bodies, multiple system atrophy, progressive supranuclear palsy, Friedreich's Ataxia, temporal lobe epilepsy, stroke, traumatic brain injury, or a mitochondrial encephalopathiy.

As used herein, the term "neuronal" or "neuron" refers to one or more cells that are a morphologic and functional unit of the brain, spinal column, and peripheral nerves consisting of nerve cell bodies, dendrites, and axons. Neuron cell types can include, but are not limited to, typical nerve cell body showing internal structure, horizontal cell from cerebral cortex, Martinotti cell, bipolar cell, unipolar cell, Purkinje cell, and pyramidal cell of motor area of cerebral cortex. Exemplary neuronal cells can include, but are not limited to, cholinergic, adrenergic, noradrenergic, dopaminergic, serotonergic, glutaminergic, GABAergic, and glycinergic.

As used herein, the term "neurodegenerative disease," "neurodegenerative disorder" or "degenerative disease" is defined as a disease or condition in which there is a progressive loss of neurons or loss of neuronal function. Thus, a neurodegenerative disorder or neurodegenerative disease, as used in the current context, should be obvious to one skilled in the art, but is meant to include any abnormal physical or mental behavior or experience where the death of neuronal cells is involved in the etiology of the disorder, or is affected by the disorder. As used herein, neurodegenerative diseases encompass disorders affecting the central and peripheral nervous systems, and include such afflictions as memory loss, stroke, dementia, personality disorders, gradual, permanent or episodic loss of muscle control. Examples of neurodegenerative disorders or diseases for which the current invention can be used preferably include, but are not limited to, Alzheimer's Disease, Parkinson's Disease, Huntington's Disease, amyotrophic lateral sclerosis (ALS), Pick's disease, prion diseases, dystonia, dementia with Lewy bodies, multiple system atrophy, progressive supranuclear palsy, Friedreich's Ataxia, temporal lobe epilepsy, stroke, traumatic brain injury, mitochondrial encephalopathies, Guillain-Barre syndrome, multiple sclerosis, epilepsy, myasthenia gravis, chronic idiopathic demyelinating disease (CID), neuropathy, ataxia, dementia, chronic axonal neuropathy and stroke.

As used herein, the term "neurodegeneration" refers to the progressive loss or function of at least one neuron or neuronal cell. One of skill in the art realizes that the term progressive loss can refer to cell death or cell apoptosis.

As used herein, the term "neuronal cell loss" refers to the loss of neuronal cells. The loss of neuronal cells may be a result of a genetic predisposition, congenital dysfunction, apoptosis, ischemic event, immune-mediated, free-radical induced, chemical induced, or any injury that results in a loss of neuronal cells, as well as a progressive loss of neuronal cells.

The disclosure provides methods for treating Parkinson's disease in a subject by administering to the subject in need thereof an effective amount of a metalloporphyrin compound, a derivative or a pharmaceutically acceptable salt thereof. As used herein, the term "Parkinson's disease" refers to a neurological disorder characterized by muscle rigidity, tremor, bradykinesia, and akinesia that is caused by the decreased stimulation of the motor cortex by the basal ganglia due to insufficient formation and action of dopamine on the dopamine receptors in the brain. The term "Parkinson's disease" includes chronic progressive parkinsonism, primary parkinsonism, idiopathic parkinsonism, secondary parkinsonism induce by drugs, trauma or other medical disorders and Parkinson-plus syndromes (e.g., multiple symptom atrophy, progressive supranuclear palsy, corticobasal degeneration, dementia with Lewy bodies and olivopontocerebellar atrophy).

The methods of inhibiting neuron degeneration can be used to inhibit or prevent neuron degeneration in patients newly diagnosed as having a neurodegenerative disease (e.g., Parkinson's disease) or at risk of developing a new neurodegenerative disease (e.g., Parkinson's disease). On the other hand, the methods of inhibiting neuron degeneration can also be used to inhibit or prevent further neuron degeneration in patients who are already suffering from, or have symptoms of, a neurodegenerative disease (e.g., Parkinson's disease). Preventing neuron degeneration includes decreasing or inhibiting neuron degeneration, which may be characterized by complete or partial inhibition of neuron degeneration. This can be assessed, for example, by analysis of neurological function.

### IV. Pharmaceutical Compositions

In some embodiments, the metalloporphyrin compounds of the invention may from part of a pharmaceutical composition. The pharmaceutical composition includes a porphyrin compound of the invention (e.g., porphyrin or metalloporphyrin compounds described in the claims) or a pharmaceutically acceptable salt thereof (also referred to herein as a "treatment compound"), and a pharmaceutically acceptable excipient. A "pharmaceutically acceptable excipient" includes pharmaceutically and physiologically acceptable, organic or inorganic carrier substances suitable for enteral or parenteral administration that do not deleteriously react with the active agent. Suitable pharmaceutically acceptable carriers include water, salt solutions (such as Ringer's solution), alcohols, oils, gelatins, and carbohydrates such as lactose, amylose or starch, fatty acid esters, hydroxymethylcellulose, and polyvinyl pyrrolidone. Such preparations can be sterilized and, if desired, mixed with auxiliary agents such as lubricants, preservatives, stabilizers, wetting agents, emulsifiers, salts for influencing osmotic pressure, buffers, coloring, and/or aromatic substances and the like that do not deleteriously react with the active agent.

In one embodiment, the treatment compound (e.g., porphyrin or metalloporphyrin compounds described in the claims) forms part of a pharmaceutical composition, wherein the pharmaceutical composition comprises said treatment compound and a pharmaceutical acceptable excipient. In one embodiment, the pharmaceutical composition includes a permeabilizer (e.g., a salicylate, a fatty acid, or a metal chelator).

The pharmaceutical composition can be formulated for any route of administration, including enteral, oral, sublingual, buccal, parenteral, ocular, intranasal, pulmonary, rectal, intravaginal, transdermal, and topical routes. Parenteral administration includes, but is not limited to, intravenous, intramuscular, subcutaneous, intradermal, intraperitoneal, intrastemal, intraarterial injection and infusion.

The pharmaceutical composition can be formulated for immediate release or modified release, e.g., modified, sustained, extended, delayed, or pulsatile release, using known methods and excipients.

In one embodiment, the pharmaceutical composition is formulated as a topical composition, an injectable composition, an inhalant, a sustained release composition, or an oral composition. The treatment compound is preferably formulated for parenteral administration, e.g., by subcutaneous injection. If subcutaneous or an alternative type of administration is used, the compounds may be derivatized or formulated such that they have a protracted profile of action.

In another embodiment, the pharmaceutical composition is formulated as a peptide micelle, a targeted micelle, a degradable polymeric dosage form, a porous microsphere, a polymer scaffold, a liposome, or a hydrogel.

The treatment compound may be formulated according to known methods to prepare pharmaceutically useful compositions. An exemplary formulation would be one that is a stable lyophilized product that is reconstituted with an appropriate diluent or an aqueous solution of high purity with optional pharmaceutically acceptable carriers, preservatives, excipients or stabilizers (Remington's Pharmaceutical Sciences 16th edition (1980)). The pharmaceutical composition may include a pharmaceutically acceptable buffer to achieve a suitable pH for stability and for administration.

For parenteral administration, the treatment compound is formulated in a unit dosage injectable form (solution, suspension, or emulsion) with a pharmaceutically acceptable carrier. Preferably, one or more pharmaceutically acceptable anti-microbial agents may be added, such as phenol, m-cresol, and benzyl alcohol.

In one embodiment, one or more pharmaceutically acceptable salts (e.g., sodium chloride), sugars (e.g., mannitol), or other excipients (e.g., glycerin) may be added to adjust the ionic strength or tonicity.

The dosage of the composition of the invention to be administered can be determined without undue experimentation and will be dependent upon various factors including the nature of the active agent (including whether metal bound or metal free), the route of administration, the patient, and the result sought to be achieved. A suitable dosage of mimetic to be administered IV or topically can be expected to be in the range of about 0.01 to 50 mg/kg/day, preferably, 0.1 to 10 mg/kg/day, more preferably 0.1 to 6 mg/kg/day. For aerosol administration, it is expected that doses will be in the range of 0.001 to 5.0 mg/kg/day, preferably, 0.01 to 1 mg/kg/day. Suitable doses will vary, for example, with the compound and with the result sought.

The concentration of mimetic presentation in a solution used to treat cells/tissues/organs in accordance with the methods of the invention can also be readily determined and will vary with the active agent, the cell/tissue/organ and the effect sought.

Certain aspects of the invention can be described in greater detail in the nonlimiting Example that follows.

### EXAMPLES

The following examples illustrate certain specific embodiments of the invention and are not meant to limit the scope of the invention. Also included are reference examples

Embodiments herein are further illustrated by the following examples and detailed protocols. However, the examples are merely intended to illustrate embodiments and are not to be construed to limit the scope herein.

### Example 1. Optimizing Metalloporphyrins for Clinical Development in Parkinson's Disease-AEOL11209

Figure 1 shows the concentration of AEOL11209 in the plasma (A) and brains (B) of C57BL/6 mice at different times points after a single dose of AEOL11209 (15mg/kg) administered by the intraperitoneal (i.p.) or oral (p.o.) route. Points represent mean ± S.E.M. Each point is the average of 3-4 animals.

**Purity analysis by mass spectrometry:** Mass spectrometric analysis of AEOL11209 revealed a compound of appropriate mass in a single peak confirming its purity.

**Pharmacokinetic analysis in plasma and brain via i.p. and p.o. route: a)** Plasma concentrations were low but achievable after i.p. administration but not after p.o. administration. b) The concentration of AEOL11209 in the brain after i.p. or p.o. administration was variable and relatively low. c) Solubility of AEOL11209 was a major issue and necessitated high concentrations of DMSO which made animals sick.

**Alternate approach:** To synthesize an analog of AEOL11209 that is more water soluble (AEOL1114B), determine its pharmacokinetic properties and efficacy in the MPTP mouse model.

### Reference Example 2. Optimizing Metalloporphyrins for Clinical Development in Parkinson's Disease-AEOL11215

Figure 2 shows the concentration of AEOL11215 in the plasma of the C57BL/6 mice at different times points after a single dose of AEOL11215 (15mg/kg) administered by the i.p route. Points represent mean ± S.E.M. Each point is the average of 3-4 animals.

**Results:** AEOL11215 achieved moderate levels in the plasma after i.p. administration. AEOL11215 was not reliably detectable in brain following i.p. administration. AEOl11215 was not reliably detected plasma after p.o. administration at any time point.

**Alternate approach:** To test an alternate compound from Dr. Day's library, AEOL11203 which has shorter meso-substituted side chains (see below).

### Reference Example 3. Optimizing Metalloporphyrins for Clinical Development in Parkinson's Disease-AEOL11216

Figure 3 shows the concentration of AEOL11216 in the plasma (A) and brains (B) of C57BL/6 mice at different times points after a single dose of AEOL11216 (15mg/kg) administered by the i.p. and p.o route. Points represent mean + S.E.M. Bars represent mean + S.E.M. n=3-6 mice per group. Figure 4 shows dopamine levels in the striata (A) number of TH+ neurons in the substantia nigra pars compacta (B) of C57BL/6 mice 7 days after injection of MPTP (15mg/kgx3, s.c., 24h intervals) alone or in the presence of AEOL11216 15 mg/kg i.p. (1 hr prior to MPTP and daily thereafter). Bars represent mean + S.E.M. n=3-6 mice per group. Figure 5 shows GSH, GSSG, GSH/GSSG and 3NT/Tyr ratios in the ventral midbrains of C57/B6 mice 24 hours after injection of MPTP (15mg/kgx3, s.c., 24h intervals) alone or in the presence of AEOL11216 i.p. (15mg/kgx3, daily). Bars represent mean + S.E.M. # p<0.01 vs MPTP. One-way, ANOVA, n=6 mice per group.

**Results:** AEOL11216 achieved high plasma concentrations after i.p. administration but not after p.o. administration. AEOL11216 penetrated the BBB and remained stable at more than 100nM in the brain for 24 hr after i.p. administration. AEOL11216 did not significantly prevent MPTP-induced striatal dopamine depletion after i.p. administration. AEOL11216 significantly protected against MPTP-induced dopaminergic neuronal loss in the substantial nigra pars compacta after i.p. administration. The dopamine neurons (TH positive neurons) were counted by an investigator blinded to the treatment of mice with stereological methods. AEOL11216 significantly protected against MPTP-induced alteration of GSH/GSSG and 3-NT/tyrosine ratio after i.p. administration.

### Reference Example 4. Optimizing Metalloporphyrins for Clinical Development in Parkinson's Disease-AEOL11203

AEOL11203's bioavailability and efficacy were tested to overcome solubility issues related to AEOL11209 and poor bioavailability of AEOL11215. Figure 6 shows the concentration of AEOL11203 in the plasma (A) and brains (B) of C57BL/6 mice at different times points after a single dose of AEOL11203 (15mg/kg) administered by the i.p. and p.o. route. Points represent mean ± S.E.M. Each point is the average of 3-4 animals. Figure 7 shows dopamine levels in the striata of C57BL/6 mice 7 days after injection of MPTP (15mg/kgx3, s.c., 24h intervals) alone or in the presence of AEOL11203 15 mg/kg i.p or p.o. (1 hr prior to MPTP and daily thereafter). Bars represent mean + S.E.M. n=6-8 mice per group. Figure 8 shows GSH, GSSG, GSH/GSSG and 3NT/Tyr ratios in the SN of C57/B6 mice 24 hours after injection of MPTP (15mg/kgx3, s.c., 24h intervals) alone or in the presence of AEOL11203 i.p. (15mg/kgx3, daily). Bars represent mean + S.E.M. # p<0.01 vs MPTP. One-way, ANOVA, n=6 mice per group.

**Results:** AEOL11203's solubility properties were good. The concentration in the plasma after i.p. administration was relative high. AEOL11203 penetrated the BBB and remain stable (>100nM) in the brain for 24 hr after i.p. administration and ~ 50 nM in the brain for 24 hr after p.o. administration. AEOL11203 achieved relatively high plasma concentrations (~5 µM) after administration via the p.o. route. AEOL11203 showed a statistically significant protection against MPTP-induced striatal dopamine depletion by i.p. administration. AEOL11203 significantly protected against MPTP-induced increased GSH/GSSG and 3-NT/tyrosine ratio after i.p. administration.

### Example 5. Optimizing Metalloporphyrins for Clinical Development in Parkinson's Disease-AEOL1114

A small quantity of an alternate compound (AEOL1114B) was synthesized and evaluated to overcome solubility issues associated with AEOL11209. Figure 9 shows the concentration of AEOL1114 in the plasma (A) and brains (B) of C57BL/6 mice at different times points after a single dose of AEOL1114 (15mg/kg) administered by the i.p. or p.o. route. Points represent mean + S.E.M. Each point is the average of 4 animals. Figure 10 shows dopamine levels in the striata of C57BL/6 mice 7 days after injection of MPTP (15mg/kgx3, s.c., 24h intervals) alone or in the presence of AEOL1114B 15 mg/kg i.p or p.o. (1 hr prior to MPTP and daily thereafterx3). Bars represent mean + S.E.M. n=6 mice per group. Figure 11 shows GSH, GSSG, GSH/GSSG and 3NT/Tyr ratios in the ventral midbrains of C57/B6 mice 24 hours after last injection of MPTP (15mg/kgx3, s.c., 24h intervals) alone or in the presence of AEOL1114 i.p. or p.o. (15mg/kgx3, daily). Bars represent mean + S.E.M. # p<0.01 vs MPTP. One-way, ANOVA, n=6 mice per group.

**Results:** AEOL1114 showed favorable solubility properties. It achieved very high plasma concentrations (~20 µM) after administration via the i.p. route and relatively good plasma concentrations (2∼3µM) after administration via the p.o. route. AEOL1114 penetrated the BBB and remain stable (>200nM) in the brain for 24 hr after administration via both of i.p. and p.o. route. AEOL1114 showed a statistically significant protection against MPTP-induced striatal dopamine depletion after administration via both of i.p. and p.o. route. AEOL1114 significantly protected against MPTP-induced alteration of GSH/GSSG and 3-NT/tyrosine ratio after administration via both of i.p. and p.o. route.

### Reference Example 6. Optimizing Metalloporphyrins for Clinical Development in Parkinson's Disease-AEOL11256

Figure 12 shows the concentration of AEOL11256 in the plasma of C57BL/6 mice at different times points after a single dose of AEOL11256 (15mg/kg) administered by the i.p. or p.o. route. Points represent mean + S.E.M. Each point is the average of 4 animals.

**Results**:_Plasma concentrations were relatively low after i.p. and p.o. administration. The concentration of AEOL11256 in the brain after i.p. or p.o. administration was undetectable. Solubility of AEOL11256 was a major issue and necessitated high concentrations of DMSO which made animals sick.

### Example 7. Effects of AEOL compounds on MPTP metabolism

Figure 13 shows striatal MPP+ levels in mice at 3 h after MPTP (15mg/kg, s.c.) alone or in the presence of AEOL11207, 11203, 11256 and 1114B (15mg/kg, i.p.) administered 1h before MPTP treatment. Bars represent mean + S.E.M. n=4-6 mice per group. As indicated in the graph above, none of the AEOL compounds tested altered the levels of MPP+ in the striata suggesting a lack of effect on MPTP metabolism.

### Example 8. Optimizing Metalloporphyrins for Clinical Development in Parkinson's Disease

As discussed, the compounds as shown in Figure 14 showed unfavorable bioavailability in vivo and/or poor solubility. Our attempt to further improve AEOL11207 efficacy by meso-substitution of -CF3 groups in all four positions was unsuccessful based on the poor oral bioavailability (Figure 12). However, the attempts to overcome the unfavorable pharmacokinetic or oral bioavailability of AEOL11209 and AEOL11215 were successful. As shown in Figure 15, shortening the side chains of AEOL11215 to the carboxy methyl side chains was met with success. AEOL11203 shows good brain bioavailability following i.p. and p.o. administration and promising efficacy in the MPTP mouse model.

Evaluation of AEOL11209 showed poor bioavailability most likely due to solubility issues. As shown in Figure 16, the structure of AEOL11209 has two meso-substituted aldehyde groups that were hypothesized to react with cellular macromolecules rendering it poorly absorbed through the gastrointestinal tract. Based on AEOL11207's structure, we hypothesized that substitution of two -CF3 groups in the AEOL11209 structure would improve bioavailability and optimize efficacy. This strategy was met with success demonstrating good pharmacokinetic properties, brain penetration, oral bioavailability and efficacy against MPTP-induced oxidative stress of AEOL 1114B.

### Example 9. Efficacy against MPTP toxicity by stereological cell counting and assessment of neurotoxicity potentially arising from manganese accumulation.

### Experimental Approach

**Assessment of tyrosine hydroxylase (TH) neurons:** Free-floating 30 µM mesencephalic serial sections will be processed with antibodies against TH for immunohistochemistry (Vector Laboratories) and counterstained with cresyl violet. Total numbers of TH and cresyl violet-stained neurons in SNpc is counted stereologically with STEREO INVESTIGATOR software.

**Stereological analysis and TH immunohistochemistry:** The 40-µm sections including the whole SN pars compacta (SNpc) from bregma -2.60 to -3.80 mm is immunostained with a rabbit antibody to TH (Chemicon, Temecula, CA) using the ABC method (ABC Elite Kit, Vector Laboratories, Burlingame, CA). The number of TH-positive neurons is quantified using stereo investigator software (MicroBrightfield, Williston, VT) by a previously described method (West, Trends in Neurosciences 22:51-61, 1999; Liang et al., J Neurosci 27:4326-4333, 2007).

**Statistical analyses** of the above parameters is conducted using a one-way ANOVA with Tukey's multiple comparison test.

**To determine potential adverse effects of AEOL112 compounds due to manganese release:** The metalloporphyrins used here have manganese as the metal center for catalyzing redox reactions. Although manganic porphyrins are extremely stable i.e. they have been found to keep the manganese chelated even in the presence of millimolar amounts of EDTA (Day et al., Arch Biochem Biophys 347:256-262, 1997) and several of these compounds have been found to be safe and efficacious when used in both *in vitro* and *in vivo* models of neurodegeneration (Patel and Day, Trends Pharmacol Sci 20:359-364, 1999), there is a possibility that chronic presence of these compounds may result in the release of manganese from porphyrin rings and a manganese based neurotoxicity. Therefore, it is important to measure any free manganese in the brain and behavioral deficits indicative of manganese accumulation in the brains of AEOL112-treated mice. Since manganese accumulation is of concern, we plan to measure manganese levels and behavioral effects after the experimental protocol used for the MPTP neurotoxicity (i.e. 1 week of injection).

**Experimental approach:** Mice (6-10 per groupX3 groups, control + compounds i.e. AEOL11207, AEOL1114B, and AEOL11203) are treated with each compound via the p.o. route in a manner identical to the treatment of drugs for evaluation of the MPTP-induced loss of TH+ cells i.e. once daily for 7 days. At the end of the 1 week period, mice are subjected to behavioral tests (see below). Following completion of behavioral testing, mice are euthanized and brain, blood, kidney and liver collected for determination of manganese levels by inductively coupled plasma mass spectrometry. The dosing regimen is selected based on Aim 2a.

**Manganese concentration assay:** Tissue samples from mice are measured by Inductively Coupled Plasma - Mass Spectrometry (ICPMS) by WCAS which is a highly sensitive analytical method for trace metal analysis.

**Behavioral assessment** Neurobehavioral function following treatment with AEOL112 compounds are evaluated using the open field test and the accelerated rotarod. Both tests provide simple assessment for general levels of activity in mice. The open field test not only provides gross locomotor activity, but also exploration habits in mice. Assessment over several days allows habituation to the environment to be evaluated.

**Open field test:** The test is performed in mice treated with AEOL compounds or vehicle. The assessment is conducted over 2-4 days in a square arena mounted within specially designed sound-proof plexiglass shells. Each day a mouse is placed in the center of the open field arena and allowed to freely move about for 20-60 minutes while being tracked by an automated tracking system.

**Motor behavior assessment by accelerating rotarod test** follows the protocol of Hamm et al. (Ferret et al., Hepatology 33:1173-1180, 2001). Rotarod test is performed by using an automated rotarod (Ugo Basile, Comerio, Italy) after AEOLs or vehicle injections. Animals are tested two consecutive daily trials (1 hour apart) with an accelerating mode (15, 20, 25, 30, 35 and 40 rpm) in 240 seconds for total 5 days. The average latency to fall from the rod will be recorded. Mice unable to grasp the rotating rod are given a latency value of 0 s.

These studies determine if the AEOL112 compounds increased free manganese levels in tissues and if this results in any adverse effects indicative of manganese toxicity in mice i.e. changes in exploration in open field test or latency in rotarod test. It is unlikely that the manganic porphyrins release any free manganese because of the following reasons. 1). They are extremely stable i.e. they have been found to keep the manganese chelated even in the presence of millimolar amounts of EDTA (Day et al., Arch Biochem Biophys 347:256-262, 1997). 2). Several of these compounds were found to be safe and efficacious when used in both *in vitro* and *in vivo* models of neurodegeneration. 3). They can be measured unchanged in the urine.

The proposed studies validate the utility of three additional lead lipophilic metalloporphyrins in a well accepted animal model of parkinsonism. These studies optimize glyoxylate class of metalloporphyrins to identify the best drug candidate for clinical development for PD. These studies specifically define the following parameters: 1) Plasma and brain pharmacokinetic profiles of three two compounds with good bioavailability and 2) antioxidant properties in the mouse MPTP model. To determine if manganese accumulation from metalloporphyrins poses a risk for adverse behavioral effects, brain manganese levels are determined in conjunction with behavioral testing after dosing with AEOL11207, AEOL1114B, AEOL11203 and AEOL11216.

### Example 10. AEOL1114B prevented MPTP-induced loss of dopaminergic neurons.

Figure 17 shows tyrosine hydroxylase positive (TH+) cell counts indicative of dopaminergic neuron viability in the substantia nigra pars compacta of C57BL/6 mice 7 days after injection of MPTP (15mg/kgx3, s.c., 24h intervals) alone or in the presence of AEOL1114B 15 mg/kg i.p or p.o. (1 hr prior to MPTP and daily thereafterx3). Bars represent mean + S.E.M. n=6. The data demonstrates that AEOL1114B prevents MPTP-induced loss of TH+ dopamine neurons in the substantia nigra pars compact when administered either via the oral or i.p. route.

### Example 11. Behavioral testing after dosing mice with AEOL11207, AEOL1114B or AEOL11203 (MnCl₂ positive control).

Mice (6 per group) were divided 5 groups treated with AEOL11207, AEOL11203, AEOL11114B, MnCl₂ (all compound dissolved in 5% DMSO) or 5%DMSO alone by 15mg/kg i.p. daily for a period of 7 days. At the end of treatment, mice were performed the behavioral tests by open field and accelerating rotorod test in 3-4h after last injection. Figure 18 shows that no statistically significant differences were observed between any groups in both behavioral tests.

## Claims

1. A compound having the formula: 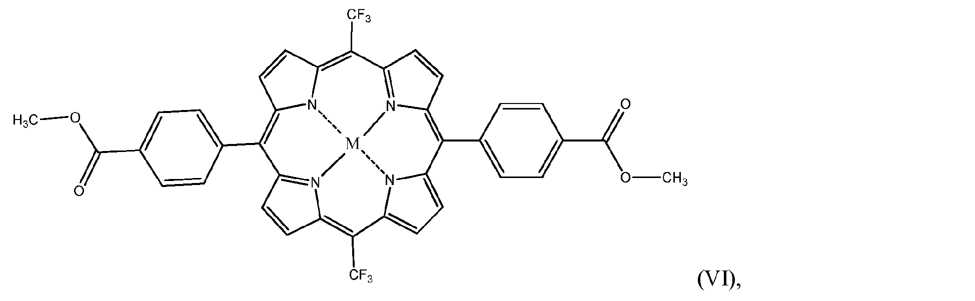 wherein,
M is a metal.

2. The compound of claim 1, wherein M is a metal ion.

3. The compound of claim 1 or claim 2, wherein M is selected from the group consisting of manganese, iron, cobalt, copper, nickel, zinc, chromium, aluminium and magnesium.

4. The compound of claim 1 or claim 2, wherein M is manganese.

5. A pharmaceutical composition comprising a compound of any preceding claim or a pharmaceutically acceptable salt thereof and a pharmaceutically acceptable excipient.

6. A compound of any one of claims 1 to 4 or a composition of claim 5 for use as a pharmaceutical.

7. A compound of any one of claims 1 to 4 or a composition of claim 5, for use in treating a neurodegenerative disorder.

8. The compound for use according to claim 7 or composition for use according to claim 7, wherein said neurodegenerative disorder is an acute or chronic neurodegenerative disease.

9. The compound for use according to claim 7 or composition for use according to claim 7, wherein said neurodegenerative disorder is Parkinson's disease, Alzheimer's disease, Pick's disease, Huntington's disease, amyotrophic lateral sclerosis, prion diseases, dystonia, dementia with Lewy bodies, multiple system atrophy, progressive supranuclear palsy, Friedreich's Ataxia, temporal lobe epilepsy, stroke, traumatic brain injury, or a mitochondrial encephalopathy.

10. The compound for use according to claim 7 or composition for use according to claim 7, wherein said neurodegenerative disorder is Parkinson's disease.

## Patentansprüche

1. Verbindung der Formel: 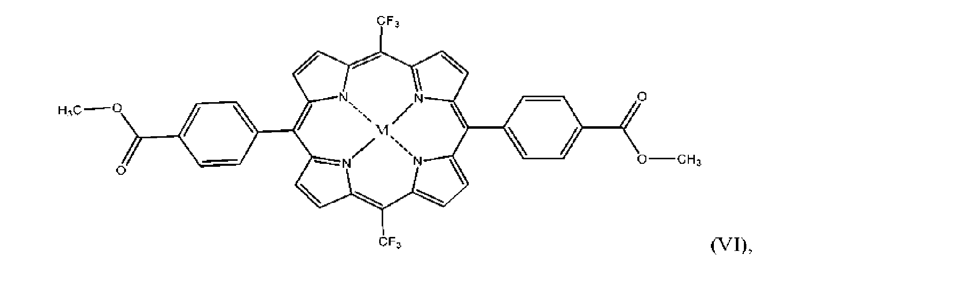 wobei
M für ein Metall steht.

2. Verbindung nach Anspruch 1, wobei M für ein Metallion steht.

3. Verbindung nach Anspruch 1 oder Anspruch 2, wobei M ausgewählt ist aus der Gruppe bestehend aus Mangan, Eisen, Cobalt, Kupfer, Nickel, Zink, Chrom, Aluminium und Magnesium.

4. Verbindung nach Anspruch 1 oder Anspruch 2, wobei M für Mangan steht.

5. Pharmazeutische Zusammensetzung, umfassend eine Verbindung nach einem vorangehenden Anspruch oder ein pharmazeutisch unbedenkliches Salz davon und einen pharmazeutisch unbedenklichen Hilfsstoff.

6. Verbindung nach einem der Ansprüche 1 bis 4 oder Zusammensetzung nach Anspruch 5 zur Verwendung als Pharmazeutikum.

7. Verbindung nach einem der Ansprüche 1 bis 4 oder Zusammensetzung nach Anspruch 5 zur Verwendung bei der Behandlung einer neurodegenerativen Störung.

8. Verbindung zur Verwendung nach Anspruch 7 oder Zusammensetzung zur Verwendung nach Anspruch 7, wobei es sich bei der neurodegenerativen Störung um eine akute oder chronische neurodegenerative Krankheit handelt.

9. Verbindung zur Verwendung nach Anspruch 7 oder Zusammensetzung zur Verwendung nach Anspruch 7, wobei es sich bei der neurodegenerativen Störung um Morbus Parkinson, Morbus Alzheimer, Morbus Pick, Chorea Huntington, amyotrophe Lateralsklerose, Prionenerkrankungen, Dystonie, Lewy-Körper-Demenz, Multisystematrophie, progressive supranukleäre Blickparese, Friedreich-Ataxie, Temporallappenepilepsie, Schlaganfall, Schädel-Hirn-Trauma oder eine mitochondriale Enzephalopathie handelt.

10. Verbindung zur Verwendung nach Anspruch 7 oder Zusammensetzung zur Verwendung nach Anspruch 7, wobei es sich bei der neurodegenerativen Störung um Morbus Parkinson handelt.

## Revendications

1. Composé ayant la formule : 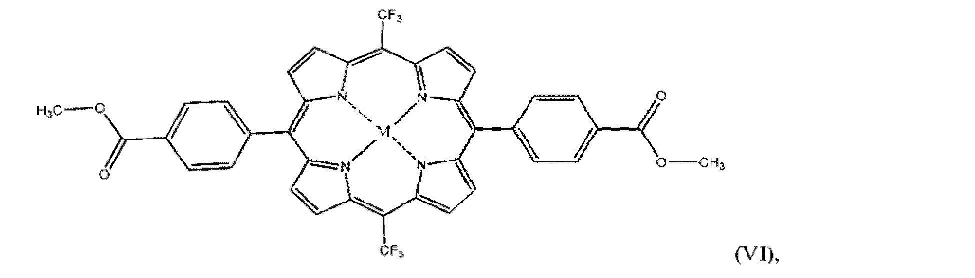 dans laquelle,
M représente un métal.

2. Composé selon la revendication 1, ledit groupe M représentant un ion métallique.

3. Composé selon la revendication 1 ou la revendication 2, ledit groupe M étant choisi dans le groupe constitué par le manganèse, le fer, le cobalt, le cuivre, le nickel, le zinc, le chrome, l'aluminium et le magnésium.

4. Composé selon la revendication 1 ou la revendication 2, ledit groupe M représentant le manganèse.

5. Composition pharmaceutique comprenant un composé selon l'une quelconque des revendications précédentes ou un sel pharmaceutiquement acceptable de celui-ci et un excipient pharmaceutiquement acceptable.

6. Composé selon l'une quelconque des revendications 1 à 4 ou composition selon la revendication 5, destiné à être utilisé comme produit pharmaceutique.

7. Composé selon l'une quelconque des revendications 1 à 4 ou composition selon la revendication 5, destiné à être utilisé dans le traitement d'un trouble neurodégénératif.

8. Composé destiné à être utilisé selon la revendication 7 ou composition destinée à être utilisée selon la revendication 7, ledit trouble neurodégénératif étant un trouble neurodégénératif aigu ou chronique.

9. Composé destiné à être utilisé selon la revendication 7 ou composition destinée à être utilisée selon la revendication 7, ledit trouble neurodégénératif étant la maladie de Parkinson, la maladie d'Alzheimer, la maladie de Pick, la maladie d'Huntington, la sclérose latérale amyotrophique, les maladies à prions, la dystonie, la démence avec corps de Lewy, l'atrophie multi-systémisée, la paralysie supranucléaire progressive, l'ataxie de Friedreich, l'épilepsie du lobe temporal, un accident vasculaire cérébral, un traumatisme crânio-cérébral ou une encéphalopathie mitochondriale.

10. Composé destiné à être utilisé selon la revendication 7 ou composition destinée à être utilisée selon la revendication 7, ledit trouble neurodégénératif étant la maladie de Parkinson.
